# EUROPEAN PATENT SPECIFICATION

(11) **EP 4 206 195 B1**
(45) Date of publication and mention of the grant of the patent: **05.11.2025**
(21) Application number: 22807610.5
(22) Date of filing: 29.03.2022
(51) Int. Cl.: C07D 403/10, C07D 251/24, C07D 401/14, C09K 11/06, C07D 237/08, C07D 239/26, C07D 239/74, C07D 257/08, C07D 253/065

(54) **NITRILE BEARING COMPOUND AND ORGANIC LIGHT-EMITTING DEVICE COMPRISING SAME**
NITRIL GRUPPE ENTHALTENDE VERBINDUNG UND ENTSPRECHENDE ORGANISCHE LICHTEMITTIERENDE VORRICHTUNG
COMPOSÉ CONTENANT UN GROUPEMENT NITRILE ET DISPOSITIF ÉLECTROLUMINESCENT ORGANIQUE LE COMPRENANT

(30) Priority: 10.05.2021 KR 20210060005
(43) Date of publication of application: 05.07.2023
(73) Proprietor: LG Chem, Ltd., Seoul 07336 (KR)
(72) Inventor: HEO, Dong Uk, Daejeon 34122 (KR); HAN, Miyeon, Daejeon 34122 (KR); LEE, Jae Tak, Daejeon 34122 (KR); YOON, Jung Min, Daejeon 34122 (KR); YUN, Heekyung, Daejeon 34122 (KR); PARK, Hoyoon, Daejeon 34122 (KR); HONG, Sung Kil, Daejeon 34122 (KR); YUN, Jun, Daejeon 34122 (KR)
(74) Representative: Hoffmann Eitle
(86) International application number: PCT/KR2022/004377
(87) International publication number: WO 2022/239962

(56) References cited:
- EP-A1- 3 674 299
- WO-A1-2019/072617
- CN-A- 111 018 797
- CN-A- 111 072 637
- CN-A- 111 072 637
- JP-A- 2013 183 010
- KR-A- 20210 032 290
- KR-A- 20210 032 290
- US-A1- 2013 270 530
- US-A1- 2019 173 020

## Description

### [Technical Field]

The present specification relates to a compound and an organic light emitting device including the same.

### [Background Art]

In general, an organic light emitting phenomenon refers to a phenomenon in which electric energy is converted into light energy by using an organic material. An organic light emitting device using the organic light emitting phenomenon usually has a structure including a positive electrode, a negative electrode, and an organic material layer interposed therebetween. Here, the organic material layer has in many cases a multi-layered structure composed of different materials in order to improve the efficiency and stability of the organic light emitting device, and for example, may be composed of a hole injection layer, a hole transport layer, a light emitting layer, an electron transport layer, an electron injection layer, and the like. In such a structure of the organic light emitting device, if a voltage is applied between the two electrodes, holes are injected from the positive electrode into the organic material layer and electrons are injected from the negative electrode into the organic material layer, and when the injected holes and electrons meet each other, an exciton is formed, and light is emitted when the exciton falls down again to a ground state.

There is a continuous need for developing a new material for the aforementioned organic light emitting device.

### [Related Art Documents]

(Patent Document 1) Korean Patent Application Laid-Open No. 10-2017-0070640

Related compounds are disclosed in patent documents US2013/270530, JP2013183010, US2019/173020 and CN111072637.

### [Detailed Description of the Invention]

### [Technical Problem]

The present specification provides a compound and an organic light emitting device including the same.

### [Technical Solution]

An exemplary embodiment of the present specification provides a compound represented by the following Chemical Formula 1.

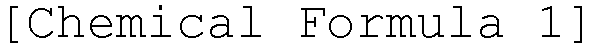

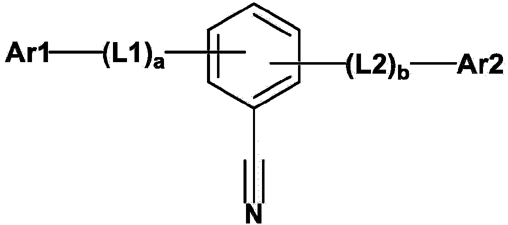

In Chemical Formula 1,
L1 and L2 are the same as or different from each other, and are each independently an arylene group which is unsubstituted or substituted with an aryl group,
Ar1 and Ar2 are the same as or different from each other, and are each independently represented by the following Chemical Formula A,

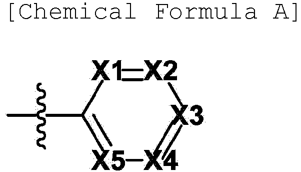

In Chemical Formula A,
X1 is N or CR1, X2 is N or CR2, X3 is N or CR3, X4 is N or CR4, and X5 is N or CR5,
at least two of X1 to X5 are N,
R1 to R5 are the same as or different from each other, and are each independently hydrogen; deuterium; a substituted or unsubstituted alkyl group; a substituted or unsubstituted cycloalkyl group; a substituted or unsubstituted aryl group; or a substituted or unsubstituted heterocyclic group, or adjacent groups are bonded to each other to form a substituted or unsubstituted aromatic hydrocarbon ring,
a and b are each an integer from 1 to 3,
when a is 2 or higher, L1's are the same as or different from each other,
when b is 2 or higher, L2's are the same as or different from each other, and 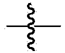 means a position bonded to Chemical Formula 1.

Further, an exemplary embodiment of the present specification provides an organic light emitting device including: an anode; a cathode; and an organic material layer having one or more layers provided between the anode and the cathode, in which one or more layers of the organic material layer include the compound represented by Chemical Formula 1.

### [Advantageous Effects]

The compound described in the present specification can be used as a material for an organic material layer of an organic light emitting device. The compound according to at least one exemplary embodiment of the present specification may improve the efficiency, achieve low driving voltage and/or improve service life characteristics in the organic light emitting device. In particular, the compound described in the present specification can be used as a material for hole injection, hole transport, hole injection and hole transport, electron blocking, light emission, hole blocking, electron transport, or electron injection. In addition, the organic light emitting device in which the compound described in the present specification is used has the effects of a low driving voltage, a high efficiency and/or a long service life compared to existing organic light emitting devices.

### [Brief Description of Drawings]

FIG. 1 illustrates an example of an organic light emitting device in which a substrate 1, an anode 2, a light emitting layer 5, and a cathode 9 are sequentially stacked.
FIG. 2 illustrates an example of an organic light emitting device in which a substrate 1, an anode 2, a hole injection layer 3, a hole transport layer 4, a light emitting layer 5, an electron transport layer 6, an electron injection layer 7, and a cathode 9 are sequentially stacked.
FIG. 3 illustrates an example of an organic light emitting device in which a substrate 1, an anode 2, a hole injection layer 3, a first hole transport layer 4-1, a second hole transport layer 4-2, a light emitting layer 5, an electron transport and injection layer 8, and a cathode 9 are sequentially stacked.
FIG. 4 illustrates an example of an organic light emitting device in which a substrate 1, an anode 2, a hole injection layer 3, a hole transport layer 4, a light emitting layer 5, an electron transport and injection layer 8, and a cathode 9 are sequentially stacked.

### [Explanation of Reference Numerals and Symbols]

1: Substrate
2: Anode
3: Hole injection layer
4: Hole transport layer
4-1: First hole transport layer
4-2: Second hole transport layer
5: Light emitting layer
6: Electron transport layer
7: Electron injection layer
8: Electron transport and injection layer
9: Cathode

### [Best Mode]

Hereinafter, the present specification will be described in more detail.

When one part "includes" one constituent element in the present specification, unless otherwise specifically described, this does not mean that another constituent element is excluded, but means that another constituent element may be further included.

When one member is disposed "on" another member in the present specification, this includes not only a case where the one member is brought into contact with another member, but also a case where still another member is present between the two members.

In the present specification, 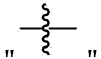 or a dotted line means a position bonded to a chemical formula or a compound.

Examples of the substituents in the present specification will be described below, but are not limited thereto.

The term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more are substituted, the two or more substituents may be the same as or different from each other.

In the present invention, the term "substituted or unsubstituted" means being substituted with one or two or more substituents selected from the group consisting of deuterium; a halogen group; a cyano group (-CN); a nitro group; a hydroxyl group; an alkyl group; a cycloalkyl group; an alkoxy group; a phosphine oxide group; an aryloxy group; an alkylthioxy group; an arylthioxy group; an alkylsulfoxy group; an arylsulfoxy group; an alkenyl group; a silyl group; a boron group; an amine group; an aryl group; or a heterocyclic group, being substituted with a substituent to which two or more substituents among the exemplified substituents are linked, or having no substituent. For example, "the substituent to which two or more substituents are linked" may be a biphenyl group. That is, the biphenyl group may also be an aryl group, and may be interpreted as a substituent to which two phenyl groups are linked.

In the present specification, the term "substituted or unsubstituted" means being substituted with one or two or more substituents selected from the group consisting of deuterium; a halogen group; a cyano group; a nitro group; a hydroxyl group; an amine group; a silyl group; a boron group; an alkoxy group; an aryloxy group; an alkyl group; a cycloalkyl group; an aryl group; and a heterocyclic group, being substituted with a substituent to which two or more substituents among the above-exemplified substituents are linked, or having no substituent.

In the present specification, the term "substituted or unsubstituted" means being substituted with one or two or more substituents selected from the group consisting of deuterium; a cyano group; an alkyl group; a cycloalkyl group; an aryl group; and a heterocyclic group, being substituted with a substituent to which two or more substituents among the exemplified substituents are linked, or having no substituent.

In the present specification, the term "substituted or unsubstituted" means being substituted with one or two or more substituents selected from the group consisting of deuterium; an alkyl group; a cycloalkyl group; an aryl group; and a heterocyclic group, being substituted with a substituent to which two or more substituents among the exemplified substituents are linked, or having no substituent.

Examples of the substituents will be described below, but are not limited thereto.

In the present specification, examples of a halogen group include fluorine (-F), chlorine (-Cl), bromine (-Br) or iodine (-I).

In the present specification, a silyl group may be represented by a chemical formula of -SiYₐY_{b}Y_{c}, and the Yₐ, Y_{b}, and Y_{c} may be each hydrogen; a substituted or unsubstituted alkyl group; or a substituted or unsubstituted aryl group. Specific examples of the silyl group include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but are not limited thereto.

In the present specification, a boron group may be represented by a chemical formula of -BY_{d}Yₑ, and the Y_{d} and Yₑ may be each hydrogen; a substituted or unsubstituted alkyl group; or a substituted or unsubstituted aryl group. Specific examples of the boron group include a trimethylboron group, a triethylboron group, a t-butyldimethylboron group, a triphenylboron group, a phenylboron group, and the like, but are not limited thereto.

In the present specification, the alkyl group may be straight-chained or branched, and the number of carbon atoms thereof is not particularly limited, but is preferably 1 to 60. According to an exemplary embodiment, the number of carbon atoms of the alkyl group is 1 to 30. According to another exemplary embodiment, the number of carbon atoms of the alkyl group is 1 to 20. According to still another exemplary embodiment, the number of carbon atoms of the alkyl group is 1 to 10. Specific examples of the alkyl group include a methyl group, an ethyl group, a propyl group, an n-propyl group, an isopropyl group, a butyl group, an n-butyl group, an isobutyl group, a tert-butyl group, a pentyl group, an n-pentyl group, a hexyl group, an n-hexyl group, a heptyl group, an n-heptyl group, an octyl group, an n-octyl group, and the like, but are not limited thereto.

In the present specification, the above-described description on the alkyl group may be applied to an arylalkyl group, except that the arylalkyl group is substituted with an aryl group.

In the present specification, the alkoxy group may be straight-chained, branched, or cyclic. The number of carbon atoms of the alkoxy group is not particularly limited, but is preferably 1 to 20. Specific examples thereof include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, and the like, but are not limited thereto. Substituents including an alkyl group, an alkoxy group, and other alkyl group moieties described in the present specification include both a straight-chained form and a branched form.

In the present specification, an alkenyl group may be straight-chained or branched, and the number of carbon atoms thereof is not particularly limited, but is preferably 2 to 40. According to an exemplary embodiment, the number of carbon atoms of the alkenyl group is 2 to 20. According to another exemplary embodiment, the number of carbon atoms of the alkenyl group is 2 to 10. According to still another exemplary embodiment, the number of carbon atoms of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a styrenyl group, and the like, but are not limited thereto.

In the present specification, the alkynyl group may be straight-chained or branched as a substituent including a triple bond between a carbon atom and a carbon atom, and the number of carbon atoms thereof is not particularly limited, but is preferably 2 to 40. According to an exemplary embodiment, the number of carbon atoms of the alkynyl group is 2 to 20. According to another exemplary embodiment, the number of carbon atoms of the alkynyl group is 2 to 10.

In the present specification, a cycloalkyl group is not particularly limited, but has preferably 3 to 60 carbon atoms, and according to an exemplary embodiment, the number of carbon atoms of the cycloalkyl group is 3 to 30. According to another exemplary embodiment, the number of carbon atoms of the cycloalkyl group is 3 to 20. According to still another exemplary embodiment, the number of carbon atoms of the cycloalkyl group is 3 to 6. Specific examples thereof include a cyclopropyl group, a cyclobutyl group, a cyclopentyl group, a cyclohexyl group, a cycloheptyl group, a cyclooctyl group, and the like, but are not limited thereto.

In the present specification, an amine group is -NH₂, and the amine group may be substituted with the above-described alkyl group, aryl group, heterocyclic group, alkenyl group, cycloalkyl group, a combination thereof, and the like. The number of carbon atoms of the substituted amine group is not particularly limited, but is preferably 1 to 30. According to an exemplary embodiment, the number of carbon atoms of the amine group is 1 to 20. According to an exemplary embodiment, the number of carbon atoms of the amine group is 1 to 10. Specific examples of the substituted amine group include a methylamine group, a dimethylamine group, an ethylamine group, a diethylamine group, a phenylamine group, a 9,9-dimethylfluorenylphenylamine group, a pyridylphenylamine group, a diphenylamine group, a phenylpyridylamine group, a naphthylamine group, a biphenylamine group, an anthracenylamine group, a dibenzofuranylphenylamine group, a 9-methylanthracenylamine group, a diphenylamine group, a phenylnaphthylamine group, a ditolylamine group, a phenyltolylamine group, a diphenylamine group, and the like, but are not limited thereto.

In the present specification, an aryl group is not particularly limited, but has preferably 6 to 60 carbon atoms, and may be a monocyclic aryl group or a polycyclic aryl group. According to an exemplary embodiment, the number of carbon atoms of the aryl group is 6 to 30. According to an exemplary embodiment, the number of carbon atoms of the aryl group is 6 to 20.

In the present specification, the aryl group may be an aryl group composed of a single ring or a polycyclic aryl group (a bicyclic or more aryl group). The aryl group composed of the single ring may mean a phenyl group; or a group to which two or more phenyl groups are linked. Examples of the aryl group composed of the sing ring include a phenyl group, a biphenyl group, a terphenyl group, a quarterphenyl group, and the like, but are not limited thereto. The polycyclic aryl group may mean a group in which two or more monocyclic rings such as a naphthyl group and a phenanthrenyl group are fused. Examples of the polycyclic aryl group include a naphthyl group, an anthracenyl group, a phenanthrenyl group, a pyrenyl group, a perylenyl group, a chrysenyl group, a fluorenyl group, a triphenylenyl group, and the like, but are not limited thereto.

In the present specification, a fluorenyl group may be substituted, and two substituents may be bonded to each other to form a spiro structure. In this case, the spiro structure may be an aromatic hydrocarbon ring or an aliphatic hydrocarbon ring.

When the fluorneyl group is substituted, the substituent may be a spirofluorenyl group 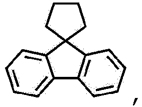 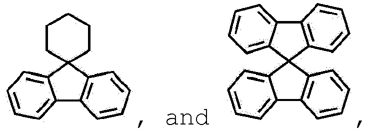 and a substituted fluorenyl group such as 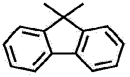 (a 9,9-dimethylfluorenyl group), and 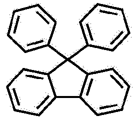 (a 9,9-diphenylfluorenyl group). However, the substituent is not limited thereto.

In the present specification, the above-described description on the aryl group may be applied to an aryl group in an aryloxy group.

In the present specification, the above-described description on the alkyl group may be applied to an alkyl group in the alkylthioxy group and the alkylsulfoxy group.

In the present specification, the above-described description on the aryl group may be applied to an aryl group in the arylthioxy group and the arylsulfoxy group.

In the present specification, a heterocyclic group is a cyclic group including one or more of N, O, P, S, Si, and Se as a heteroatom, and the number of carbon atoms thereof is not particularly limited, but is preferably 2 to 60. According to an exemplary embodiment, the number of carbon atoms of the heterocyclic group is 2 to 30. According to an exemplary embodiment, the number of carbon atoms of the heterocyclic group is 2 to 20. Examples of the heterocyclic group include a pyridine group, a pyrrole group, a pyrimidine group, a quinoline group, a pyridazinyl group, a furan group, a thiophene group, an imidazole group, a pyrazole group, a dibenzofuran group, a dibenzothiophene group, a carbazole group, a benzocarbazole group, a naphthobenzofuran group, a benzonaphthothiophene group, an indenocarbazole group, a triazinyl group, and the like, but are not limited thereto.

In the present specification, the above-described description on the heterocyclic group may be applied to a heteroaryl group except for an aromatic heteroaryl group.

In the present specification, the description on the aryl group may be applied to an arylene group except for a divalent arylene group.

In the present specification, the description on the heterocyclic group may be applied to a divalent heterocyclic group except for a divalent heterocyclic group.

In the present specification, the description on the heteroaryl group may be applied to a heteroarylene group except for a divalent heteroarylene group.

In the present specification, in a substituted or unsubstituted ring formed by being bonded to an adjacent group, the "ring" means a hydrocarbon ring; or a hetero ring.

The hydrocarbon ring may be an aromatic ring, an aliphatic ring, or a fused ring of the aromatic ring and the aliphatic ring, and may be selected from the examples of the cycloalkyl group or the aryl group.

In the present specification, being bonded to an adjacent group to form a ring means being bonded to an adjacent group to form a substituted or unsubstituted aliphatic hydrocarbon ring; a substituted or unsubstituted aromatic hydrocarbon ring; a substituted or unsubstituted aliphatic hetero ring; a substituted or unsubstituted aromatic hetero ring; or a fused ring thereof. The hydrocarbon ring means a ring composed only of carbon and hydrogen atoms. The hetero ring means a ring including one or more selected from elements such as N, O, P, S, Si and Se. In the present specification, the aliphatic hydrocarbon ring, the aromatic hydrocarbon ring, the aliphatic hetero ring, and the aromatic hetero ring may be monocyclic or polycyclic.

In the present specification, the aliphatic hydrocarbon ring means a ring composed only of carbon and hydrogen atoms as a ring which is not an aromatic group. Examples of the aliphatic hydrocarbon ring include cyclopropane, cyclobutane, cyclobutene, cyclopentane, cyclopentene, cyclohexane, cyclohexene, 1,4-cyclohexadiene, cycloheptane, cycloheptene, cyclooctane, cyclooctene, and the like, but are not limited thereto.

In the present specification, an aromatic hydrocarbon ring means an aromatic ring composed only of carbon and hydrogen atoms. Examples of the aromatic hydrocarbon ring include benzene, naphthalene, anthracene, phenanthrene, perylene, fluoranthene, triphenylene, phenalene, pyrene, tetracene, chrysene, pentacene, fluorene, indene, acenaphthylene, benzofluorene, spirofluorene, and the like, but are not limited thereto. In the present specification, the aromatic hydrocarbon ring may be interpreted to have the same meaning as the aryl group.

In the present specification, an aliphatic hetero ring means an aliphatic ring including one or more of hetero atoms. Examples of the aliphatic hetero ring include oxirane, tetrahydrofuran, 1,4-dioxane, pyrrolidine, piperidine, morpholine, oxepane, azocane, thiocane, and the like, but are not limited thereto.

In the present specification, an aromatic hetero ring means an aromatic ring including one or more of hetero atoms. Examples of the aromatic hetero ring include pyridine, pyrrole, pyrimidine, pyridazine, furan, thiophene, imidazole, pyrazole, oxazole, isoxazole, thiazole, isothiazole, triazole, oxadiazole, thiadiazole, dithiazole, tetrazole, pyran, thiopyran, diazine, oxazine, thiazine, dioxine, triazine, tetrazine, isoquinoline, quinoline, quinone, quinazoline, quinoxaline, naphthyridine, acridine, phenanthridine, diaza naphthalene, triazaindene, indole, indolizine, benzothiazole, benzoxazole, benzoimidazole, benzothiophene, benzofuran, dibenzothiophene, dibenzofuran, carbazole, benzocarbazole, dibenzocarbazole, phenazine, imidazopyridine, phenoxazine, indolocarbazole, indenocarbazole, and the like, but are not limited thereto.

Hereinafter, preferred exemplary embodiments of the present invention will be described in detail. However, the exemplary embodiments of the present invention may be modified into various other forms, and the scope of the present invention is not limited to the exemplary embodiments which will be described below.

The compound represented by Chemical Formula 1 according to the present invention is characterized in that two N-containing cyclic groups represented by Chemical Formula A are linked to a benzene ring (a phenylene group) including a cyano group through linkers (L1 and L2), and exhibits effects of increasing the efficiency of an organic light emitting device by increasing the electron mobility due to two N-containing cyclic groups in the molecule, and increasing the service life of the organic light emitting device by including one cyano group in the molecule to adjust electron injection characteristics. In addition, by certainly including linkers (L1 and L2) between a benzene ring including a cyano group and an N-containing cyclic group represented by Chemical Formula A, it is possible to obtain high efficiency, low voltage and/or long service life characteristics when the compound is applied to an organic light emitting device.

Therefore, when the above-described compound represented by Chemical Formula 1 is applied to an organic light emitting device, it is possible to obtain an organic light emitting device having high efficiency, low voltage, and/or long service life characteristics.

Hereinafter, Chemical Formula 1 will be described in detail.

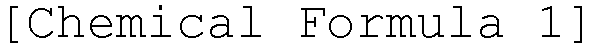

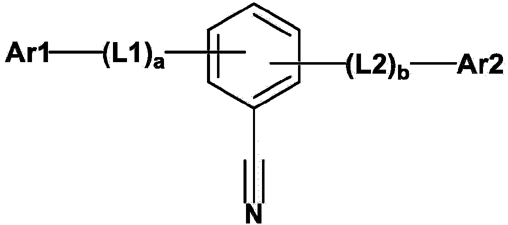

In Chemical Formula 1,
L1 and L2 are the same as or different from each other, and are each independently an arylene group which is unsubstituted or substituted with an aryl group,
Ar1 and Ar2 are the same as or different from each other, and are each independently represented by the following Chemical Formula A,

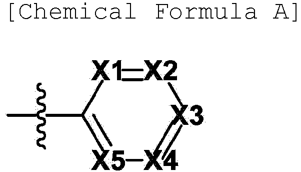

In Chemical Formula A,
X1 is N or CR1, X2 is N or CR2, X3 is N or CR3, X4 is N or CR4, and X5 is N or CR5,
at least two of X1 to X5 are N,
R1 to R5 are the same as or different from each other, and are each independently hydrogen; deuterium; a substituted or unsubstituted alkyl group; a substituted or unsubstituted cycloalkyl group; a substituted or unsubstituted aryl group; or a substituted or unsubstituted heterocyclic group, or adjacent groups are bonded to each other to form a substituted or unsubstituted aromatic hydrocarbon ring,
a and b are each an integer from 1 to 3,
when a is 2 or higher, L1's are the same as or different from each other,
when b is 2 or higher, L2's are the same as or different from each other, and 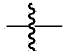 means a position bonded to Chemical Formula 1.

In an exemplary embodiment of the present specification, L1 and L2 are the same as or different from each other, and are each independently an arylene group which is unsubstituted or substituted with an aryl group.

In an exemplary embodiment of the present specification, L1 and L2 are the same as or different from each other, and are each independently an arylene group having 6 to 60 carbon atoms, which is unsubstituted or substituted with an aryl group having 6 to 60 carbon atoms.

In an exemplary embodiment of the present specification, L1 and L2 are the same as or different from each other, and are each independently an arylene group having 6 to 30 carbon atoms, which is unsubstituted or substituted with an aryl group having 6 to 30 carbon atoms.

In an exemplary embodiment of the present specification, L1 and L2 are the same as or different from each other, and are each independently a phenylene group which is unsubstituted or substituted with an aryl group; a biphenylene group which is unsubstituted or substituted with an aryl group; a terphenylene group which is unsubstituted or substitutede with an aryl group; or a naphthylene group which is unsubstituted or substituted with an aryl group.

In an exemplary embodiment of the present specification, L1 and L2 are the same as or different from each other, and are each independently a phenylene group; a biphenylene group; or a naphthylene group.

In an exemplary embodiment of the present specification, L1 and L2 are the same as or different from each other, and are each independently represented by any one of the following structural formulae.

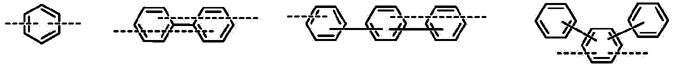

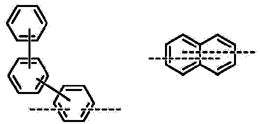

In the structural formulae, a dotted line means a bonding position.

In an exemplary embodiment of the present specification, L1 and L2 are the same as or different from each other, and are each independently represented by any one of the following structural formulae.

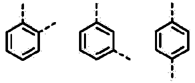

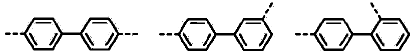

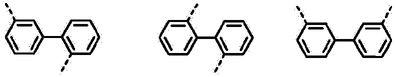

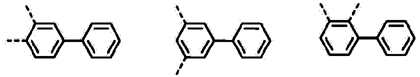

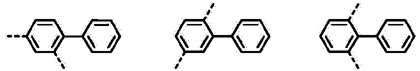

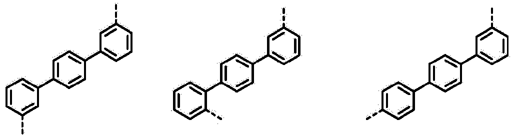

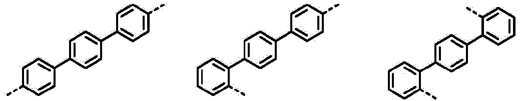

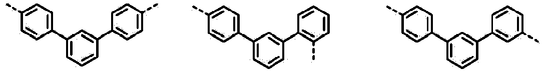

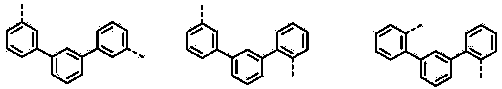

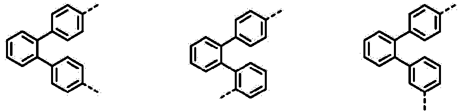

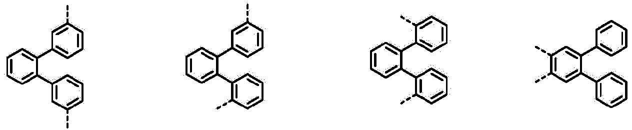

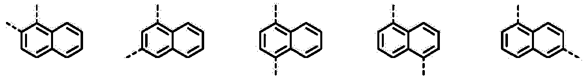

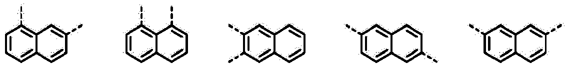

In the structural formulae, a dotted line means a bonding position.

In an exemplary embodiment of the present specification, L1 and L2 are the same as or different from each other, and are each independently represented by any one of the following structural formulae.

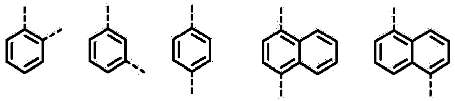

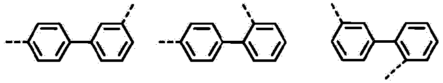

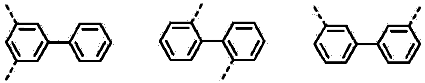

In the structural formulae, a dotted line means a bonding position.

In an exemplary embodiment of the present specification, Ar1 and Ar2 are the same as or different from each other, and are each independently represented by the following Chemical Formula A.

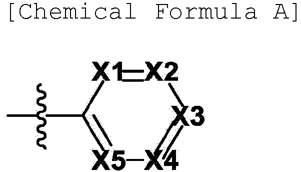

In Chemical Formula A,
X1 is N or CR1, X2 is N or CR2, X3 is N or CR3, X4 is N or CR4, and X5 is N or CR5,
at least two of X1 to X5 are N,
R1 to R5 are the same as or different from each other, and are each independently hydrogen; deuterium; a substituted or unsubstituted alkyl group; a substituted or unsubstituted cycloalkyl group; a substituted or unsubstituted aryl group; or a substituted or unsubstituted heterocyclic group, or adjacent groups are bonded to each other to form a substituted or unsubstituted aromatic hydrocarbon ring, and 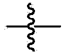 means a position bonded to Chemical Formula 1.

In an exemplary embodiment of the present specification, X1 and X2 are N, X3 is CR3, X4 is CR4, and X5 is CR5.

In an exemplary embodiment of the present specification, X1 and X3 are N, X2 is CR2, X4 is CR4, and X5 is CR5.

In an exemplary embodiment of the present specification, X1 and X4 are N, X2 is CR2, X3 is CR3, and X5 is CR5.

In an exemplary embodiment of the present specification, X1 and X5 are N, X2 is CR2, X3 is CR3, and X4 is CR4.

In an exemplary embodiment of the present specification, X2 and X3 are N, X1 is CR1, X4 is CR4, and X5 is CR5.

In an exemplary embodiment of the present specification, X2 and X4 are N, X1 is CR1, X3 is CR3, and X5 is CR5.

In an exemplary embodiment of the present specification, X1, X3 and X5 are N, X2 is CR2, and X4 is CR4.

In an exemplary embodiment of the present specification, X1, X4 and X5 are N, X2 is CR2, and X3 is CR3.

In an exemplary embodiment of the present specification, X1, X2, X4 and X5 are N, and X3 is CR3.

In an exemplary embodiment of the present specification, two to four of X1 to X5 are N.

In an exemplary embodiment of the present specification, R1 to R5 are the same as or different from each other, and are each independently hydrogen; deuterium; a substituted or unsubstituted alkyl group; a substituted or unsubstituted cycloalkyl group; a substituted or unsubstituted aryl group; or a substituted or unsubstituted heterocyclic group, or adjacent groups are bonded to each other to form a substituted or unsubstituted aromatic hydrocarbon ring.

In an exemplary embodiment of the present specification, R1 to R5 are the same as or different from each other, and are each independently hydrogen; deuterium; a substituted or unsubstituted alkyl group having 1 to 30 carbon atoms; a substituted or unsubstituted cycloalkyl group having 3 to 60 carbon atoms; a substituted or unsubstituted aryl group having 6 to 60 carbon atoms; or a substituted or unsubstituted heterocyclic group having 2 to 60 carbon atoms, or adjacent groups are bonded to each other to form a substituted or unsubstituted aromatic hydrocarbon ring having 6 to 60 carbon atoms.

In an exemplary embodiment of the present specification, R1 to R5 are the same as or different from each other, and are each independently hydrogen; deuterium; a substituted or unsubstituted alkyl group having 1 to 20 carbon atoms; a substituted or unsubstituted cycloalkyl group having 3 to 30 carbon atoms; a substituted or unsubstituted aryl group having 6 to 30 carbon atoms; or a substituted or unsubstituted heterocyclic group having 2 to 30 carbon atoms, or adjacent groups are bonded to each other to form a substituted or unsubstituted aromatic hydrocarbon ring having 6 to 30 carbon atoms.

In an exemplary embodiment of the present specification, R1 to R5 are the same as or different from each other, and are each independently hydrogen; deuterium; a substituted or unsubstituted alkyl group having 1 to 20 carbon atoms; a substituted or unsubstituted cycloalkyl group having 3 to 30 carbon atoms; a substituted or unsubstituted aryl group having 6 to 30 carbon atoms; or a substituted or unsubstituted O-**, S-** or N-containing heterocyclic group having 2 to 30 carbon atoms, or adjacent groups are bonded to each other to form a substituted or unsubstituted aromatic hydrocarbon ring having 6 to 30 carbon atoms.

In an exemplary embodiment of the present specification, R1 to R5 are the same as or different from each other, and are each independently hydrogen; deuterium; an alkyl group; a cycloalkyl group which is unsubstituted or substituted with a cyano group, an alkyl group, an aryl group or a cycloalkyl group; an aryl group which is unsubstituted or substituted with a cyano group, an alkyl group, an aryl group or a cycloalkyl group; or a heterocyclic group which is unsubstituted or substituted with an alkyl group, an aryl group or a cycloalkyl group, or adjacent groups are bonded to each other to form an aromatic hydrocarbon ring which is unsubstituted or substituted with an alkyl group, an aryl group or a cycloalkyl group.

In an exemplary embodiment of the present specification, R1 to R5 are the same as or different from each other, and are each independently hydrogen; deuterium; an alkyl group having 1 to 20 carbon atoms; a cycloalkyl group having 3 to 30 carbon atoms, which is unsubstituted or substituted with a cyano group, an alkyl group, an aryl group or a cycloalkyl group; an aryl group having 6 to 30 carbon atoms, which is unsubstituted or substituted with a cyano group, an alkyl group, an aryl group or a cycloalkyl group; or a heterocyclic group having 2 to 30 carbon atoms, which is unsubstituted or substituted with a cyano group, an alkyl group, an aryl group or a cycloalkyl group, or adjacent groups are bonded to each other to form an aromatic hydrocarbon ring having 6 to 30 carbon atoms, which is unsubstituted or substituted with a cyano group, an alkyl group, an aryl group or a cycloalkyl group.

In an exemplary embodiment of the present specification, R1 to R5 are the same as or different from each other, and are each independently hydrogen; deuterium; an alkyl group having 1 to 20 carbon atoms; a cycloalkyl group having 3 to 30 carbon atoms; an aryl group having 6 to 30 carbon atoms, which is unsubstituted or substituted with a cyano group, an alkyl group, an aryl group or a cycloalkyl group; or a heterocyclic group having 2 to 30 carbon atom, which is unsubstituted or substituted with an alkyl group, an aryl group or a cycloalkyl group, or adjacent groups are bonded to each other to form an aromatic hydrocarbon ring having 6 to 30 carbon atoms, which is unsubstituted or substituted with an alkyl group, an aryl group or a cycloalkyl group.

In an exemplary embodiment of the present specification, R1 to R5 are the same as or different from each other, and are each independently hydrogen; deuterium; a substituted or unsubstituted methyl group; a substituted or unsubstituted propyl group; a substituted or unsubstituted cyclohexyl group; a substituted or unsubstituted phenyl group; a substituted or unsubstituted biphenyl group; a substituted or unsubstituted naphthyl group; or a substituted or unsubstituted pyridyl group, or adjacent groups are bonded to each other to form a substituted or unsubstituted benzene ring.

In an exemplary embodiment of the present specification, R1 to R5 are the same as or different from each other, and are each independently hydrogen; deuterium; a methyl group; a propyl group; a cyclohexyl group which is unsubstituted or substituted with a cyano group, an alkyl group, an aryl group or a cycloalkyl group; a phenyl group which is unsubstituted or substituted with a cyano group, an alkyl group, an aryl group or a cycloalkyl group; a biphenyl group which is unsubstituted or substituted with a cyano group, an alkyl group, an aryl group or a cycloalkyl group; a naphthyl group which is unsubstituted or substituted with a cyano group, an alkyl group, an aryl group or a cycloalkyl group; or a pyridyl group which is unsubstituted or substituted with a cyano group, an alkyl group, an aryl group or a cycloalkyl group, or adjacent groups are bonded to each other to form a benzene ring which is unsubstituted or substituted with a cyano group, an alkyl group, an aryl group or a cycloalkyl group.

In an exemplary embodiment of the present specification, R1 to R5 are the same as or different from each other, and are each independently hydrogen; deuterium; a methyl group; a propyl group; a cyclohexyl group; a phenyl group which is unsubstituted or substituted with a cyano group, an alkyl group, an aryl group or a cycloalkyl group; a biphenyl group which is unsubstituted or substituted with a cyano group, an alkyl group, an aryl group or a cycloalkyl group; a naphthyl group which is unsubstituted or substituted with a cyano group, an alkyl group, an aryl group or a cycloalkyl group; or a pyridyl group which is unsubstituted or substituted with a cyano group, an alkyl group, an aryl group or a cycloalkyl group, or adjacent groups are bonded to each other to form a benzene ring.

In an exemplary embodiment of the present specification, R1 to R5 are the same as or different from each other, and are each independently hydrogen; deuterium; a methyl group; an i-propyl group; a cyclohexyl group; a phenyl group which is unsubstituted or substituted with an alkyl group or a cycloalkyl group; a biphenyl group which is unsubstituted or substituted with a cyano group; a naphthyl group; or a pyridyl group which is unsubstituted or substituted with an alkyl group, or adjacent groups are bonded to each other to form a benzene ring.

In an exemplary embodiment of the present specification, R1 to R5 are the same as or different from each other, and are each independently hydrogen; deuterium; a methyl group; an i-propyl group; a cyclohexyl group; a phenyl group which is unsubstituted or substituted with a methyl group, a t-butyl group or a cyclohexyl group; a biphenyl group which is unsubstituted or substituted with a cyano group; a naphthyl group; or a pyridyl group which is unsubstituted or substituted with a methyl group, or adjacent groups are bonded to each other to form a benzene ring.

In an exemplary embodiment of the present specification, R1 to R5 are the same as or different from each other, and are each independently hydrogen; a methyl group; an i-propyl group; a cyclohexyl group; a phenyl group which is unsubstituted or substituted with a methyl group, a t-butyl group or a cyclohexyl group; a biphenyl group which is unsubstituted or substituted with a cyano group; a naphthyl group; or a pyridyl group which is unsubstituted or substituted with a methyl group, or adjacent groups are bonded to each other to form a benzene ring.

In an exemplary embodiment of the present specification, R1 to R5 are the same as or different from each other, and are each independently hydrogen; a methyl group; a phenyl group which is unsubstituted or substituted with a t-butyl group; a biphenyl group which is unsubstituted or substituted with a cyano group; or a pyridyl group, or adjacent groups are bonded to each other to form a benzene ring.

In an exemplary embodiment of the present specification, R3 and R4 are bonded to each other to form a substituted or unsubstituted benzene ring.

In an exemplary embodiment of the present specification, R3 and R4 are bonded to each other to form a benzene ring which is unsubstituted or substituted with a cyano group, an alkyl group, an aryl group or a cycloalkyl group.

In an exemplary embodiment of the present specification, R3 and R4 are bonded to each other to form a benzene ring.

In an exemplary embodiment of the present specification, R4 and R5 are bonded to each other to form a substituted or unsubstituted benzene ring.

In an exemplary embodiment of the present specification, R4 and R5 are bonded to each other to form a benzene ring which is unsubstituted or substituted with a cyano group, an alkyl group, an aryl group or a cycloalkyl group.

In an exemplary embodiment of the present specification, R4 and R5 are bonded to each other to form a benzene ring.

In an exemplary embodiment of the present specification, when adjacent groups in R1 to R5 are bonded to each other to form no substituted or unsubstituted aromatic hydrocarbon ring, at least two of R1 to R5 are not hydrogen.

In an exemplary embodiment of the present specification, when adjacent groups in R1 to R5 are bonded to each other to form no substituted or unsubstituted aromatic hydrocarbon ring, at least two of R1 to R5 are not hydrogen; or deuterium.

In an exemplary embodiment of the present specification, when adjacent groups in R1 to R5 are bonded to each other to form no substituted or unsubstituted aromatic hydrocarbon ring, at least three of R1 to R5 are not hydrogen.

In an exemplary embodiment of the present specification, when adjacent groups in R1 to R5 are bonded to each other to form no substituted or unsubstituted aromatic hydrocarbon ring, at least three of R1 to R5 are not hydrogen; or deuterium.

In an exemplary embodiment of the present specification, Chemical Formula A is represented by any one of the following Structural Formulae A-1 to A-8.

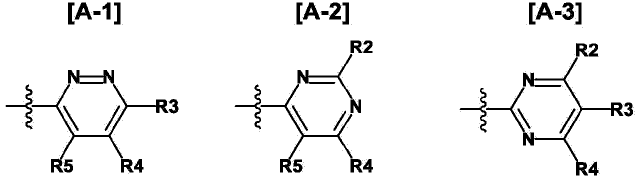

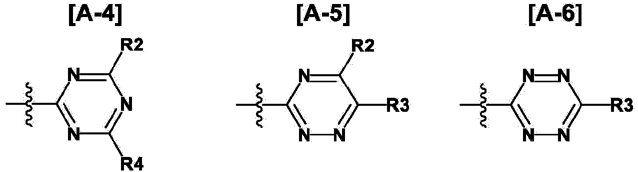

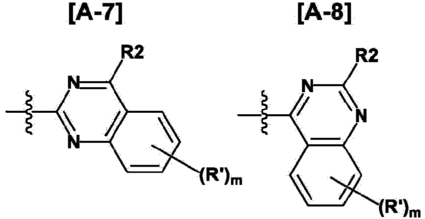

In Structural Formulae A-1 to A-8, the definitions of R2 to R5 are the same as the definitions in Chemical Formula A,
R' is hydrogen; deuterium; a cyano group; a substituted or unsubstituted alkyl group; a substituted or unsubstituted cycloalkyl group; a substituted or unsubstituted aryl group; or a substituted or unsubstituted heterocyclic group,
m is an integer from 0 to 4, and when m is 2 or higher, two or more R"s are the same as or different from each other, and 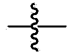 means a position bonded to Chemical Formula 1.

In an exemplary embodiment of the present specification, Ar1 and Ar2 are the same as or different from each other, and are each independently represented by any one of the following Structural Formulae A-1 to A-8.

In an exemplary embodiment of the present specification, Ar1 and Ar2 are the same as or different from each other, and are each independently represented by any one of Structural Formulae A-1 to A-4, A-7 and A-8.

In an exemplary embodiment of the present specification, Chemical Formula 1 is represented by any one of the following Chemical Formulae 1-1 to 1-6.

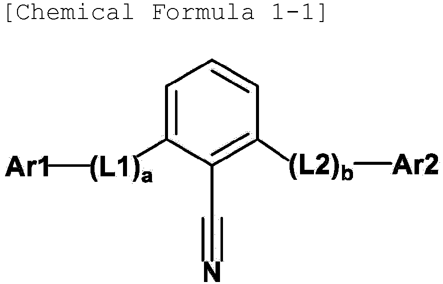

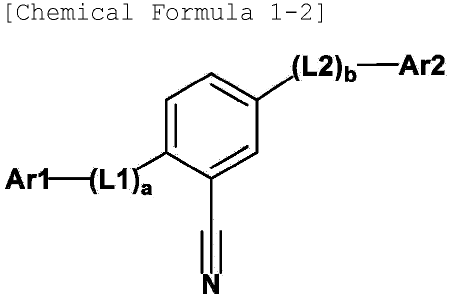

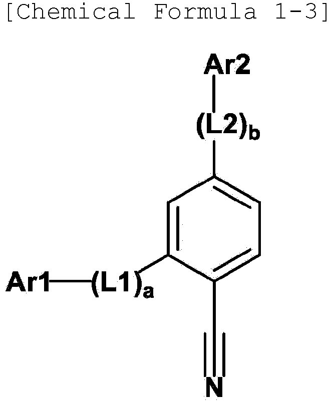

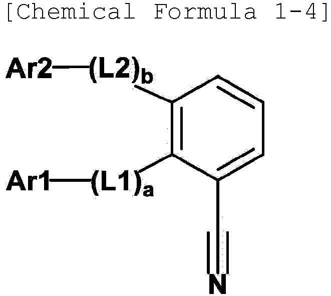

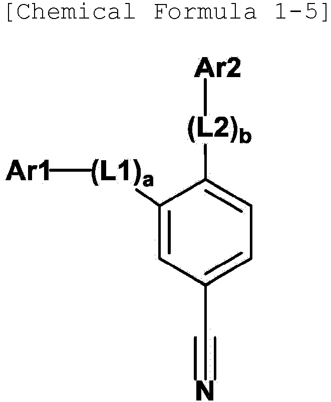

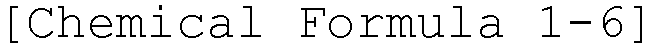

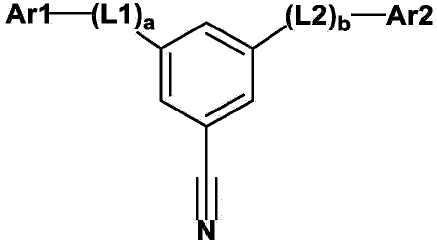

In Chemical Formulae 1-1 to 1-6, the definitions of L1, L2, Ar1, Ar2, a and b are the same as the definitions in Chemical Formula 1.

In an exemplary embodiment of the present specification, Chemical Formula 1 is represented by any one of the following Chemical Formulae 2-1 to 2-6.

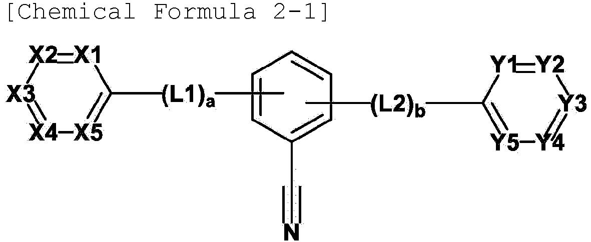

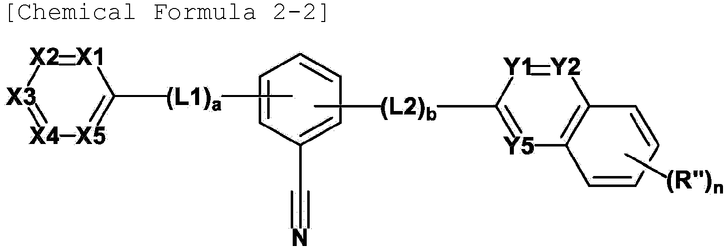

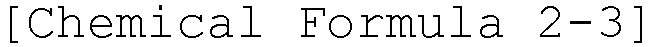

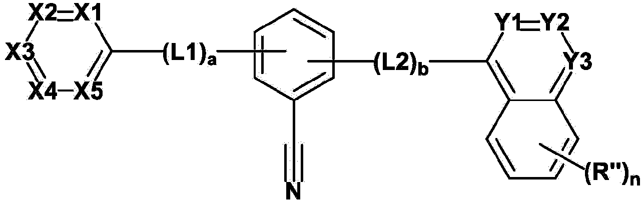

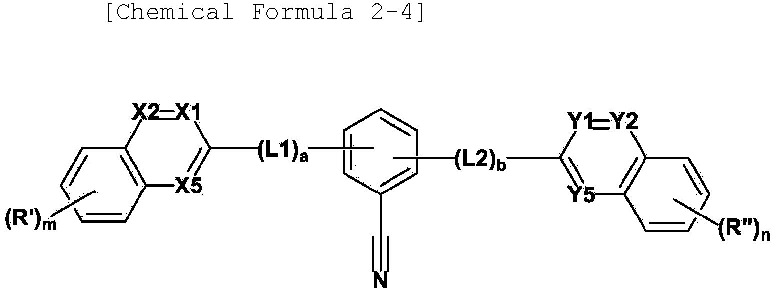

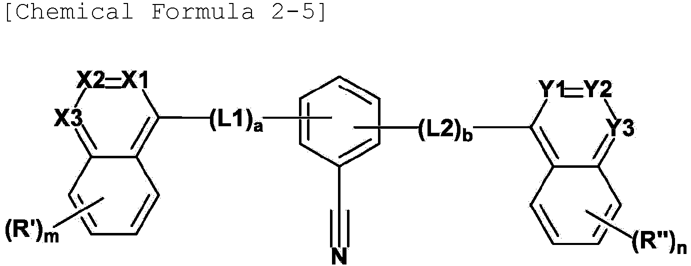

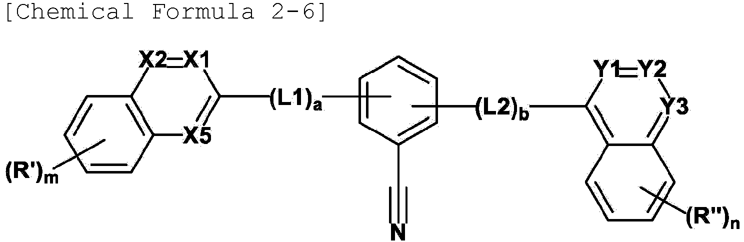

In Chemical Formulae 2-1 to 2-6, the definitions of X1 to X5, L1, L2, a and b are the same as the definitions in Chemical Formula 1,
Y1 is N or CR11, Y2 is N or CR12, Y3 is N or CR13, Y4 is N or CR14, and Y5 is N or CR15,
at least two of Y1 to Y5 are N,
R11 to R15 are the same as or different from each other, and are each independently hydrogen; deuterium; a substituted or unsubstituted alkyl group; a substituted or unsubstituted cycloalkyl group; a substituted or unsubstituted aryl group; or a substituted or unsubstituted heterocyclic group,
R' and R" are the same as or different from each other, and are each independently hydrogen; deuterium; a substituted or unsubstituted alkyl group; a substituted or unsubstituted cycloalkyl group; a substituted or unsubstituted aryl group; or a substituted or unsubstituted heterocyclic group, and
m and n are each an integer from 0 to 4, and when m and n are each 2 or higher, two or more R"s are the same as or different from each other and two or more R‴s are the same as or different from each other.

In an exemplary embodiment of the present specification, R' and R" are the same as or different from each other, and are each independently hydrogen; or deuterium.

In an exemplary embodiment of the present specification, R' and R" are hydrogen.

In an exemplary embodiment of the present specification, m and n are each 0 or 1.

In an exemplary embodiment of the present specification, m and n are 1.

In an exemplary embodiment of the present specification, Chemical Formula 1 is represented by any one of the following Chemical Formulae 3-1 to 3-3.

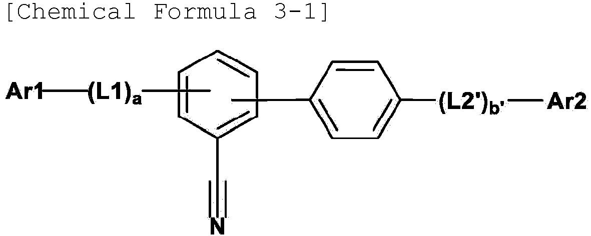

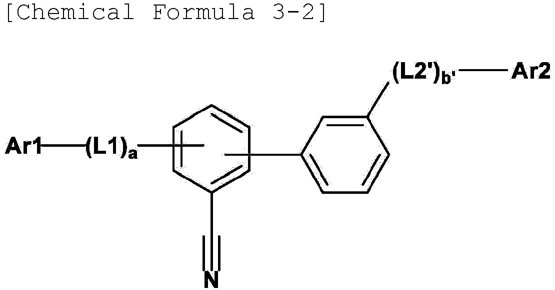

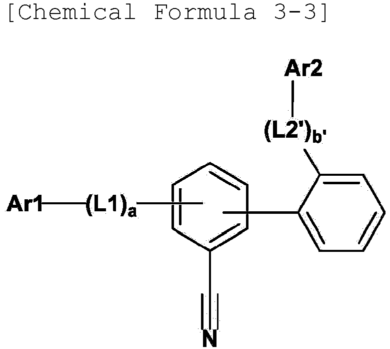

In Chemical Formulae 3-1 to 3-3, the definitions of L1, Ar1, Ar2, and a are the same as the definitions in Chemical Formula 1,
L2' is an arylene group which is unsubstituted or substituted with an aryl group,
b' is an integer from 0 to 2, and
when b' is 2, two L2"s are the same as or different from each other.

In an exemplary embodiment of the present specification, the definition of L2' is the same as the above-described definition of L2.

In an exemplary embodiment of the present specification, b' is 0 or 1.

In an exemplary embodiment of the present specification, b' is 0.

In an exemplary embodiment of the present specification, b' is 1.

In an exemplary embodiment of the present specification, -(L1)ₐ-Ar1 and -(L2)_{b}-Ar2 or Chemical Formula 1 are the same as each other.

In an exemplary embodiment of the present specification, -(L1)ₐ-Ar1 and -(L2)_{b}-Ar2 of Chemical Formula 1 are different from each other.

In an exemplary embodiment of the present specification, Chemical Formula 1 is represented by any one of the following compounds.

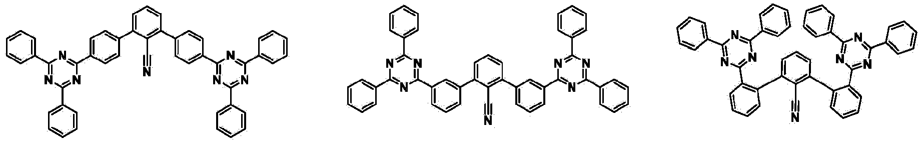

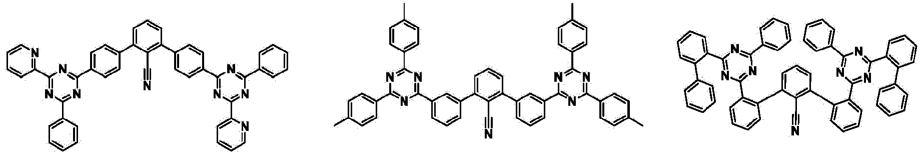

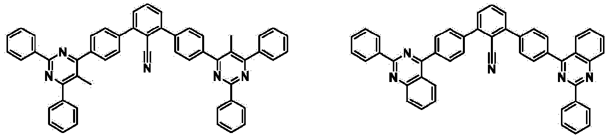

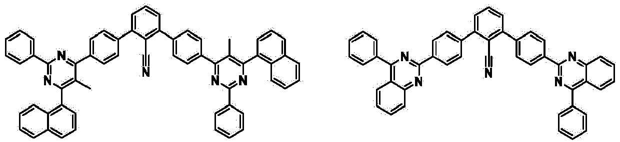

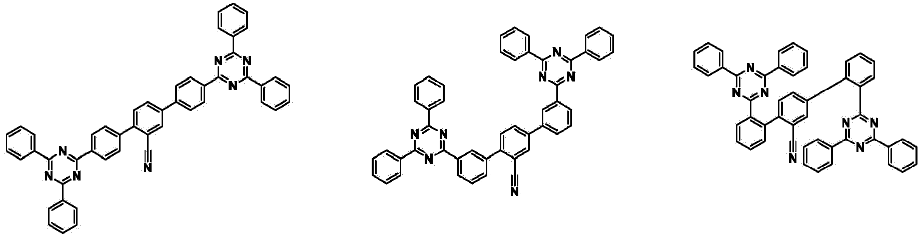

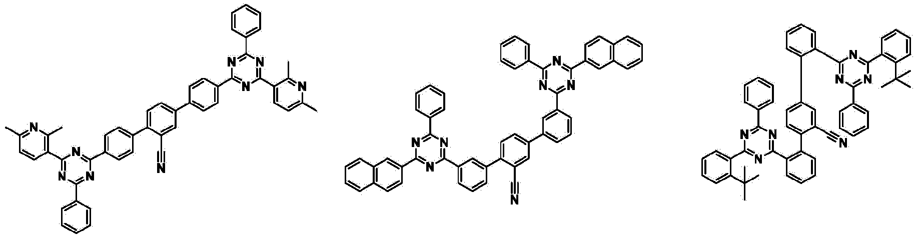

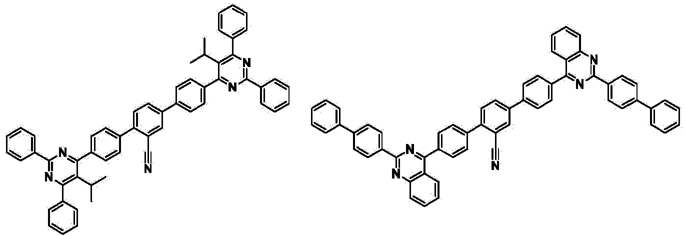

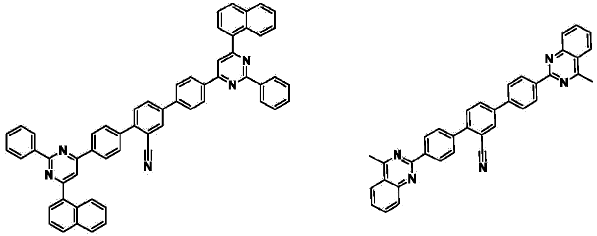

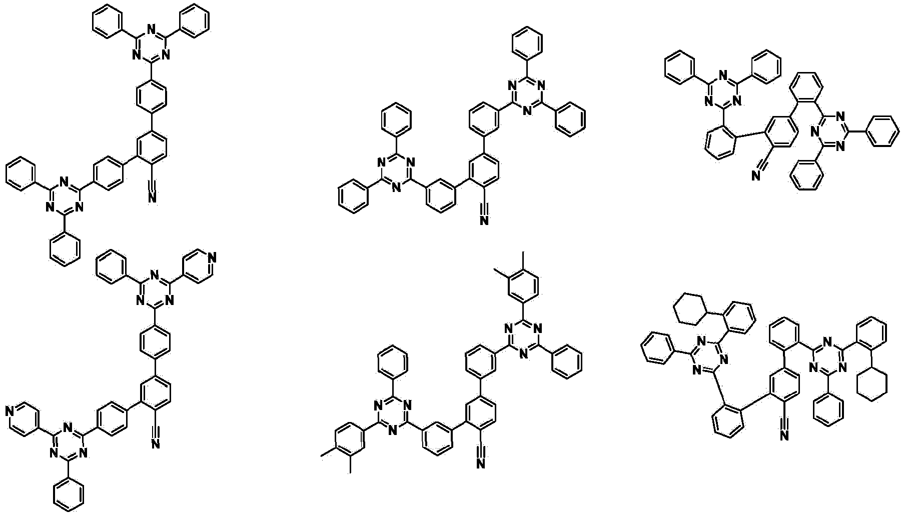

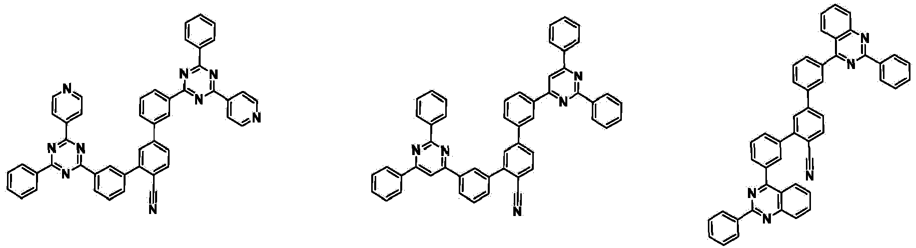

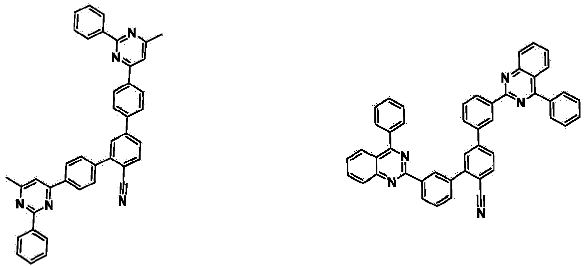

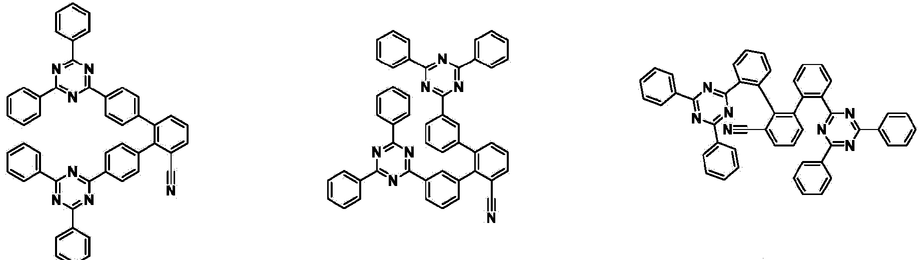

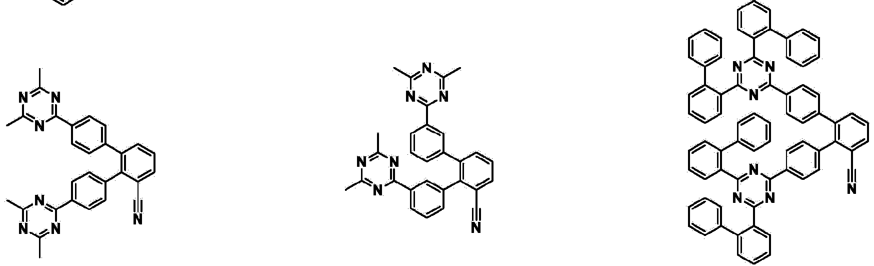

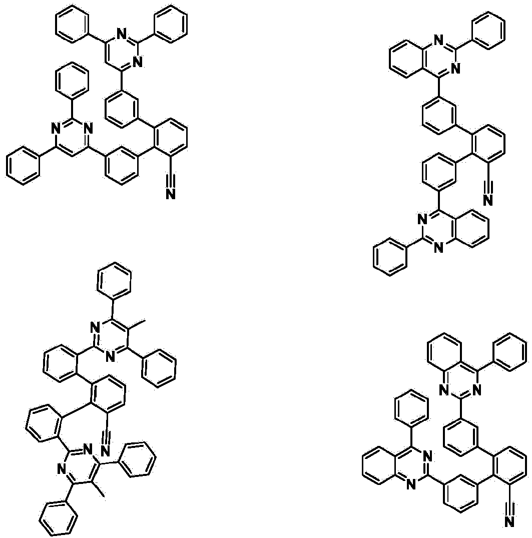

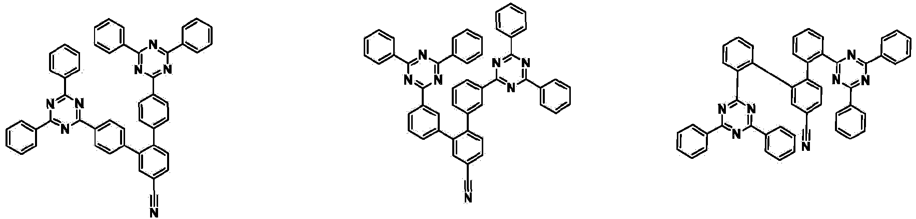

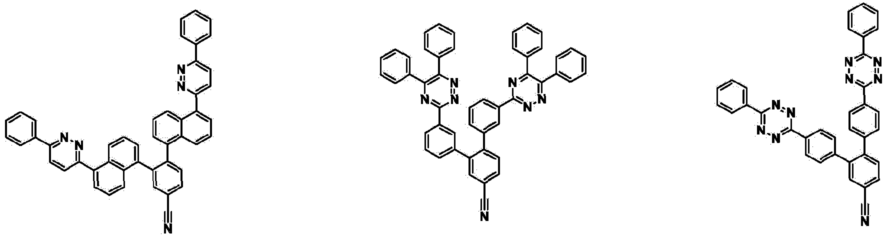

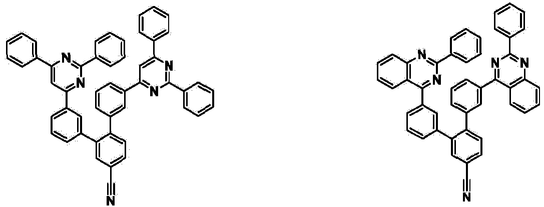

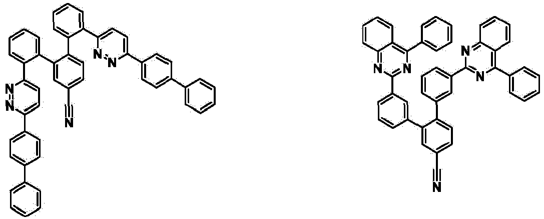

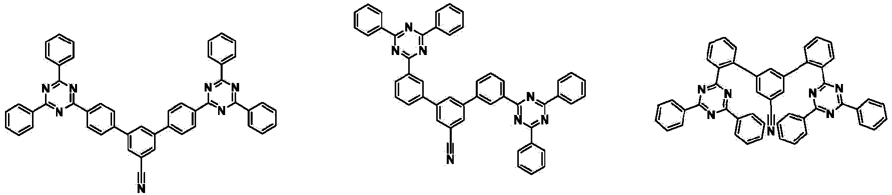

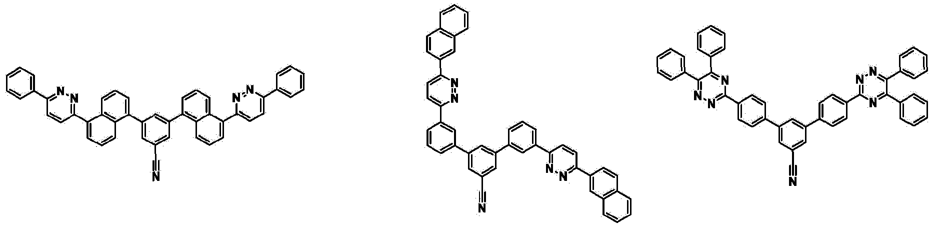

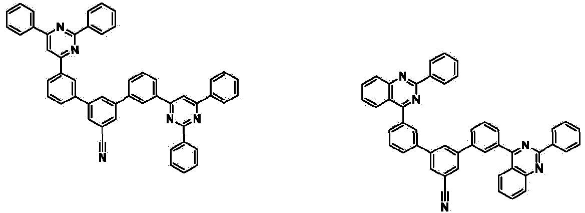

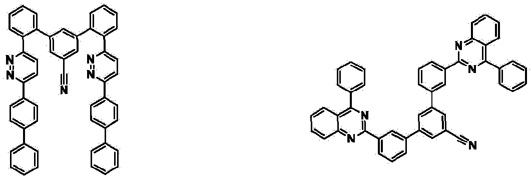

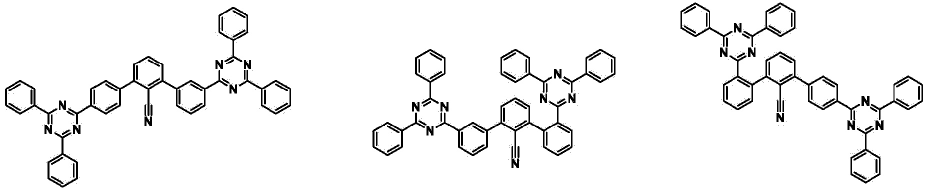

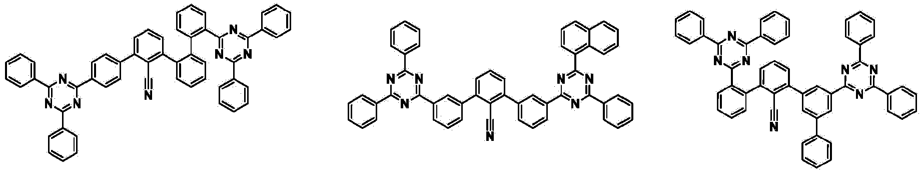

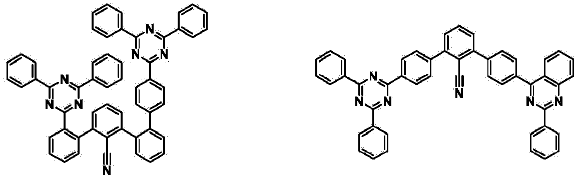

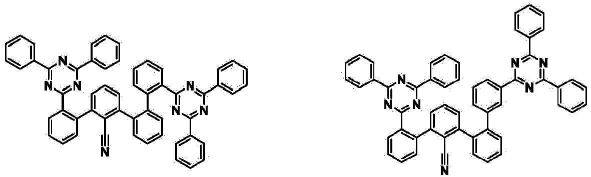

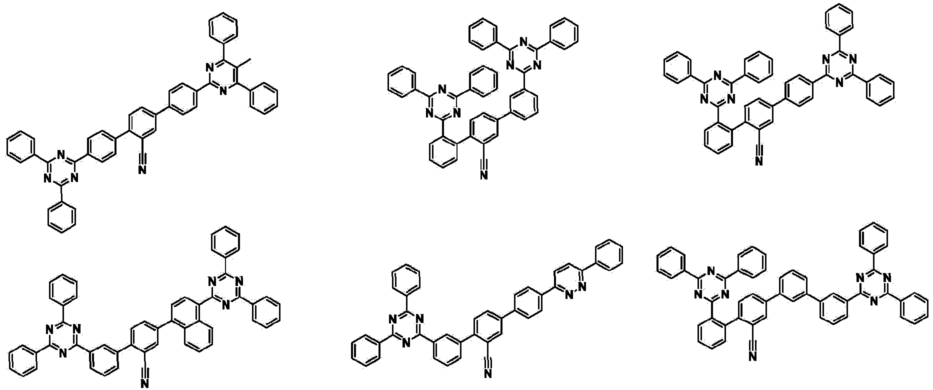

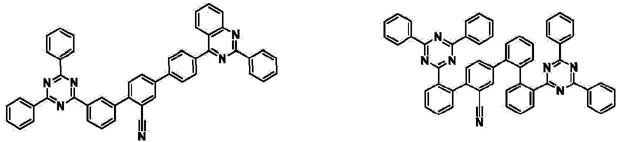

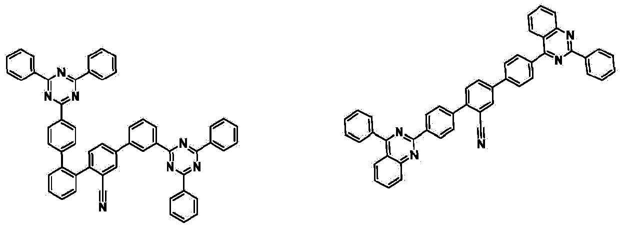

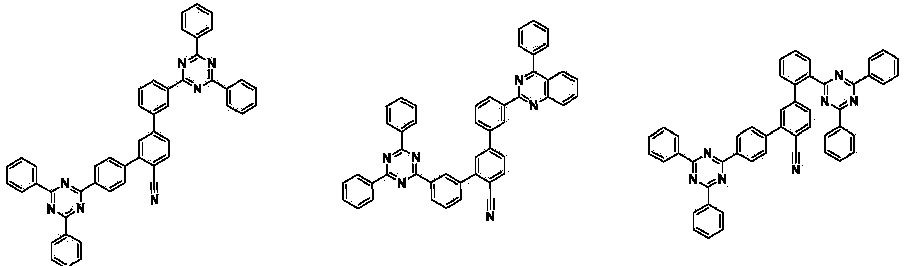

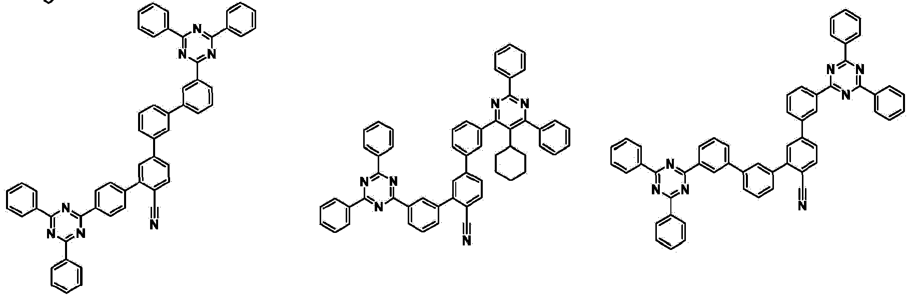

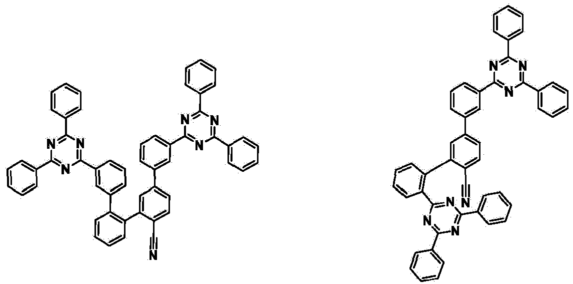

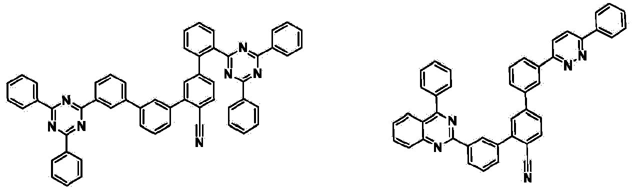

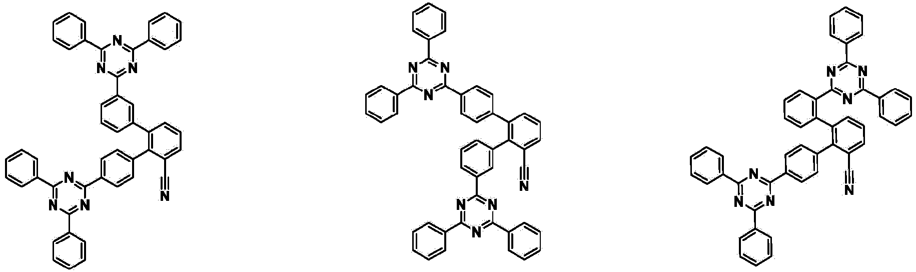

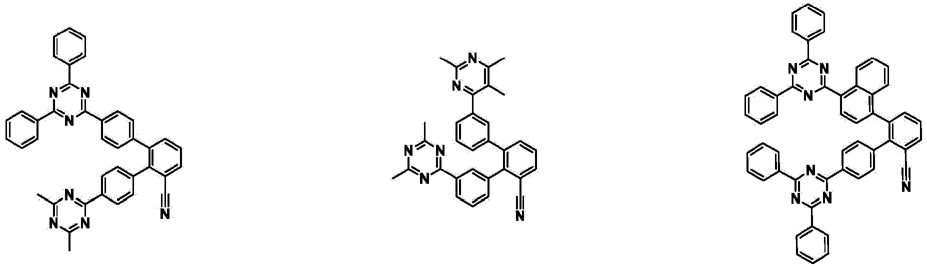

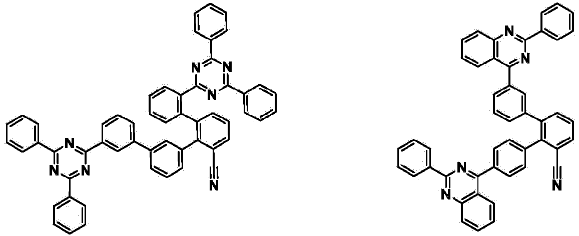

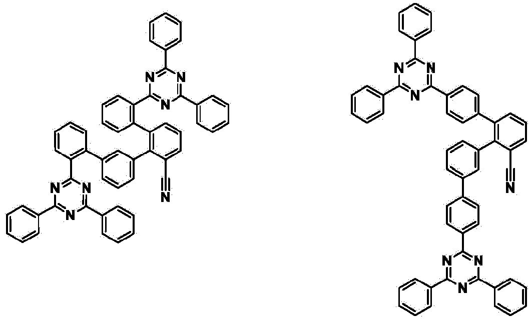

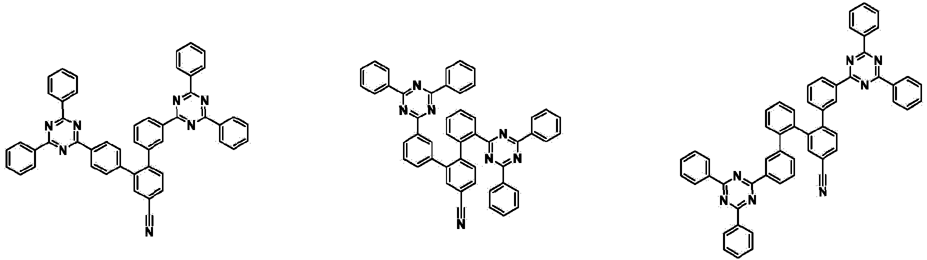

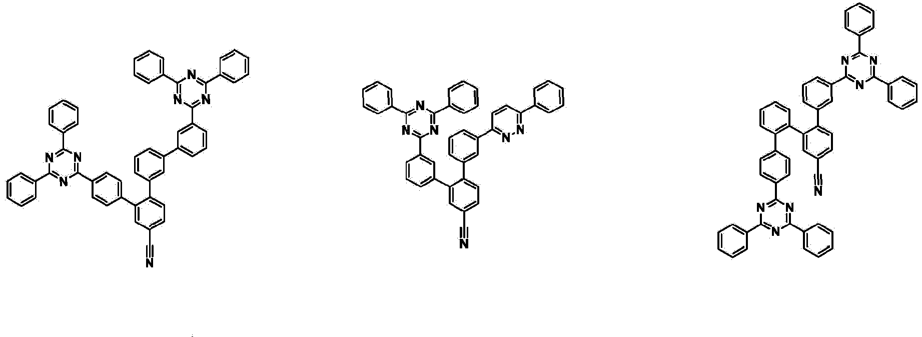

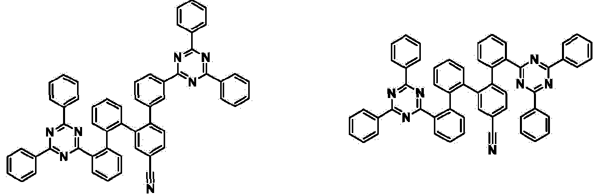

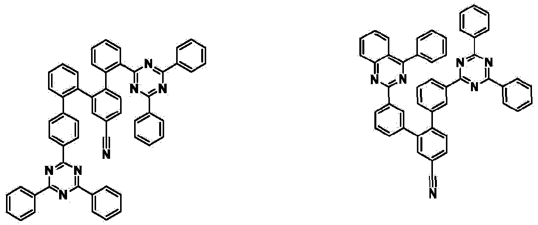

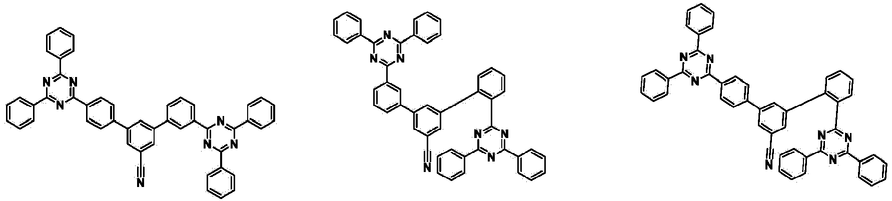

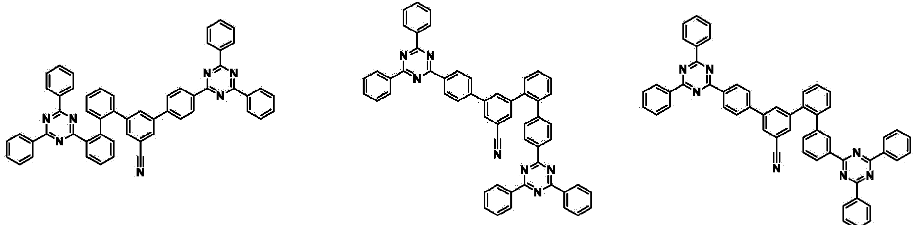

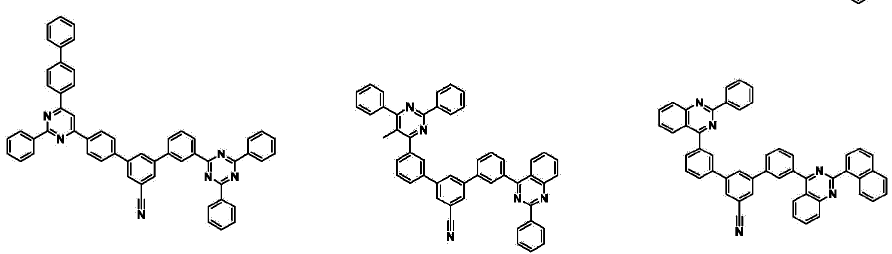

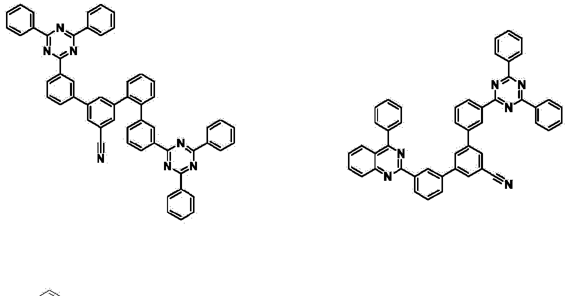

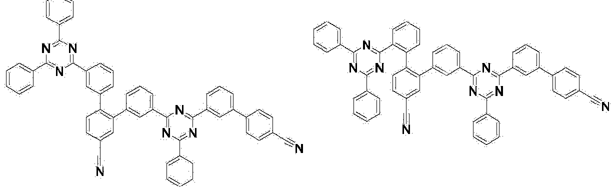

A core structure may be prepared in the same manner as in the following General Formula 1 or 2 from the compound represented by Chemical Formula 1 according to an exemplary embodiment of the present specification. The substituent may be bonded by a method known in the art, and the kind and position of the substituent or the number of substituents may be changed according to the technology known in the art.

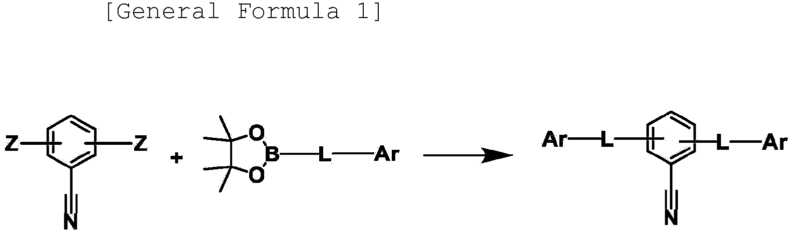

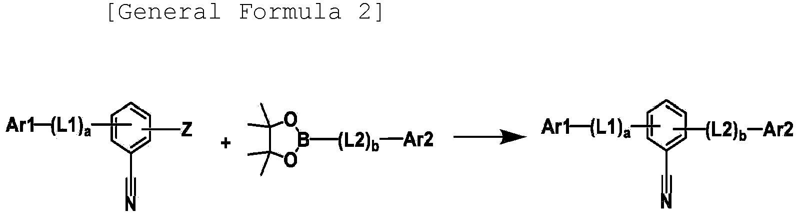

In General Formulae 1 and 2, the definition of the substituent is the same as described above, and Z's are the same as or different from each other, and are each independently a halogen or -SO₃C₄F₉, preferably chloro, bromo, or -SO₃C₄F₉. L's are each independently (L1)ₐ or (L2)_{b}, and Ar's are each independently Ar1 or Ar2. In this case, compounds corresponding to the range of Chemical Formula 1 may be synthesized by a synthesis method known in the art using starting materials, intermediate materials, and the like known in the art.

In the present specification, compounds having various energy band gaps may be synthesized by introducing various substituents into the core structure of the compound represented by Chemical Formula 1. Further, in the present specification, various substituents may be introduced into the core structures having the structure described above to adjust the HOMO and LUMO energy levels of a compound.

The present specification provides a composition for an organic material layer of an organic light emitting device, including the compound represented by Chemical Formula 1; and an n-type dopant or organic metal compound.

In an exemplary embodiment of the present specification, as the n-type dopant or organic metal compound, those known in the art may be used, for example, a metal or a metal complex may be used. For example, the n-type dopant or organic metal compound may be LiQ, and is not limited thereto.

According to an example, the compound represented by Chemical Formula 1 and the n-type dopant or organic metal compound may be included in the composition at a weight ratio of 2:8 to 8:2, for example, 4:6 to 6:4. According to an example, the compound represented by Chemical Formula 1 and the n-type dopant or organic metal compound may be included at a weight ratio of 1:1.

Further, the present specification provides an organic light emitting device including the above-described compound.

The organic light emitting device according to the present specification is an organic light emitting device including: an anode; a cathode; and an organic material layer having one or more layers provided between the anode and the cathode, in which one or more layers of the organic material layer include the above-described compound represented by Chemical Formula 1.

The organic light emitting device of the present specification may be manufactured using typical manufacturing methods and materials of an organic light emitting device, except that the above-described compound of Chemical Formula 1 is used to form an organic material layer.

The compound may be formed as an organic material layer by not only a vacuum deposition method, but also a solution application method when an organic light emitting device is manufactured. Here, the solution application method means spin coating, dip coating, inkjet printing, screen printing, a spray method, roll coating, and the like, but is not limited thereto.

The organic material layer of the organic light emitting device of the present specification may also be composed of a single-layered structure, but may be composed of a multi-layered structure in which two or more organic material layers are stacked. For example, the organic light emitting device of the present specification may have a structure including one or more layers of a hole transport layer, a hole injection layer, an electron blocking layer, a hole transport and injection layer, an electron transport layer, an electron injection layer, a hole blocking layer, and an electron transport and injection layer as organic material layers. However, the structure of the organic light emitting device of the present specification is not limited thereto, and may include a fewer or greater number of organic material layers.

In the organic light emitting device of the present specification, the organic material layer includes a hole injection layer, a hole transport layer, or a hole injection and transport layer, and the hole injection layer, the hole transport layer, or the hole injection and transport layer may include the above-described compound represented by Chemical Formula 1.

In the organic light emitting device of the present specification, the organic material layer includes a hole transport layer or a hole injection layer, and the hole transport layer or the hole injection layer may include the above-described compound represented by Chemical Formula 1.

In an exemplary embodiment of the present specification, the organic material layer includes an electron injection layer, an electron transport layer, an electron transport and injection layer, or a hole blocking layer, and the electron injection layer, the electron transport layer, the electron transport and injection layer, or the hole blocking layer may include the above-described compound represented by Chemical Formula 1.

In the organic light emitting device of the present specification, the organic material layer includes an electron transport layer, an electron injection layer, or an electron transport and injection layer, and the electron transport layer, the electron injection layer, or the electron transport and injection layer may include the above-described compound represented by Chemical Formula 1.

In an exemplary embodiment of the present specification, the organic material layer includes an electron adjusting layer, and the electron adjusting layer may include the above-described compound represented by Chemical Formula 1.

In an exemplary embodiment of the present specification, the organic material layer includes a hole blocking layer, and the hole blocking layer includes the compound represented by Chemical Formula 1.

In the organic light emitting device of the present specification, the organic material layer includes an electron transport and injection layer, and the electron transport and injection layer includes the above-described compound represented by Chemical Formula **1.**

In an exemplary embodiment of the present specification, the organic material layer including the compound of Chemical Formula 1 has a thickness of 50 Å to 600 Å, preferably 100 Å to 500 Å, and more preferably 200 Å to 400 Å.

In an exemplary embodiment of the present specification, the organic material layer includes a light emitting layer, and the light emitting layer includes the above-described compound represented by Chemical Formula **1.**

In an exemplary embodiment of the present specification, the organic material layer includes a light emitting layer, and the light emitting layer includes the above-described compound represented by Chemical Formula 1 as a host.

In an exemplary embodiment of the present specification, the organic material layer includes a light emitting layer, and the light emitting layer includes the above-described compound represented by Chemical Formula 1 as a dopant.

In another exemplary embodiment, the organic material layer may further include other organic compounds, metals or metal compounds in addition to the above-described compound represented by Chemical Formula 1.

In the organic light emitting device according to an exemplary embodiment of the present specification, the light emitting layer further includes a fluorescent dopant or a phosphorescent dopant. In this case, the dopant in the light emitting layer is included in an amount of 1 part by weight to 50 parts by weight with respect to 100 parts by weight of a host.

As another example, the organic material layer includes a light emitting layer, and the light emitting layer includes the compound represented by Chemical Formula 1 as a host, and may further include an additional host.

In an exemplary embodiment of the present specification, the dopant includes an arylamine-based compound, a heterocyclic compound including boron and nitrogen, or an Ir complex, and the like.

The organic light emitting device of the present specification may further include an organic material layer having one or more layers of a hole transport layer, a hole injection layer, an electron blocking layer, an electron transport and injection layer, an electron transport layer, an electron injection layer, a hole blocking layer, and a hole transport and injection layer.

In an exemplary embodiment of the present specification, the organic light emitting device includes: an anode; a cathode; and an organic material layer having two or more layers provided between the anode and the cathode, and at least one of the organic material layer having two or more layers includes the compound represented by Chemical Formula **1.**

In an exemplary embodiment of the present specification, as the organic material layer having two or more layers, two or more may be selected from the group consisting of a light emitting layer, a hole transport layer, a hole injection layer, a hole transport and injection layer, and an electron blocking layer.

In an exemplary embodiment of the present specification, as the organic material layer having two or more layers, two or more may be selected from the group consisting of a light emitting layer, an electron transport layer, an electron injection layer, an electron transport and injection layer, an electron adjusting layer and a hole blocking layer.

In an exemplary embodiment of the present specification, the organic material layer includes an electron transport layer having two or more layers, and at least one of the two or more electron transport layers includes the compound represented by Chemical Formula 1. Specifically, in an exemplary embodiment of the present specification, the compound represented by Chemical Formula 1 may also be included in one layer of the electron transport layer having two or more layers, and may be included in each of the electron transport layer having two or more layers.

Further, in an exemplary embodiment of the present specification, when the compound is included in each of the electron transport layer having two or more layers, the other materials except for the compound represented by Chemical Formula 1 may be the same as or different from each other.

When the organic material layer including the compound represented by Chemical Formula 1 is an electron transport layer, an electron injection layer, or an electron transport and injection layer, the electron transport layer, the electron injection layer, or the electron transport and injection layer may further include an n-type dopant or organic metal compound. As the n-type dopant or organic metal compound, those known in the art may be used, for example, a metal or a metal complex may be used.

For example, the n-type dopant or organic metal compound may be LiQ, and is not limited thereto. An electron transport layer, an electron injection layer, or an electron transport and injection layer, which includes the compound represented by Chemical Formula 1, may further include lithium quinolate (LiQ).

According to an example, the compound represented by Chemical Formula 1 and the n-type dopant or organic metal compound may be included at a weight ratio of 2:8 to 8:2, for example, 4:6 to 6:4. According to an example, the compound represented by Chemical Formula 1 and the n-type dopant or organic metal compound may be included at a weight ratio of 1:1.

In an exemplary embodiment of the present specification, the organic material layer includes a hole transport layer having two or more layers, and at least one of the hole transport layer having two or more layers includes the compound represented by Chemical Formula 1. Specifically, in an exemplary embodiment of the present specification, the compound represented by Chemical Formula 1 may also be included in one layer of the hole transport layer having two or more layers, and may be included in each of the hole transport layer having two or more layers.

In addition, in an exemplary embodiment of the present specification, when the compound represented by Chemical Formula 1 is included in each of the hole transport layer having two or more layers, the other materials except for the compound represented by Chemical Formula 1 may be the same as or different from each other.

In an exemplary embodiment of the present specification, the organic material layer may further include a hole injection layer or a hole transport layer, which includes a compound including an arylamine group, a carbazolyl group, or a benzocarbazolyl group, in addition to the organic material layer including the compound represented by Chemical Formula **1.**

In an exemplary embodiment of the present specification, the organic light emitting device may be a normal type organic light emitting device in which an anode, an organic material layer having one or more layers, and a cathode are sequentially stacked on a substrate.

In an exemplary embodiment of the present specification, the organic light emitting device may be an inverted type organic light emitting device in which a cathode, an organic material layer having one or more layers, and an anode are sequentially stacked on a substrate.

In the organic light emitting device of the present invention, the organic material layer may include an electron blocking layer, and for the electron blocking layer, materials known in the art may be used.

The organic light emitting device may have, for example, the stacking structure described below, but the stacking structure is not limited thereto.
(1) Anode/Hole transport layer/Light emitting layer/Cathode
(2) Anode/Hole injection layer/Hole transport layer/Light emitting layer/Cathode
(3) Anode/Hole injection layer/Hole buffer layer/Hole transport layer/Light emitting layer/Cathode
(4) Anode/Hole transport layer/Light emitting layer/Electron transport layer/Cathode
(5) Anode/Hole transport layer/Light emitting layer/Electron transport layer/Electron injection layer/Cathode
(6) Anode/Hole injection layer/Hole transport layer/Light emitting layer/Electron transport layer/Cathode
(7) Anode/Hole injection layer/Hole transport layer/Light emitting layer/Electron transport layer/Electron injection layer/Cathode
(8) Anode/Hole injection layer/Hole buffer layer/Hole transport layer/Light emitting layer/Electron transport layer/Cathode
(9) Anode/Hole injection layer/Hole buffer layer/Hole transport layer/Light emitting layer/Electron transport layer/Electron injection layer/Cathode
(10) Anode/Hole transport layer/Electron blocking layer/Light emitting layer/Electron transport layer/Cathode
(11) Anode/Hole transport layer/Electron blocking layer/Light emitting layer/Electron transport layer/Electron injection layer/Cathode
(12) Anode/Hole injection layer/Hole transport layer/Electron blocking layer/Light emitting layer/Electron transport layer/Cathode
(13) Anode/Hole injection layer/Hole transport layer/Electron blocking layer/Light emitting layer/Electron transport layer/Electron injection layer/Cathode
(14) Anode/Hole transport layer/Light emitting layer/Hole blocking layer/Electron transport layer/Cathode
(15) Anode/Hole transport layer/Light emitting layer/Hole blocking layer/Electron transport layer/Electron injection layer/Cathode
(16) Anode/Hole injection layer/Hole transport layer/Light emitting layer/Hole blocking layer/Electron transport layer/Cathode
(17) Anode/Hole injection layer/Hole transport layer/Light emitting layer/Hole blocking layer/Electron transport layer/Electron injection layer/Cathode
(18) Anode/Hole injection layer/First hole transport layer/Second hole transport layer/Light emitting layer/Electron injection and transport layer/Cathode
(19) Anode/Hole injection layer/First hole transport layer/Second hole transport layer/Light emitting layer/Hole blocking layer/Electron injection and transport layer/Cathode
(20) Anode/Hole injection layer/Hole transport layer/Light emitting layer/Electron injection and transport layer/Cathode

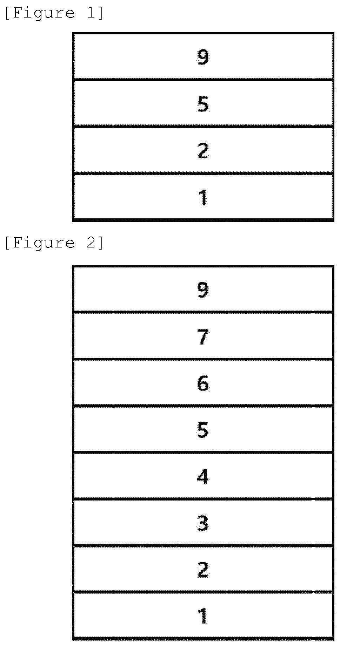

The structure of the organic light emitting device of the present specification may have structures illustrated in FIGS. 1 to 4, but is not limited thereto.
FIG. 1 illustrates an example of an organic light emitting device in which a substrate 1, an anode 2, a light emitting layer 5, and a cathode 9 are sequentially stacked. In the structure described above, the compound may be included in the light emitting layer 5.
FIG. 2 illustrates an example of an organic light emitting device in which a substrate 1, an anode 2, a hole injection layer 3, a hole transport layer 4, a light emitting layer 5, an electron transport layer 6, an electron injection layer 7, and a cathode 9 are sequentially stacked. In the structure described above, the compound may be included in the hole injection layer 3, the hole transport layer 4, the light emitting layer 5, the electron transport layer 6, or the electron injection layer 7.

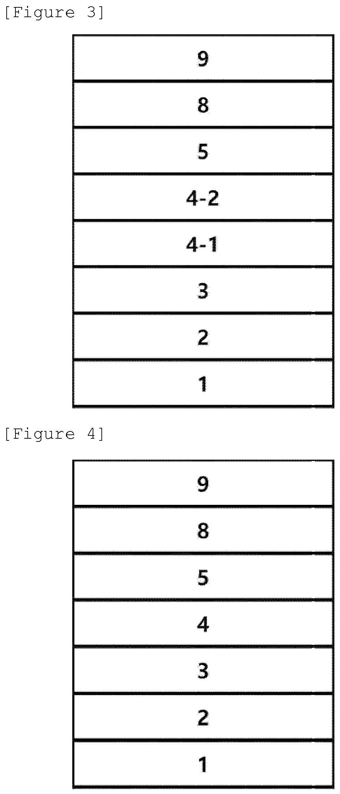

FIG. 3 illustrates an example of an organic light emitting device in which a substrate 1, an anode 2, a hole injection layer 3, a first hole transport layer 4-1, a second hole transport layer 4-2, a light emitting layer 5, an electron transport and injection layer 8, and a cathode 9 are sequentially stacked. In the structure described above, the compound may be included in the hole injection layer 3, the first hole transport layer 4-1, the second hole transport layer 4-2, the light emitting layer 5, or the electron transport and injection layer 8.
FIG. 4 illustrates an example of an organic light emitting device in which a substrate 1, an anode 2, a hole injection layer 3, a hole transport layer 4, a light emitting layer 5, an electron transport and injection layer 8, and a cathode 9 are sequentially stacked. In the structure described above, the compound may be included in the hole injection layer 3, the hole transport layer 4, the light emitting layer 5, or the electron transport and injection layer 8.

In an exemplary embodiment of the present specification, the electron transport and injection layer and the light emitting layer may be provided adjacent to each other.

In an exemplary embodiment of the present specification, the electron transport layer and the light emitting layer may be provided adjacent to each other. In an exemplary embodiment of the present specification, the electron adjusting layer and the light emitting layer may be provided adjacent to each other.

In an exemplary embodiment of the present specification, the hole blocking layer and the light emitting layer may be provided adjacent to each other.

In an exemplary embodiment of the present specification, the hole blocking layer and the electron transport layer may be provided adjacent to each other.

The organic light emitting device of the present specification may be manufactured by the materials and methods known in the art, except that one or more layers of the organic material layer include the compound, that is, the compound represented by Chemical Formula 1.

When the organic light emitting device includes a plurality of organic material layers, the organic material layers may be formed of the same material or different materials.

For example, the organic light emitting device according to the present specification may be manufactured by depositing a metal or a metal oxide having conductivity, or an alloy thereof on a substrate to form an anode, forming an organic material layer including a hole injection layer, a hole transport layer, a light emitting layer, an electron blocking layer, an electron transport layer, and an electron injection layer thereon, and then depositing a material, which may be used as a cathode, thereon, by using a physical vapor deposition (PVD) method such as sputtering or e-beam evaporation. In addition to the method described above, an organic light emitting device may be made by sequentially depositing a cathode material, an organic material layer, and an anode material on a substrate.

The organic material layer may further include one or more layers of a hole injection layer, a hole transport layer, an electron blocking layer, an electron transport and injection layer, an electron transport layer, an electron injection layer, a hole blocking layer, and a hole transport and injection layer.

The organic material layer may further include one or more layers of a hole transport layer, a hole injection layer, an electron blocking layer, an electron transport and injection layer, an electron transport layer, an electron injection layer, a hole blocking layer, and a hole transport and injection layer.

The organic material layer may have a multi-layered structure including a hole injection layer, a hole transport layer, a hole injection and transport layer, an electron blocking layer, a light emitting layer, an electron transport layer, an electron injection layer, an electron transport and injection layer, and the like, but is not limited thereto, and may also have a single-layered structure. Further, the organic material layer may be manufactured to include a fewer number of layers by a method such as a solvent process, for example, spin coating, dip coating, doctor blading, screen printing, inkjet printing, or a thermal transfer method instead of a deposition method, using various polymer materials.

The anode is an electrode which injects holes, and as an anode material, materials having a high work function are usually preferred so as to facilitate the injection of holes into an organic material layer. Specific examples of the anode material which may be used in the present invention include: a metal, such as vanadium, chromium, copper, zinc, and gold, or an alloy thereof; a metal oxide, such as zinc oxide, indium oxide, indium tin oxide (ITO), and indium zinc oxide (IZO); a combination of a metal and an oxide, such as ZnO : Al or SnO₂ : Sb; a conductive polymer, such as poly(3-methylthiophene), poly[3,4-(ethylene-1,2-dioxy)thiophene] (PEDOT), polypyrrole, and polyaniline; and the like, but are not limited thereto.

The cathode is an electrode which injects electrons, and as a cathode material, materials having a low work function are usually preferred so as to facilitate the injection of electrons into an organic material layer. Specific examples of the cathode material include: a metal such as magnesium, calcium, sodium, potassium, titanium, indium, yttrium, lithium, gadolinium, aluminum, silver, tin, and lead, or an alloy thereof; a multi-layer structured material, such as LiF/Al or LiO₂/Al; and the like, but are not limited thereto.

The hole injection layer is a layer which serves to facilitate the injection of holes from an anode to a light emitting layer, and a hole injection material is preferably a material which may proficiently accept holes from an anode at a low voltage, and the highest occupied molecular orbital (HOMO) of the hole injection material is preferably a value between the work function of the anode material and the HOMO of the neighboring organic material layer. Specific examples of the hole injection material include metal porphyrin, oligothiophene, arylamine-based organic materials, hexanitrile hexaazatriphenylene-based organic materials, quinacridone-based organic materials, perylene-based organic materials, anthraquinone, polyaniline-based and polythiophene-based conductive polymers, and the like, but are not limited thereto. The hole injection layer may have a thickness of 1 nm to 150 nm. When the hole injection layer has a thickness of 1 nm or more, there is an advantage in that it is possible to prevent hole injection characteristics from deteriorating, and when the hole injection layer has a thickness of 150 nm or less, there is an advantage in that it is possible to prevent the driving voltage from being increased in order to improve the movement of holes due to the too thick hole injection layer.

In an exemplary embodiment of the present specification, the hole injection layer may include a diamine compound including an aryl group or a heteroaryl group. According to an example, the amine compound may have a structure in which an amine group is bonded to a spiro-acridine-fluorene structure.

The hole transport layer may serve to facilitate the transport of holes. A hole transport material is suitably a material having high hole mobility which may accept holes from an anode or a hole injection layer and transfer the holes to a light emitting layer. Specific examples thereof include arylamine-based organic materials, conductive polymers, block copolymers having both conjugated portions and non-conjugated portions, and the like, but are not limited thereto.

In an exemplary embodiment of the present specification, the hole transport layer may include one or more of an N-containing polycyclic compound including a cyano group or an amine compound including a carbazole group. In this case, the N-containing polycyclic compound may be 1,4,5,8,9,11-hexaazatriphenylenehexacarbonitrile (HATCN). According to an example, the hole transport layer may include the compound alone or may include two or more of the compound. According to another example, the HATCN may be deposited and used as a first hole transport layer, and an amine compound including the carbazole group may be deposited thereon and used as a second hole transport layer.

A hole buffer layer may be additionally provided between a hole injection layer and a hole transport layer, and hole injection or transport materials known in the art may be included.

An electron blocking layer may be provided between a hole transport layer and a light emitting layer. The above-described compound or a material known in the art may be used in the electron blocking layer.

The light emitting layer may emit red, green, or blue light, and may be composed of a phosphorescent material or a fluorescent material. The light emitting material is a material which may receive holes and electrons from a hole transport layer and an electron transport layer, respectively, and combine the holes and the electrons to emit light in a visible ray region, and is preferably a material having high quantum efficiency for fluorescence or phosphorescence. Specific examples thereof include: 8-hydroxy-quinoline aluminum complexes (Alq₃); carbazole-based compounds; dimerized styryl compounds; BAlq; 10-hydroxybenzoquinoline-metal compounds; benzoxazole-based, benzthiazole-based and benzimidazole-based compounds; poly(p-phenylenevinylene) (PPV)-based polymers; spiro compounds; polyfluorene, lubrene, and the like, but are not limited thereto.

Examples of the host material for the light emitting layer include fused aromatic ring derivatives, or hetero ring-containing compounds, and the like. Specifically, examples of the fused aromatic ring derivative include anthracene derivatives, pyrene derivatives, naphthalene derivatives, pentacene derivatives, phenanthrene compounds, fluoranthene compounds, and the like, and examples of the hetero ring-containing compound include carbazole derivatives, dibenzofuran derivatives, ladder-type furan compounds, pyrimidine derivatives, and the like, but the examples thereof are not limited thereto.

When the light emitting layer emits red light, it is possible to use a phosphorescent material such as bis(1-phenylisoquinoline)acetylacetonate iridium (PIQIr(acac)), bis(1-phenylquinoline)acetylacetonate iridium (PQIr(acac)), tris(1-phenylquinoline)iridium (PQIr), or octaethylporphyrin platinum (PtOEP), or a fluorescent material such as tris(8-hydroxyquinolino)aluminum (Alq₃) as a light emitting dopant, but the light emitting dopant is not limited thereto. When the light emitting layer emits green light, it is possible to use a phosphorescent material such as fac tris(2-phenylpyridine)iridium (Ir(ppy)₃), or a fluorescent material such as tris(8-hydroxyquinolino)aluminum (Alq3), as the light emitting dopant, but the light emitting dopant is not limited thereto. When the light emitting layer emits blue light, it is possible to use a phosphorescent material such as (4,6-F2ppy)₂Irpic, or a fluorescent material such as spiro-DPVBi, spiro-6P, distyryl benzene(DSB), distyryl arylene (DSA), a PFO-based polymer or a PPV-based polymer as the light emitting dopant, but the light emitting dopant is not limited thereto.

In an exemplary embodiment of the present specification, the light emitting layer includes an anthracene compound in which an aryl group or a heterocyclic group is substituted as a host, and may include a pyrene compound in which an amine group is substituted as a dopant. According to an example, the anthracene compound may have a structure in which carbons 9 and 10 are substituted with an aryl group or a heterocyclic group. The host and the dopant may be included at an appropriate weight ratio, and according to an example, the host and the dopant may be included at a weight ratio of 100:1 to 100:10, respectively.

A hole blocking layer may be provided between the electron transport layer and the light emitting layer, and materials known in the art may be used.

The electron transport layer may serve to facilitate the transport of electrons. An electron transport material is suitably a material having high electron mobility which may proficiently accept electrons from a cathode and transfer the electrons to a light emitting layer. Specific examples thereof include: Al complexes of the above-described compound or 8-hydroxyquinoline; complexes including Alq₃; organic radical compounds; hydroxyflavone-metal complexes; and the like, but are not limited thereto. The electron transport layer may have a thickness of 1 nm to 50 nm. When the electron transport layer has a thickness of 1 nm or more, there is an advantage in that it is possible to prevent electron transport characteristics from deteriorating, and when the electron transport layer has a thickness of 50 nm or less, there is an advantage in that it is possible to prevent the driving voltage from being increased in order to improve the movement of electrons due to the too thick electron transport layer.

In an exemplary embodiment of the present specification, the electron transport layer may include a compound represented by Chemical Formula 1 of the present invention, and may further include an n-type dopant or organic metal compound. According to an example, the n-type dopant or organic metal compound may be LiQ, and the compound represented by Chemical Formula 1 of the present invention and the n-type dopant (or organic metal compound) may be included at a weight ratio of 2:8 to 8:2, for example, 4:6 to 6:4.

The electron injection layer may serve to facilitate the injection of electrons. An electron injection material is preferably a compound which has a capability of transporting electrons, an effect of injecting electrons from a cathode, and an excellent effect of injecting electrons into a light emitting layer or a light emitting material, prevents excitons produced from a light emitting layer from moving to a hole injection layer, and is also excellent in the ability to form a thin film. Specific examples thereof include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, metal complex compounds, nitrogen-containing 5-membered ring derivatives, and the like, but are not limited thereto.

Examples of the metal complex compounds include 8-hydroxyquinolinato lithium, bis(8-hydroxyquinolinato) zinc, bis(8-hydroxyquinolinato) copper, bis(8-hydroxyquinolinato) manganese, tris(8-hydroxyquinolinato) aluminum, tris(2-methyl-8-hydroxyquinolinato) aluminum, tris(8-hydroxyquinolinato) gallium, bis(10-hydroxybenzo[h]quinolinato) beryllium, bis(10-hydroxybenzo[h]quinolinato) zinc, bis(2-methyl-8-quinolinato) chlorogallium, bis(2-methyl-8-quinolinato) (o-cresolato) gallium, bis(2-methyl-8-quinolinato) (1-naphtholato) aluminum, bis(2-methyl-8-quinolinato) (2-naphtholato) gallium, and the like, but are not limited thereto.

The hole blocking layer is a layer which blocks holes from reaching a cathode, and may be generally formed under the same conditions as those of the hole injection layer. Specific examples thereof include oxadiazole derivatives or triazole derivatives, phenanthroline derivatives, BCP, aluminum complexes, and the like, but are not limited thereto.

The organic light emitting device according to the present invention may be a top emission type, a bottom emission type, or a dual emission type according to the material to be used.

The organic light emitting device according to the present specification may be included and used in various electronic devices. For example, the electronic device may be a display panel, a touch panel, a solar module, a lighting device, and the like, and is not limited thereto.

### [Mode for Invention]

Hereinafter, the present specification will be described in detail with reference to Examples for specifically describing the present specification. However, the Examples according to the present specification may be modified in various forms, and it is not interpreted that the scope of the present application is limited to the Examples described in detail below. The Examples of the present application are provided to explain the present specification more completely to a person with ordinary skill in the art.

### [Synthesis Examples]

### Synthesis Example 1: Preparation of Compound E1

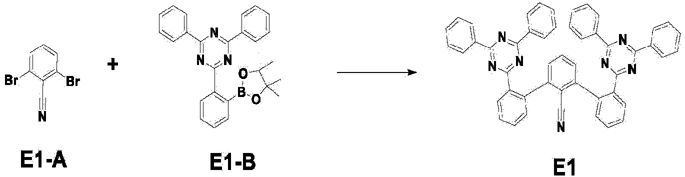

E1-A (20 g, 76.7 mmol) and E1-B (66.7 g, 153.3 mmol) were put into 400 ml of tetrahydrofuran in a nitrogen atmosphere, and the resulting mixture was stirred and refluxed. Thereafter, potassium carbonate (31.8 g, 230 mmol) was dissolved in 32 ml of water, the resulting solution was added thereto, the resulting mixture was sufficiently stirred, and then tetrakistriphenyl-phosphinopalladium (2.7 g, 2.3 mmol) was added thereto. After the reaction for 3 hours, the temperature of the mixture was cooled to room temperature, then an organic layer and an aqueous layer were separated, and then the organic layer was distilled. The distilled organic layer was again added to and dissolved in 1100 mL of chloroform, the solution was washed twice with water, and then the organic layer was separated, anhydrous magnesium sulfate was put thereinto, and the resulting mixture was stirred, and then filtered, and the filtrate was distilled under reduced pressure. The concentrated compound was recrystallized with chloroform and ethyl acetate to prepare a white solid Compound E1 (33 g, 60%).
MS: [M+H]+ = 718

### Synthesis Example 2: Preparation of Compound E2

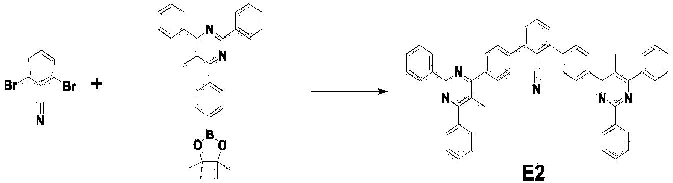

Compound E2 was prepared in the same manner as in the preparation method in Synthesis Example 1, except that each starting material was used as in the reaction formula.
MS: [M+H]+ = 744

### Synthesis Example 3: Preparation of Compound E3

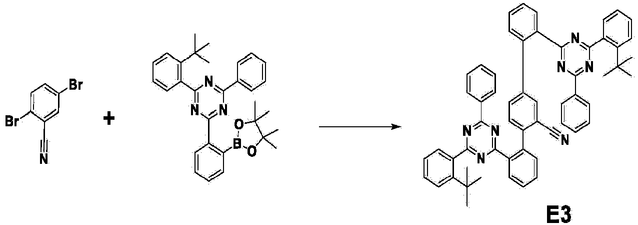

Compound E3 was prepared in the same manner as in the preparation method in Synthesis Example 1, except that each starting material was used as in the reaction formula.
MS: [M+H]+ = 830

### Synthesis Example 4: Preparation of Compound E4

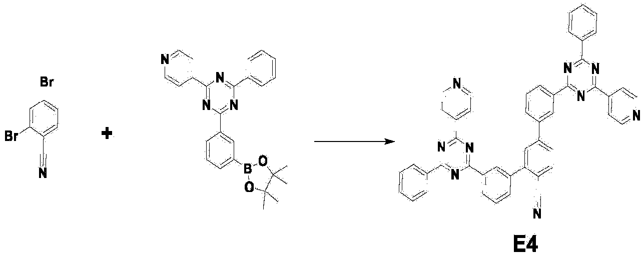

Compound E4 was prepared in the same manner as in the preparation method in Synthesis Example 1, except that each starting material was used as in the reaction formula.
MS: [M+H]+ = 720

### Synthesis Example 5: Preparation of Compound E5

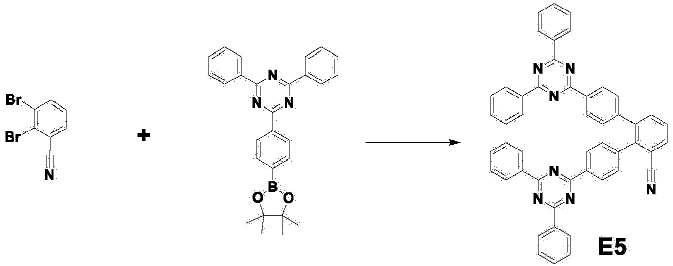

Compound E5 was prepared in the same manner as in the preparation method in Synthesis Example 1, except that each starting material was used as in the reaction formula.
MS: [M+H]+ = 718

### Synthesis Example 6: Preparation of Compound E6

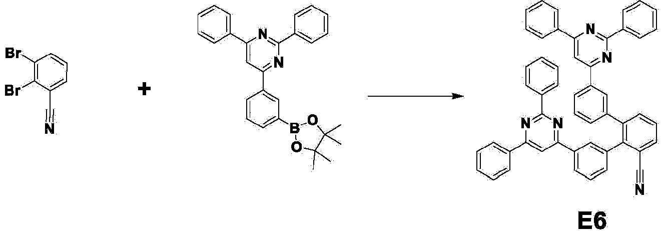

Compound E6 was prepared in the same manner as in the preparation method in Synthesis Example 1, except that each starting material was used as in the reaction formula.
MS: [M+H]+ = 716

### Synthesis Example 7: Preparation of Compound E7

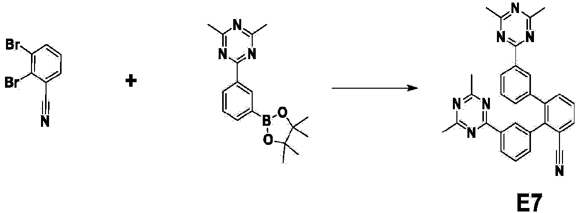

Compound E7 was prepared in the same manner as in the preparation method in Synthesis Example 1, except that each starting material was used as in the reaction formula.
MS: [M+H]+ = 470

### Synthesis Example 8: Preparation of Compound E8

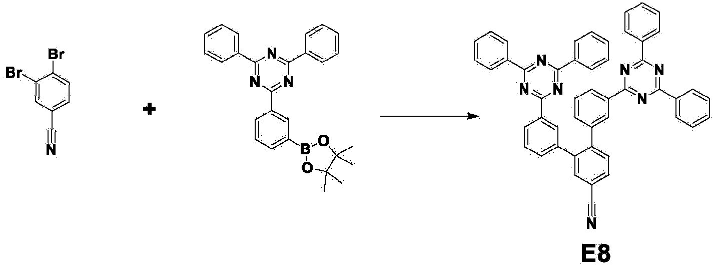

Compound E8 was prepared in the same manner as in the preparation method in Synthesis Example 1, except that each starting material was used as in the reaction formula.
MS: [M+H]+ = 718

### Synthesis Example 9: Preparation of Compound E9

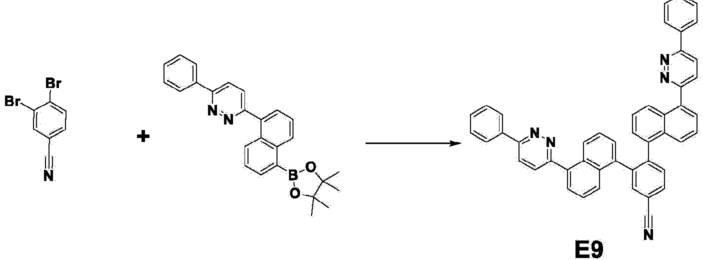

Compound E9 was prepared in the same manner as in the preparation method in Synthesis Example 1, except that each starting material was used as in the reaction formula.
MS: [M+H]+ = 664

### Synthesis Example 10: Preparation of Compound E10

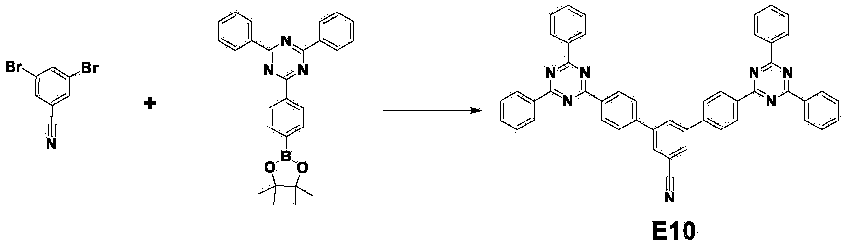

Compound E10 was prepared in the same manner as in the preparation method in Synthesis Example 1, except that each starting material was used as in the reaction formula.
MS: [M+H]+ = 718

### Synthesis Example 11: Preparation of Compound E11

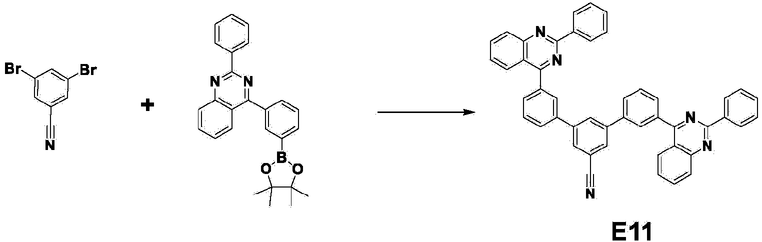

Compound E11 was prepared in the same manner as in the preparation method in Synthesis Example 1, except that each starting material was used as in the reaction formula.
MS: [M+H]+ = 664

### Synthesis Example 12: Preparation of Compound E12

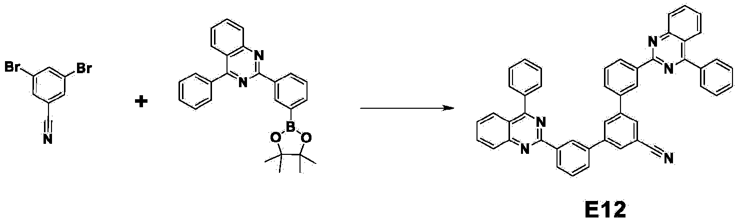

Compound E12 was prepared in the same manner as in the preparation method in Synthesis Example 1, except that each starting material was used as in the reaction formula.
MS: [M+H]+ = 664

### Synthesis Example 13: Preparation of Compound E13

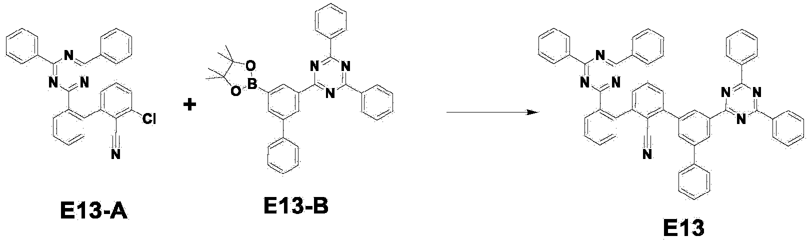

E13-A (20 g, 45 mmol) and E13-B (23 g, 45 mmol) were put into 400 ml of 1,4-dioxane (Diox) in a nitrogen atmosphere, and the resulting mixture was stirred and refluxed. Thereafter, potassium phosphate tribasic (28.6 g, 134.9 mmol) was dissolved in 29 ml of water, the resulting solution was introduced thereinto, the resulting mixture was sufficiently stirred, and then dibenzylideneacetone palladium (0.8 g, 1.3 mmol) and tricyclohexylphosphine (0.8 g, 2.7 mmol) was introduced thereinto. After the reaction for 9 hours, the temperature of the product was lowered to normal temperature and a produced solid was filtered. The solid was added to and dissolved in 1071 mL of 30-fold chloroform, the solution was washed twice with water, and then the organic layer was separated, anhydrous magnesium sulfate was put thereinto, and the resulting mixture was stirred, and then filtered, and the filtrate was distilled under reduced pressure. The concentrated compound was recrystallized with chloroform and ethyl acetate to prepare a yellow solid Compound E13 (5.4 g, 15%).
MS: [M+H]+ = 794

### Synthesis Example 14: Preparation of Compound E14

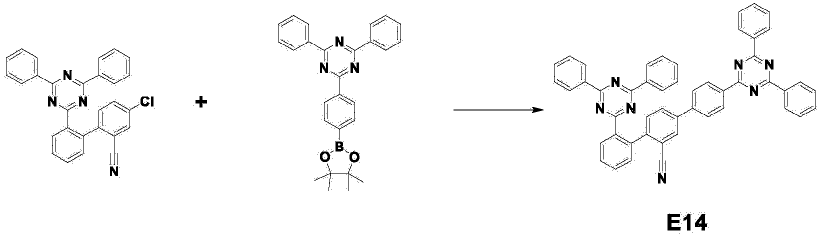

Compound E14 was prepared in the same manner as in the preparation method in Synthesis Example 13, except that each starting material was used as in the reaction formula.
MS: [M+H]+ = 718

### Synthesis Example 15: Preparation of Compound E15

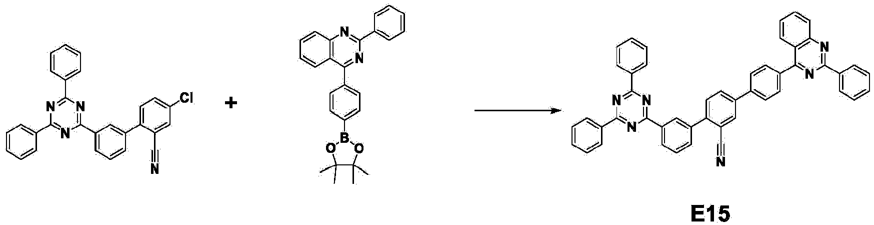

Compound E15 was prepared in the same manner as in the preparation method in Synthesis Example 13, except that each starting material was used as in the reaction formula.
MS: [M+H]+ = 691

### Synthesis Example 16: Preparation of Compound E16

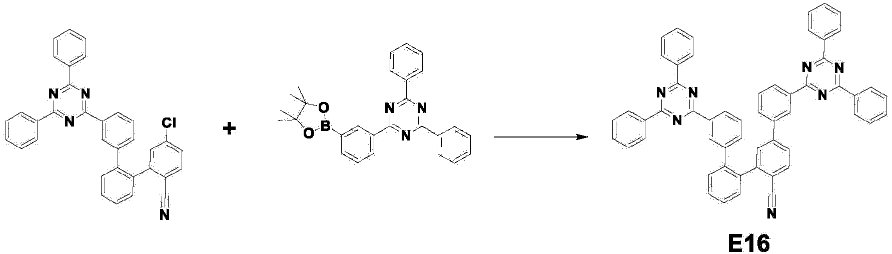

Compound E16 was prepared in the same manner as in the preparation method in Synthesis Example 13, except that each starting material was used as in the reaction formula.
MS: [M+H]+ = 794

### Synthesis Example 17: Preparation of Compound E17

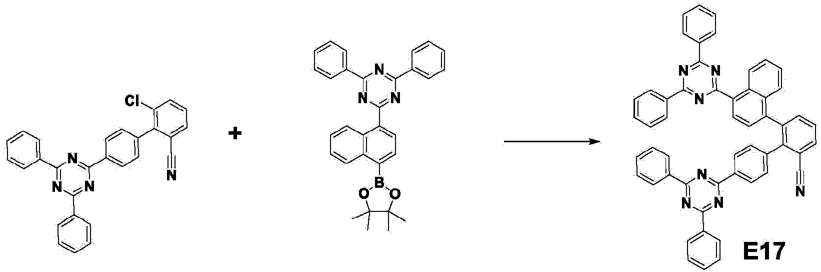

Compound E17 was prepared in the same manner as in the preparation method in Synthesis Example 13, except that each starting material was used as in the reaction formula.
MS: [M+H]+ = 768

### Synthesis Example 18: Preparation of Compound E18

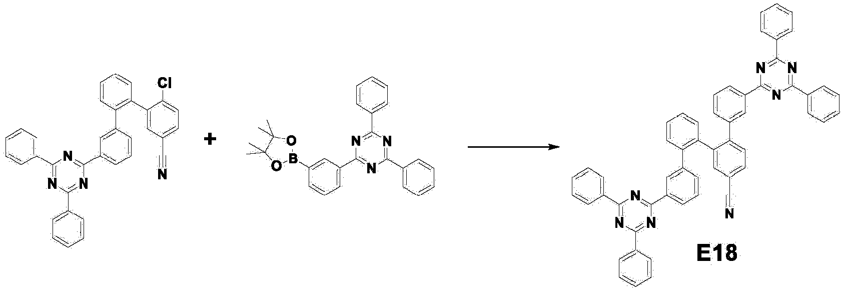

Compound E18 was prepared in the same manner as in the preparation method in Synthesis Example 13, except that each starting material was used as in the reaction formula.
MS: [M+H]+ = 794

### Synthesis Example 19: Preparation of Compound E19

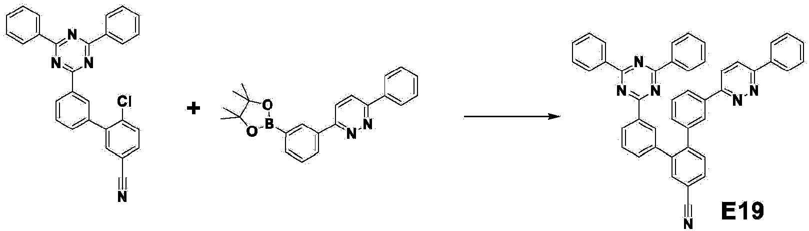

Compound E19 was prepared in the same manner as in the preparation method in Synthesis Example 13, except that each starting material was used as in the reaction formula.
MS: [M+H]+ = 641

### Synthesis Example 20: Preparation of Compound E20

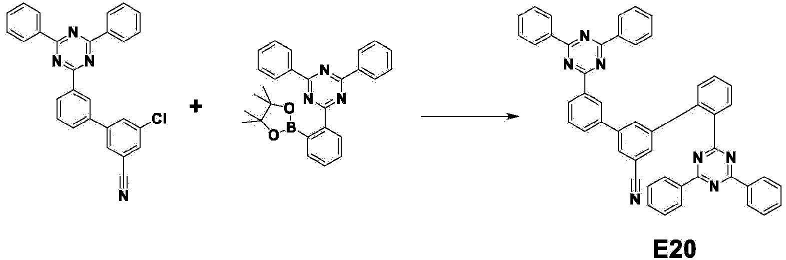

Compound E20 was prepared in the same manner as in the preparation method in Synthesis Example 13, except that each starting material was used as in the reaction formula.
MS: [M+H]+ = 718

### Synthesis Example 21: Preparation of Compound E21

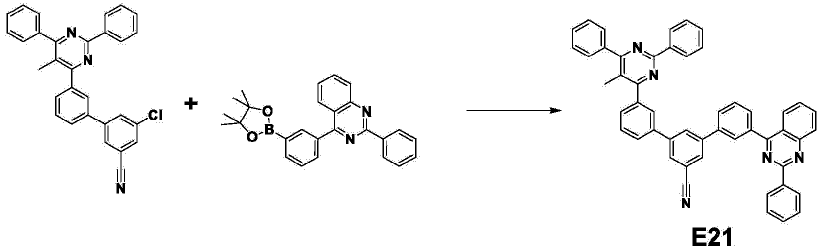

Compound E21 was prepared in the same manner as in the preparation method in Synthesis Example 13, except that each starting material was used as in the reaction formula.
MS: [M+H]+ = 704

### Synthesis Example 22: Preparation of Compound E22

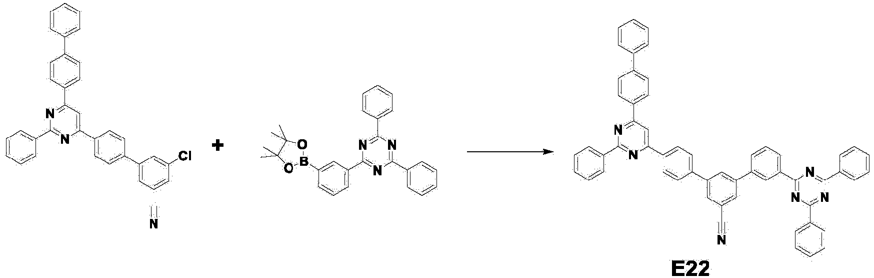

Compound E22 was prepared in the same manner as in the preparation method in Synthesis Example 13, except that each starting material was used as in the reaction formula.
MS: [M+H]+ = 793

### Synthesis Example 23: Preparation of Compound E23

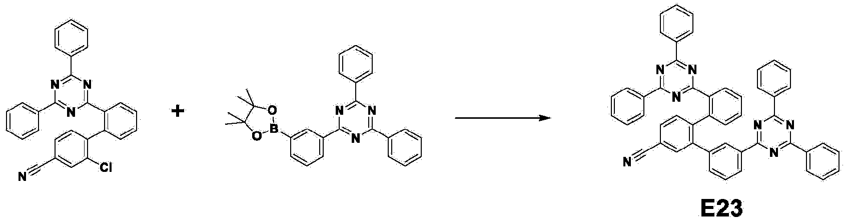

Compound E23 was prepared in the same manner as in the preparation method in Synthesis Example 13, except that each starting material was used as in the reaction formula.
MS: [M+H]+ = 718

### Synthesis Example 24: Preparation of Compound E24

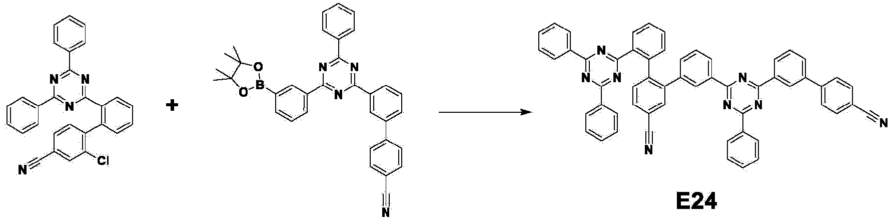

Compound E24 was prepared in the same manner as in the preparation method in Synthesis Example 13, except that each starting material was used as in the reaction formula.
MS: [M+H]+ = 819

### [Experimental Examples]

### Experimental Example 1

A glass substrate thinly coated with indium tin oxide (ITO) to have a thickness of 1,000 Å was put into distilled water in which a detergent was dissolved, and ultrasonically washed. In this case, a product manufactured by Fischer Co., was used as the detergent, and distilled water twice filtered using a filter manufactured by Millipore Co., was used as the distilled water. After the ITO was washed for 30 minutes, ultrasonic washing was repeated twice by using distilled water for 10 minutes. After the washing using distilled water was completed, ultrasonic washing was conducted by using isopropyl alcohol, acetone, and methanol solvents, and the resulting product was dried and then transported to a plasma washing machine. Furthermore, the substrate was cleaned by using oxygen plasma for 5 minutes, and then was transported to a vacuum deposition machine.

The following Compound HI-A was thermally vacuum deposited to have a thickness of 600 Å on the transparent ITO electrode, which was thus prepared, thereby forming a hole injection layer. Hexanitrile hexaazatriphenylene (HAT, 50 Å) of the following chemical formula and the following compound HT-A (600 Å) were sequentially vacuum deposited on the hole injection layer, thereby forming a hole transport layer.

Subsequently, the following Compounds BH and BD were vacuum deposited at a weight ratio of 25:1 to have a film thickness of 200 Å on the hole transport layer, thereby forming a light emitting layer.

Compound E1 prepared in Synthesis Example 1 and the following compound lithium quinolate (LiQ) were vacuum deposited at a weight ratio of 1:1 on the light emitting layer, thereby forming an electron transport and injection layer having a thickness of 360 Å. Lithium fluoride (LiF) and aluminum were subsequently deposited to have a thickness of 10 Å and 1,000 Å, respectively, on the electron transport and injection layer, thereby forming a negative electrode.

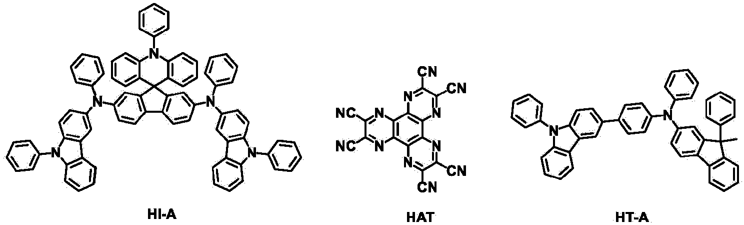

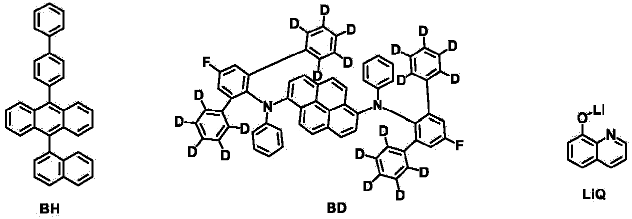

In the aforementioned procedure, the deposition rate of the organic materials was maintained at 0.4 to 0.9 Å/sec, the deposition rates of lithium fluoride and aluminum of the negative electrode were maintained at 0.3 Å/sec and at 2 Å/sec, respectively, and the degree of vacuum during the deposition was maintained at 5 × 10⁻⁸ torr to 1 × 10⁻⁷ torr, thereby manufacturing an organic light emitting device.

### Experimental Examples 2 to 24

Organic light emitting devices were manufactured in the same manner as in Experimental Example 1, except that the compounds in the following Table 1 were used instead of Compound E1.

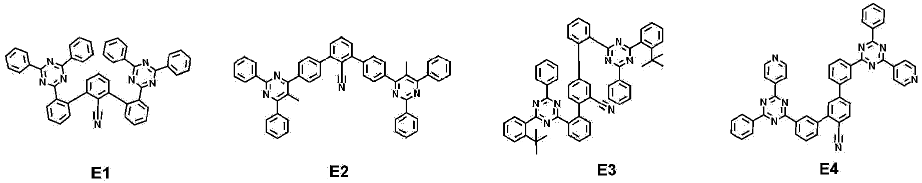

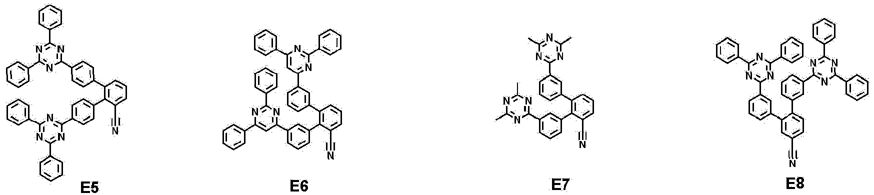

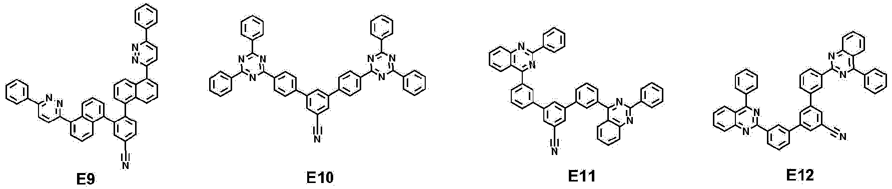

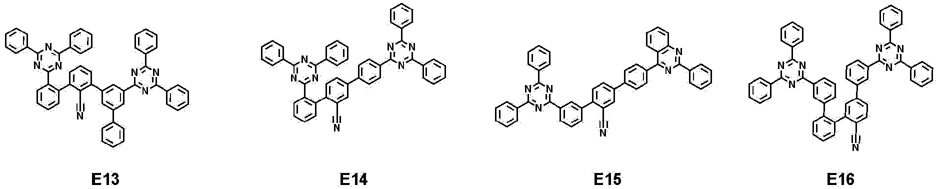

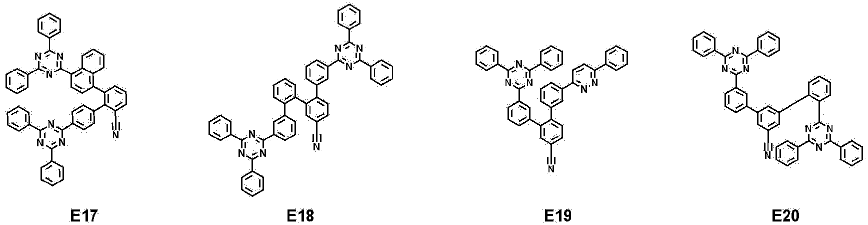

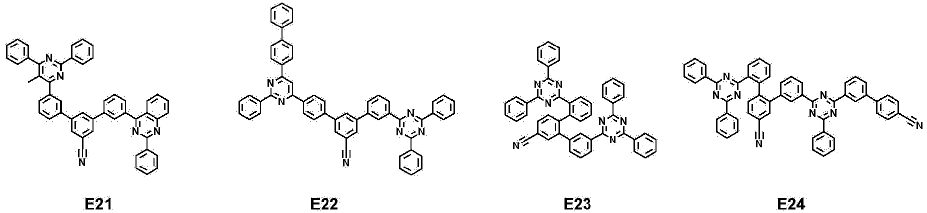

### Comparative Experimental Examples 1 to 18

Organic light emitting devices were manufactured in the same manner as in Experimental Example 1, except that the compounds in the following Table 1 were used instead of Compound E1. ET-1 to ET-18 Compounds used in the following Table 1 were as follows.

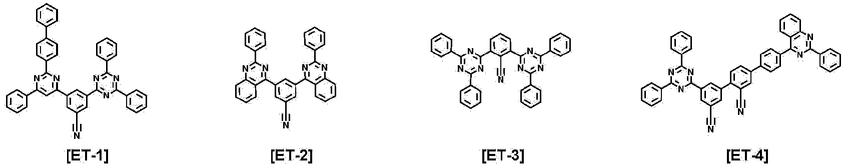

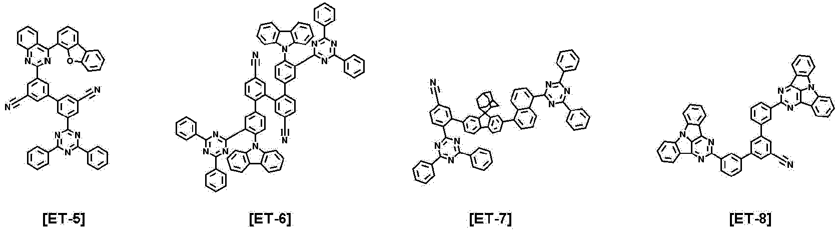

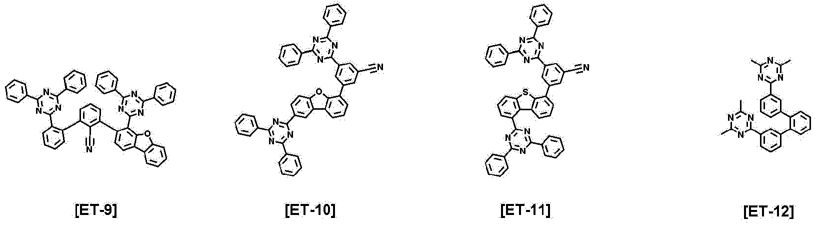

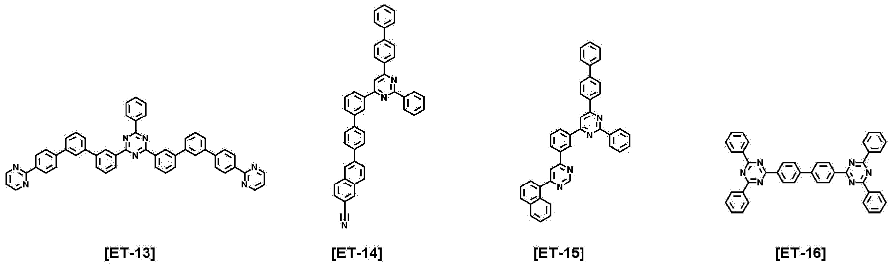

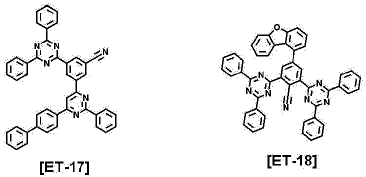

For the organic light emitting devices manufactured in the Experimental Examples and the Comparative Experimental Examples, the driving voltage, the light emitting efficiency and the color coordinate were measured at a current density of 10 mA/cm², and a time (T₉₀) for reaching a 90% value compared to the initial luminance was measured at a current density of 20 mA/cm². The results are shown in the following Table 1.

**[Table 1]**

| | Compound (Electron transport and injection layer) | Voltage (V@10mA /cm²) | Efficiency (cd/A@10mA/cm²) | Color coordinate (x, y) | T₉₀ (hr@20mA/ cm²) |
|---|---|---|---|---|---|
| Experimental Example 1 | E1 | 3.94 | 5.44 | (0.136, 0.112) | 200 |
| Experimental Example 2 | E2 | 4.10 | 5.17 | (0.136, 0.111) | 216 |
| Experimental Example 3 | E3 | 3.98 | 5.39 | (0.136, 0.112) | 212 |
| Experimental Example 4 | E4 | 4.06 | 5.28 | (0.136, 0.111) | 228 |
| Experimental Example 5 | E5 | 3.82 | 5.66 | (0.136, 0.111) | 190 |
| Experimental Example 6 | E6 | 3.94 | 5.54 | (0.136, 0.111) | 184 |
| Experimental Example 7 | E7 | 4.22 | 5.49 | (0.136, 0.112) | 188 |
| Experimental Example 8 | E8 | 3.82 | 5.68 | (0.136, 0.111) | 194 |
| Experimental Example 9 | E9 | 4.22 | 5.11 | (0.136, 0.112) | 204 |
| Experimental Example 10 | E10 | 3.86 | 5.39 | (0.136, 0.111) | 214 |
| Experimental Example 11 | E11 | 3.94 | 5.33 | (0.136, 0.111) | 199 |
| Experimental Example 12 | E12 | 3.98 | 5.30 | (0.136, 0.111) | 203 |
| Experimental Example 13 | E13 | 4.02 | 5.50 | (0.136, 0.112) | 191 |
| Experimental Example 14 | E14 | 3.90 | 5.52 | (0.136, 0.111) | 195 |
| Experimental Example 15 | E15 | 4.06 | 5.27 | (0.136, 0.112) | 224 |
| Experimental Example 16 | E16 | 3.80 | 5.65 | (0.136, 0.111) | 189 |
| Experimental Example 17 | E17 | 3.90 | 5.54 | (0.136, 0.111) | 205 |
| Experimental Example 18 | E18 | 3.84 | 5.53 | (0.136, 0.111) | 199 |
| Experimental Example 19 | E19 | 4.03 | 5.31 | (0.136, 0.112) | 220 |
| Experimental Example 20 | E20 | 3.90 | 5.51 | (0.136, 0.111) | 188 |
| Experimental Example 21 | E21 | 4.07 | 5.51 | (0.136, 0.112) | 180 |
| Experimental Example 22 | E22 | 4.00 | 5.50 | (0.136, 0.111) | 189 |
| Experimental Example 23 | E23 | 3.83 | 5.69 | (0.136, 0.112) | 195 |
| Experimental Example 24 | E24 | 4.22 | 5.10 | (0.136, 0.111) | 230 |
| Comparative Experimental Example 1 | ET-1 | 4.64 | 3.58 | (0.136, 0.111) | 108 |
| Comparative Experimental Example 2 | ET-2 | 4.78 | 3.40 | (0.136, 0.111) | 110 |
| Comparative Experimental Example 3 | ET-3 | 5.01 | 3.22 | (0.136, 0.112) | 86 |
| Comparative Experimental Example 4 | ET-4 | 5.10 | 3.29 | (0.136, 0.111) | 123 |
| Comparative Experimental Example 5 | ET-5 | 5.15 | 3.26 | (0.136, 0.112) | 120 |
| Comparative Experimental Example 6 | ET-6 | 5.24 | 2.90 | (0.136, 0.111) | 54 |
| Comparative Experimental Example 7 | ET-7 | 4.87 | 3.33 | (0.136, 0.111) | 114 |
| Comparative Experimental Example 8 | ET-8 | 4.54 | 3.65 | (0.136, 0.111) | 140 |
| Comparative Experimental Example 9 | ET-9 | 4.50 | 3.94 | (0.136, 0.112) | 130 |
| Comparative Experimental Example 10 | ET-10 | 4.95 | 3.70 | (0.136, 0.111) | 120 |
| Comparative Experimental Example 11 | ET-11 | 4.54 | 3.72 | (0.136, 0.112) | 124 |
| Comparative Experimental Example 12 | ET-12 | 4.41 | 4.08 | (0.136, 0.111) | 43 |
| Comparative Experimental Example 13 | ET-13 | 4.27 | 4.11 | (0.136, 0.111) | 49 |
| Comparative Experimental Example 14 | ET-14 | 4.36 | 3.97 | (0.136, 0.111) | 130 |
| Comparative Experimental Example 15 | ET-15 | 4.44 | 3.95 | (0.136, 0.112) | 55 |
| Comparative Experimental Example 16 | ET-16 | 4.22 | 4.19 | (0.136, 0.111) | 50 |
| Comparative Experimental Example 17 | ET-17 | 4.80 | 3.30 | (0.136, 0.112) | 125 |
| Comparative Experimental Example 18 | ET-18 | 5.06 | 3.19 | (0.136, 0.111) | 128 |

As shown in Table 1, it was confirmed that an organic light emitting device in which the compound represented by Chemical Formula 1 of the present invention was used exhibited excellent characteristics in voltage, efficiency and/or service life (T₉₀).

Specifically, the compound represented by Chemical Formula 1 of the present invention is characterized in that two N-containing cyclic groups represented by Chemical Formula A are linked to a benzene ring including a cyano group through linkers, and exhibited effects of increasing the efficiency of an organic light emitting device by increasing the electron mobility due to two N-containing cyclic groups in the molecule, and increasing the service life of the organic light emitting device by including one cyano group in the molecule to adjust electron injection characteristics.

When compared to Experimental Examples 1 to 22 and Comparative Experimental Examples 1 to 3 and 17 in Table 1, it could be confirmed that the organic light emitting device including the compound of Chemical Formula 1 of the present invention exhibited remarkably excellent characteristics in terms of efficiency and service life compared to organic light emitting devices of the Comparative Experimental Examples, including a compound in which L1 and L2 are a direct bond.

When compared to Experimental Examples 1 to 22 and Comparative Experimental Examples 4 to 6 in Table 1, it could be confirmed that the organic light emitting device including the compound of Chemical Formula 1 of the present invention exhibited remarkably excellent characteristics in terms of efficiency and service life compared to organic light emitting devices of the Comparative Experimental Examples, including a compound, in which L1 or L2 is an arylene group which is substituted with CN.

When compared to Experimental Examples 1 to 22 and Comparative Experimental Examples 7 and 9 to 11 in Table 1, it could be confirmed that the organic light emitting device including the compound of Chemical Formula 1 of the present invention exhibited remarkably excellent characteristics in terms of efficiency and service life compared to organic light emitting devices of the Comparative Experimental Examples, including a compound in which L1 or L2 is not an arylene group.

When compared to Experimental Examples 1 to 22 and Comparative Experimental Example 8 in Table 1, it could be confirmed that the organic light emitting device including the compound of Chemical Formula 1 of the present invention exhibited remarkably excellent characteristics in terms of efficiency and service life compared to organic light emitting devices of the Comparative Experimental Examples, including a compound which does not have the structure of Chemical Formula A.

When compared to Experimental Examples 1 to 22 and Comparative Experimental Examples 12 to 16 in Table 1, it could be confirmed that the organic light emitting device including the compound of Chemical Formula 1 of the present invention exhibited remarkably excellent characteristics in terms of service life compared to organic light emitting devices including the compound of the Comparative Experimental Example, in which a cyano group is not substituted.

When compared to Experimental Examples 1 to 22 and Comparative Experimental Example 18 in Table 1, it could be confirmed that the organic light emitting device including the compound of Chemical Formula 1 of the present invention exhibited remarkably excellent characteristics in terms of voltage, efficiency and service life compared to organic light emitting devices including the compound of the Comparative Experimental Example, in which a phenylene group, in which a cyano group is substituted, has an additional substituent (a dibenzofuran group).

## Claims

1. A compound represented by the following Chemical Formula 1: 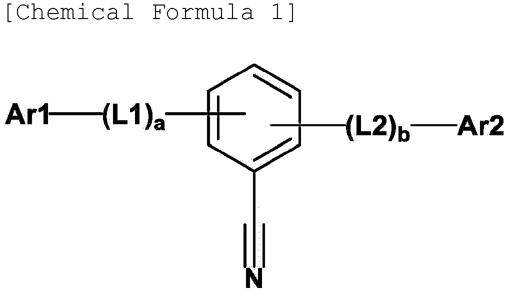 in Chemical Formula 1,
L1 and L2 are the same as or different from each other, and are each independently an arylene group which is unsubstituted or substituted with an aryl group,
Ar1 and Ar2 are the same as or different from each other, and are each independently represented by the following Chemical Formula A, 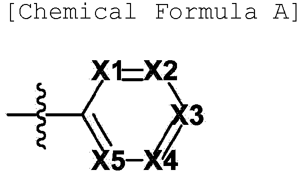
in Chemical Formula A,
X1 is N or CR1, X2 is N or CR2, X3 is N or CR3, X4 is N or CR4, and X5 is N or CR5,
at least two of X1 to X5 are N,
R1 to R5 are the same as or different from each other, and are each independently hydrogen; deuterium; a substituted or unsubstituted alkyl group; a substituted or unsubstituted cycloalkyl group; a substituted or unsubstituted aryl group; or a substituted or unsubstituted heterocyclic group, or adjacent groups are bonded to each other to form a substituted or unsubstituted aromatic hydrocarbon ring,
a and b are each an integer from 1 to 3,
when a is 2 or higher, L1's are the same as or different from each other,
when b is 2 or higher, L2's are the same as or different from each other, and 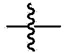 means a position bonded to Chemical Formula 1.

2. The compound of claim 1, wherein Chemical Formula A is represented by any one of the following Structural Formulae A-1 to A-8: 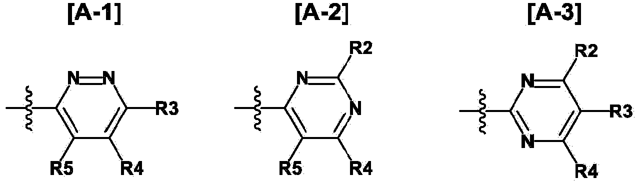 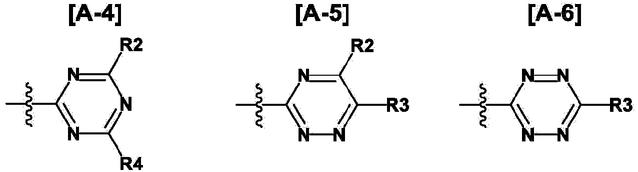 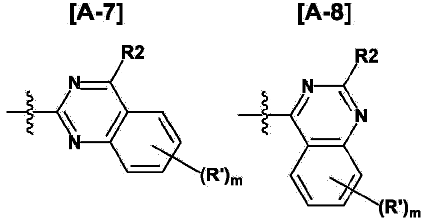
in Structural Formulae A-1 to A-8, the definitions of R2 to R5 are the same as the definitions in Chemical Formula A,
R' is hydrogen; deuterium; a cyano group; a substituted or unsubstituted alkyl group; a substituted or unsubstituted cycloalkyl group; a substituted or unsubstituted aryl group; or a substituted or unsubstituted heterocyclic group,
m is an integer from 0 to 4, and when m is 2 or higher, two or more R"s are the same as or different from each other, and 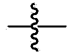 means a position bonded to Chemical Formula 1.

3. The compound of claim 1, wherein Chemical Formula 1 is represented by any one of the following Chemical Formulae 1-1 to 1-6: 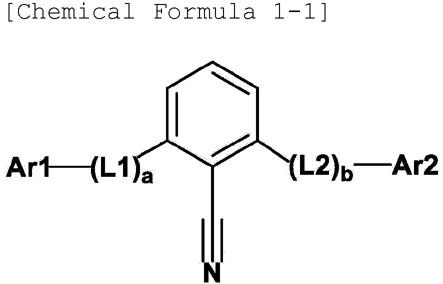 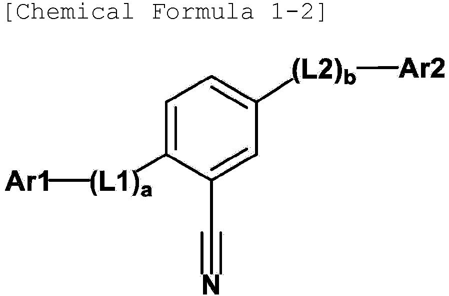 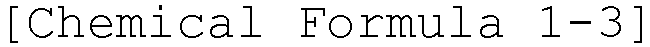 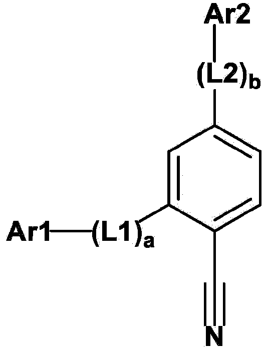 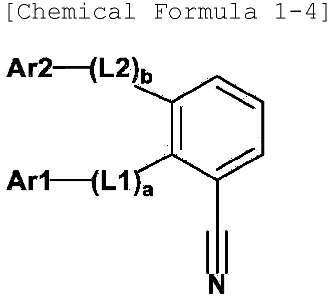 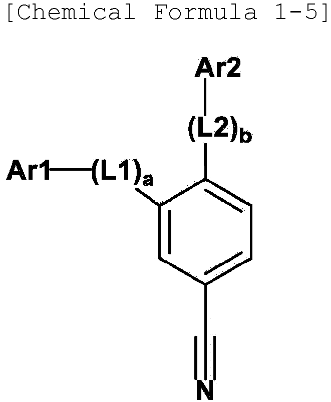 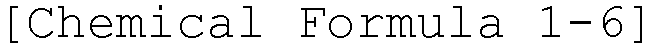 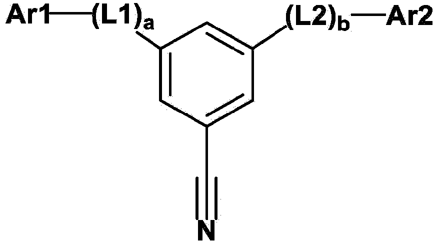 in Chemical Formulae 1-1 to 1-6, the definitions of L1, L2, Ar1, Ar2, a and b are the same as the definitions in Chemical Formula 1.

4. The compound of claim 1, wherein Chemical Formula 1 is represented by any one of the following Chemical Formulae 2-1 to 2-6: 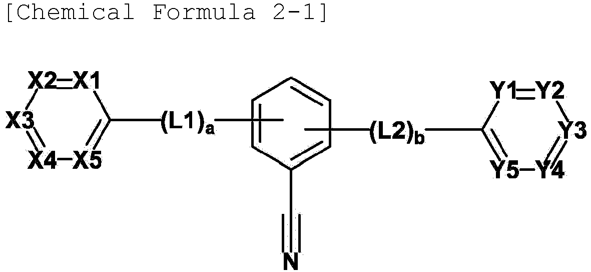 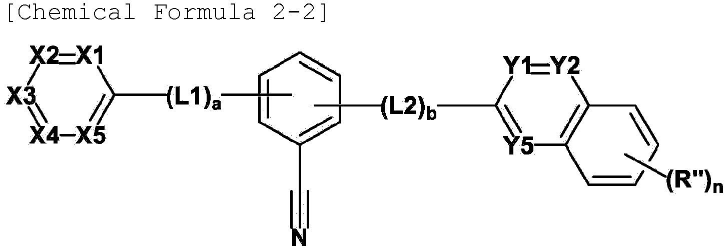 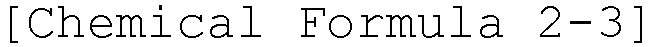 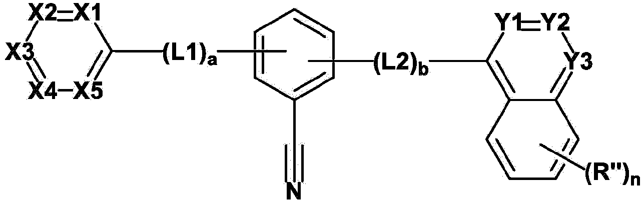 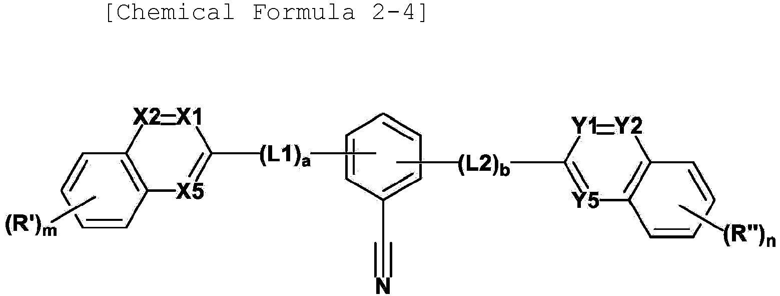 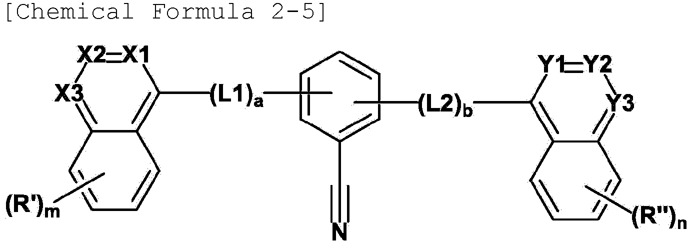 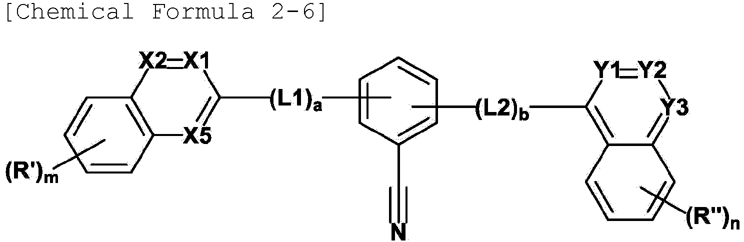
in Chemical Formulae 2-1 to 2-6, the definitions of X1 to X5, L1, L2, a and b are the same as the definitions in Chemical Formula 1,
Y1 is N or CR11, Y2 is N or CR12, Y3 is N or CR13, Y4 is N or CR14, and Y5 is N or CR15,
at least two of Y1 to Y5 are **N,**
R11 to R15 are the same as or different from each other, and are each independently hydrogen; deuterium; a substituted or unsubstituted alkyl group; a substituted or unsubstituted cycloalkyl group; a substituted or unsubstituted aryl group; or a substituted or unsubstituted heterocyclic group,
**R'** and **R''** are the same as or different from each other, and are each independently hydrogen; deuterium; a substituted or unsubstituted alkyl group; a substituted or unsubstituted cycloalkyl group; a substituted or unsubstituted aryl group; or a substituted or unsubstituted heterocyclic group, and
m and n are each an integer from 0 to **4,** and when m and n are each 2 or higher, two or more R"s are the same as or different from each other and two or more R‴s are the same as or different from each other.

5. The compound of claim **1,** wherein Chemical Formula 1 is represented by any one of the following compounds:

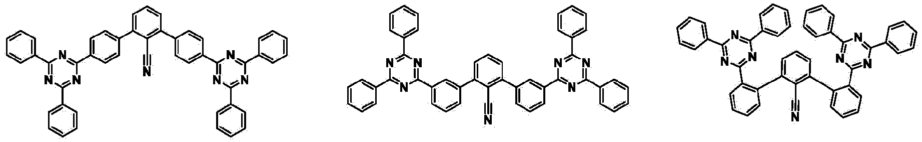

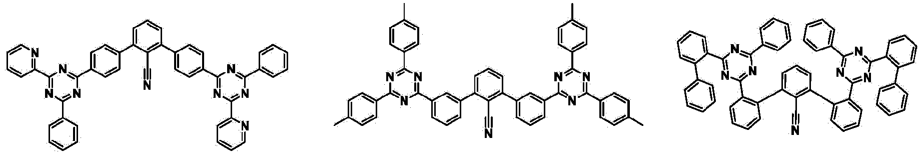

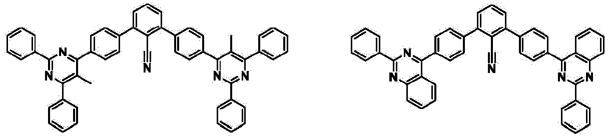

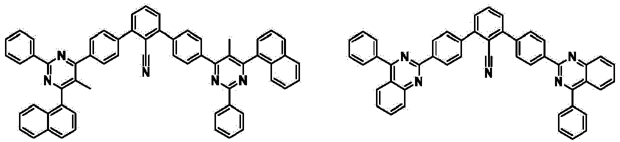

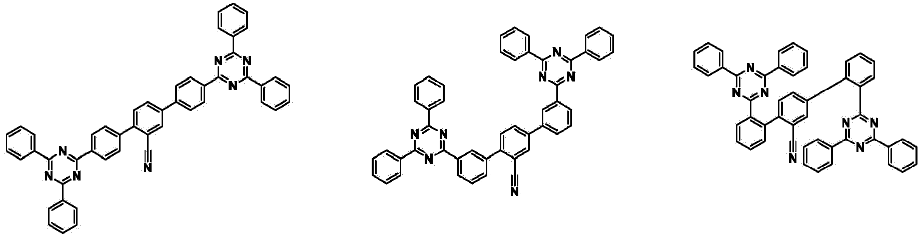

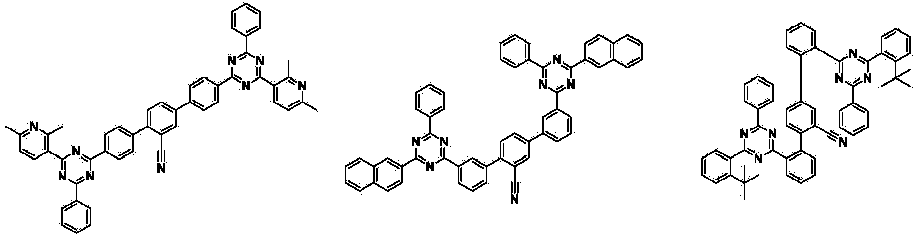

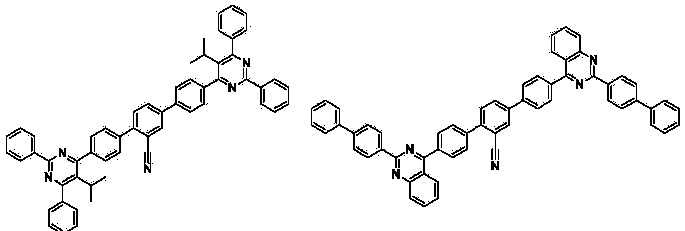

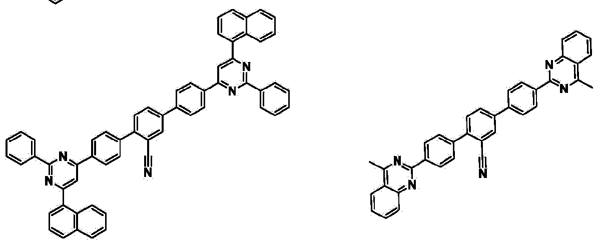

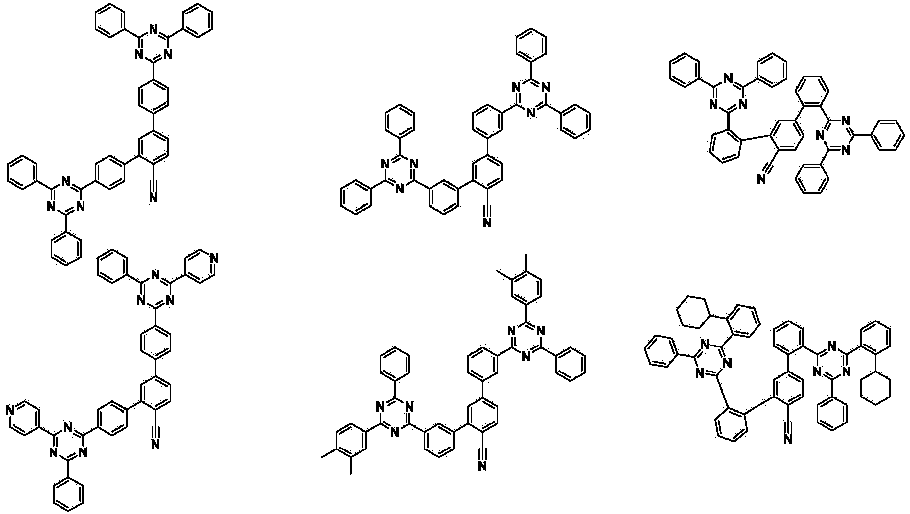

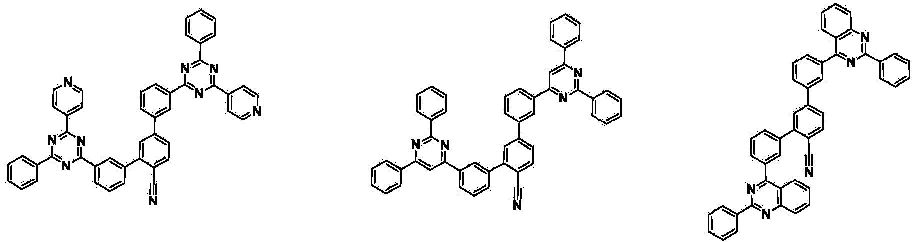

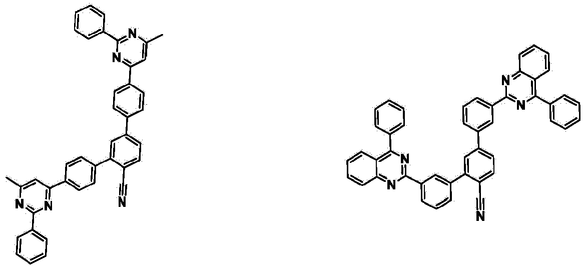

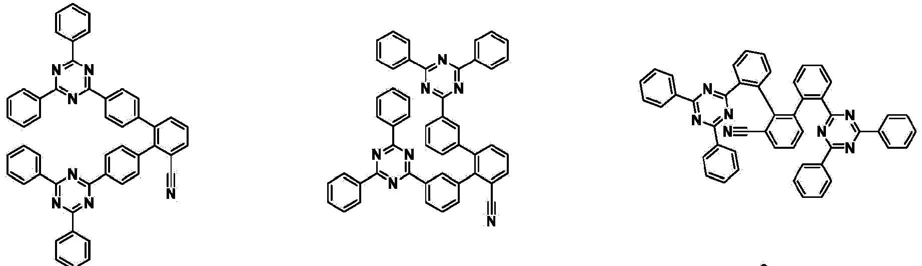

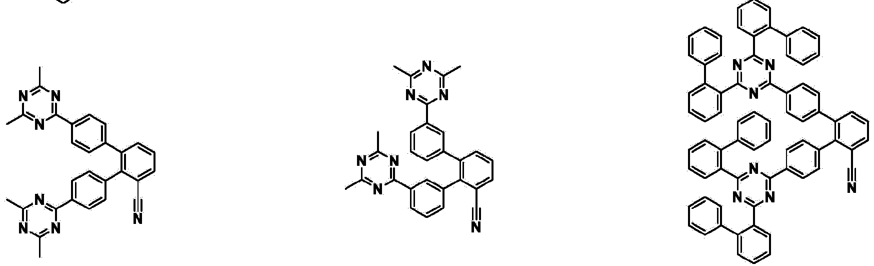

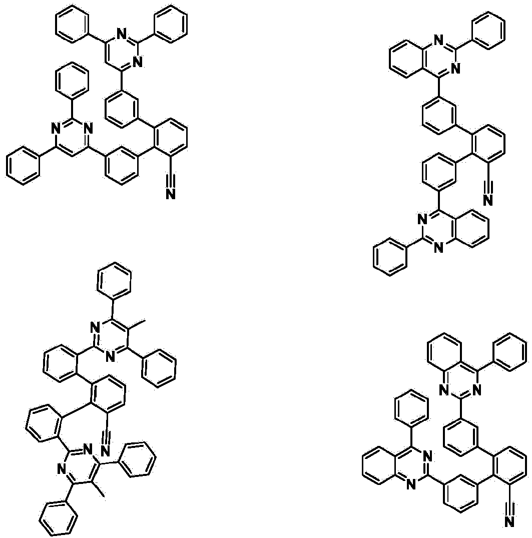

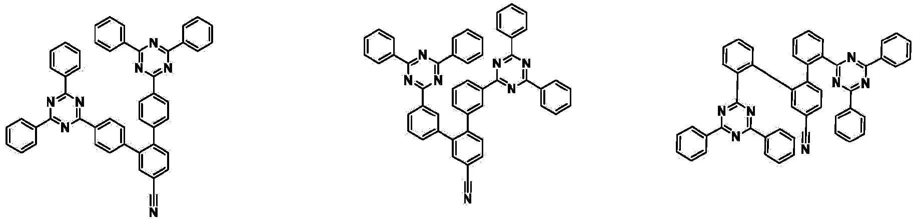

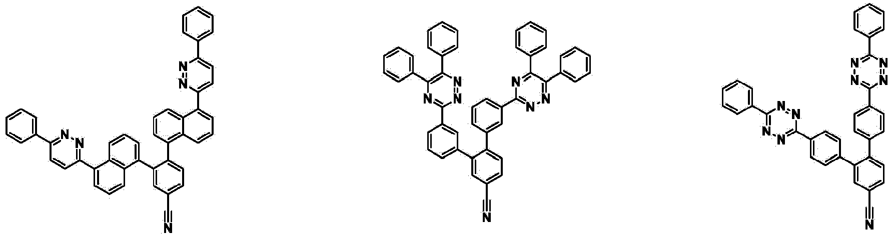

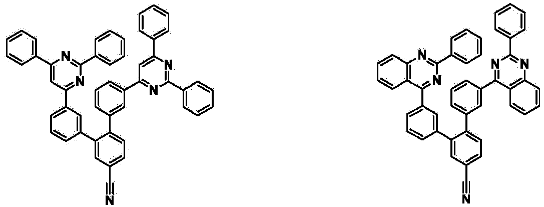

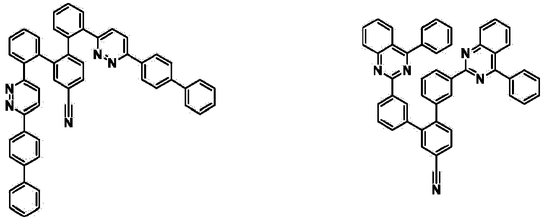

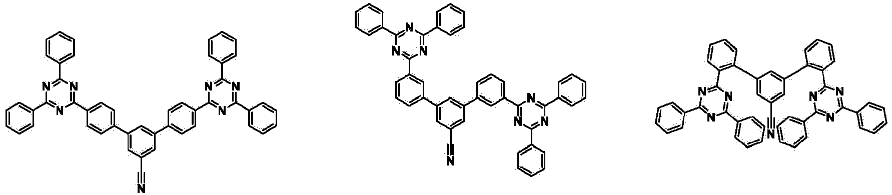

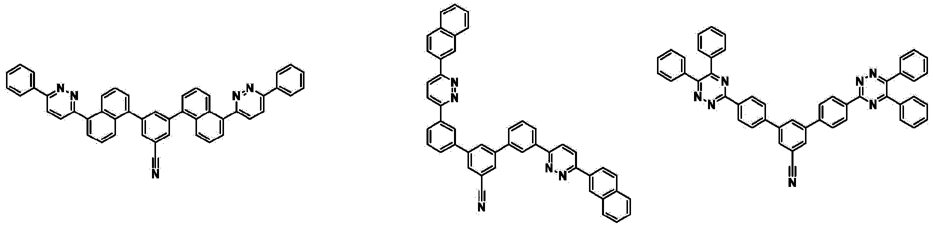

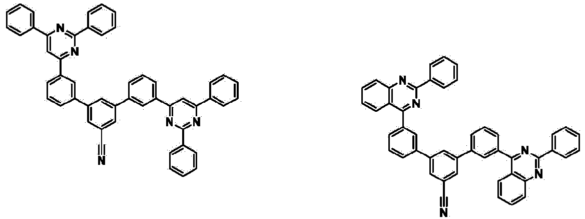

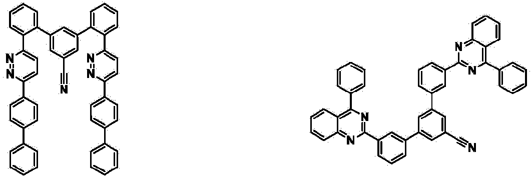

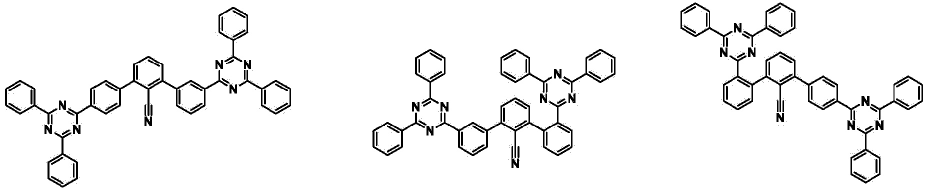

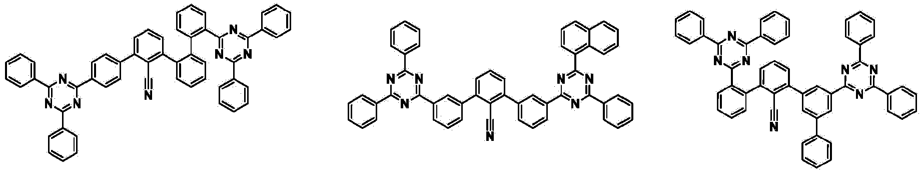

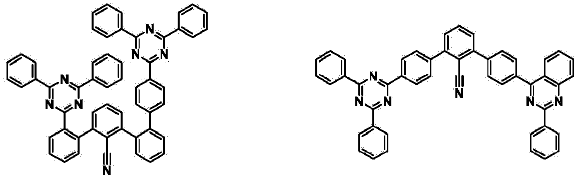

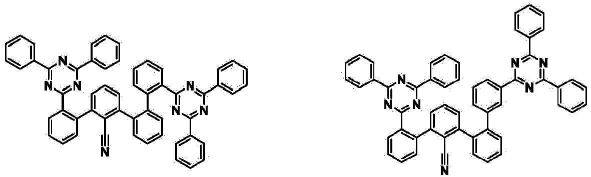

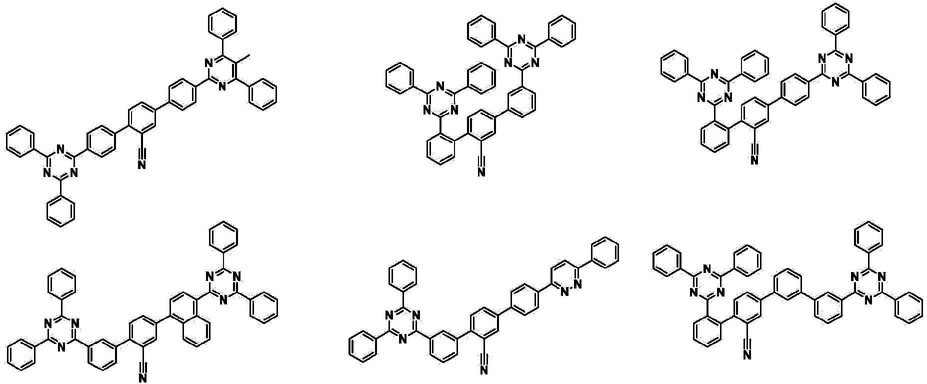

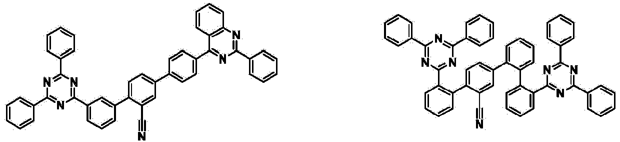

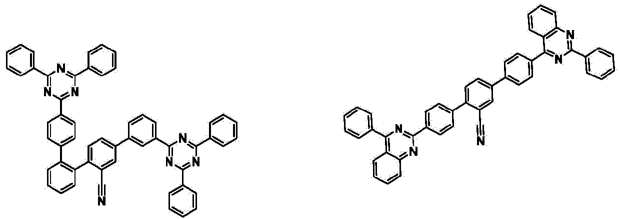

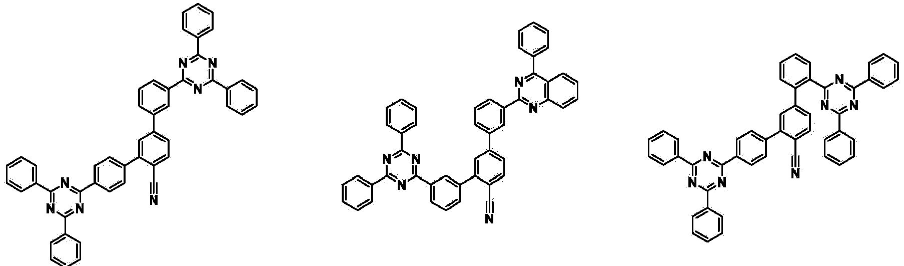

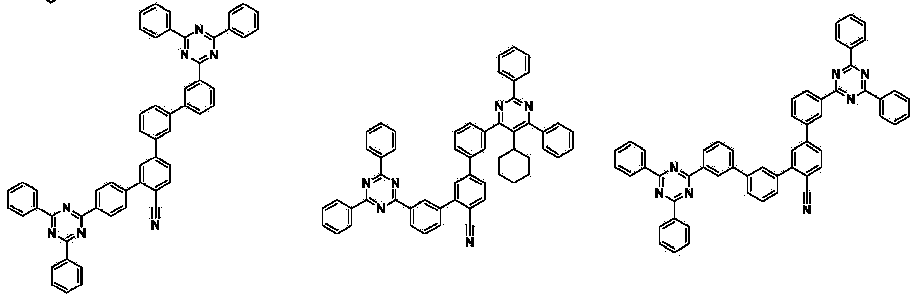

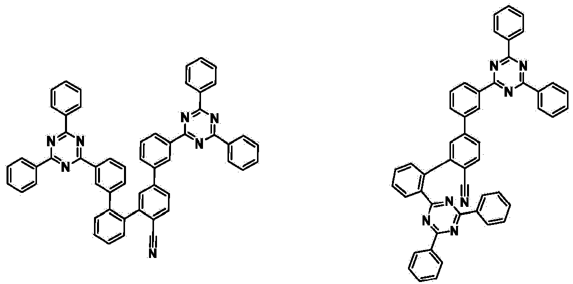

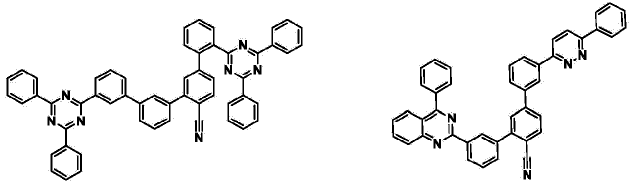

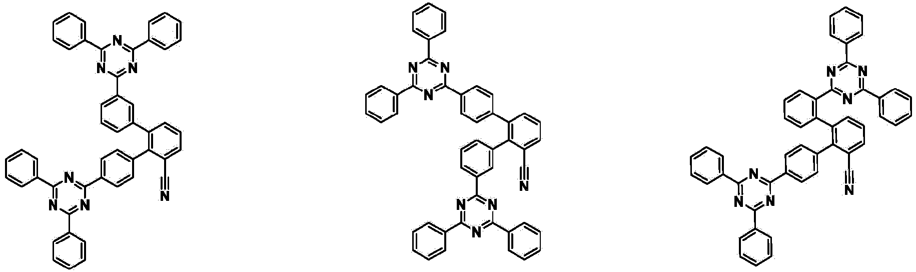

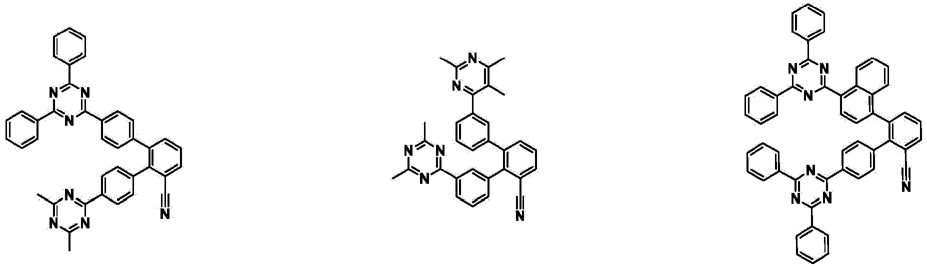

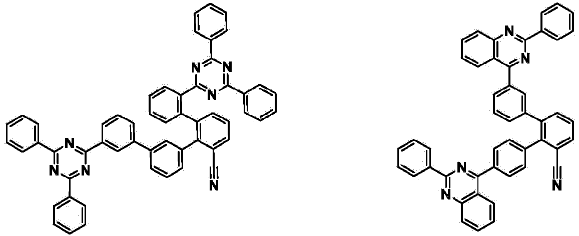

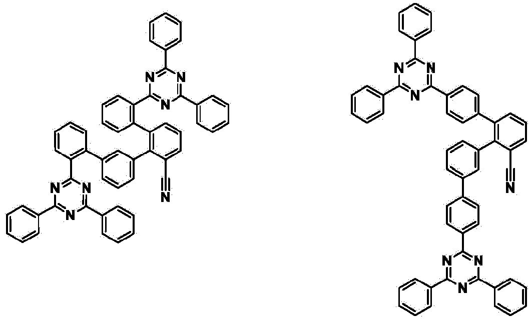

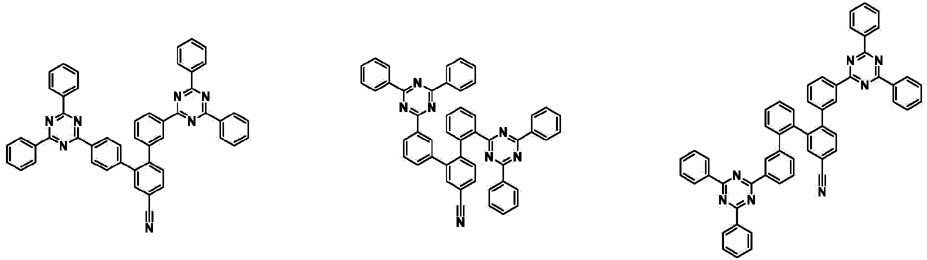

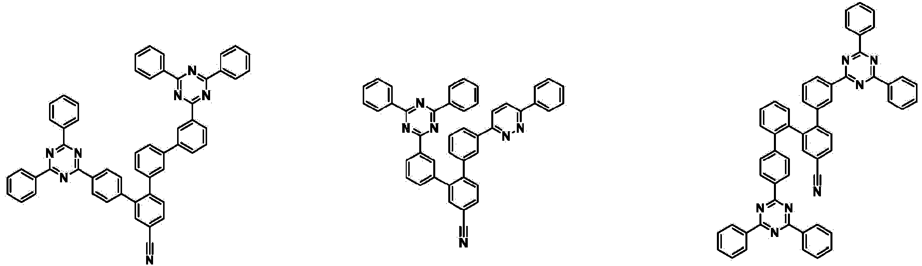

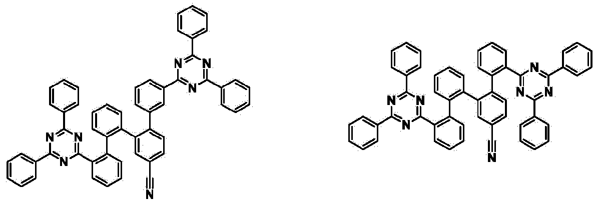

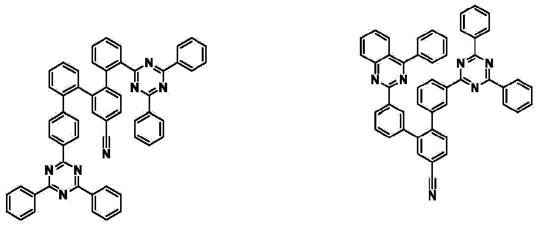

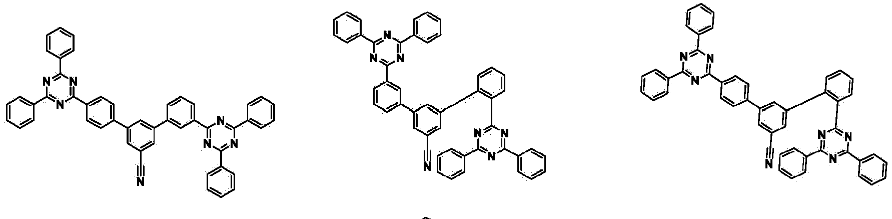

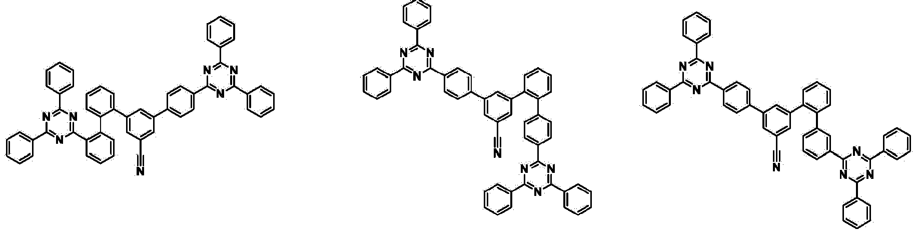

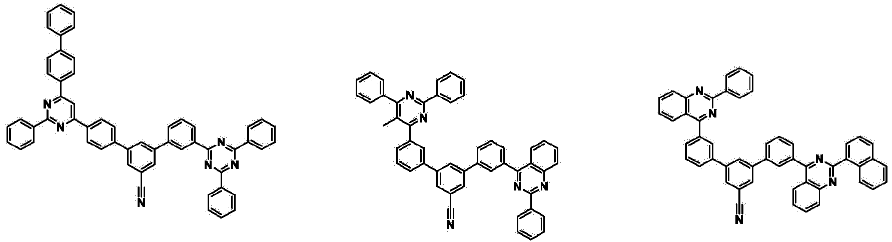

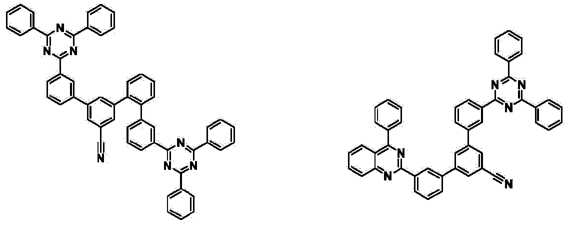

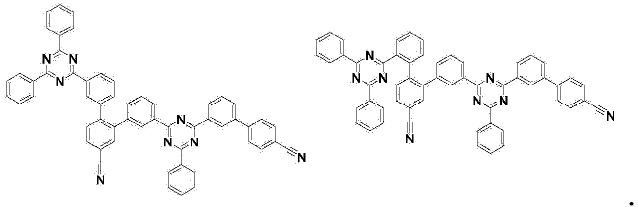

6. An organic light emitting device comprising:
an anode;
a cathode; and
an organic material layer having one or more layers provided between the anode and the cathode,
wherein one or more layers of the organic material layer comprise the compound according to any one of claims 1 to 5.

7. The organic light emitting device of claim 6,
wherein the organic material layer comprises an electron transport layer, an electron injection layer, or an electron transport and injection layer, and the electron transport layer, the electron injection layer, or the electron transport and injection layer comprises the compound.

8. The organic light emitting device of claim 7,
wherein the electron transport layer, the electron injection layer, or the electron transport and injection layer further comprises an n-type dopant or organic metal compound.

9. The organic light emitting device of claim 8,
wherein the compound and the n-type dopant or organic metal compound are comprised at a weight ratio of 2:8 to 8:2.

10. The organic light emitting device of claim 6,
wherein the organic material layer comprises a hole blocking layer, and the hole blocking layer comprises the compound.

11. The organic light emitting device of claim 6,
wherein the organic material layer further comprises one or more layers of a hole injection layer, a hole transport layer, an electron blocking layer, a hole injection and transport layer, a light emitting layer, an electron transport layer, an electron injection layer, a hole blocking layer, and an electron transport and injection layer.

## Patentansprüche

1. Verbindung, dargestellt durch die folgende Chemische Formel 1: 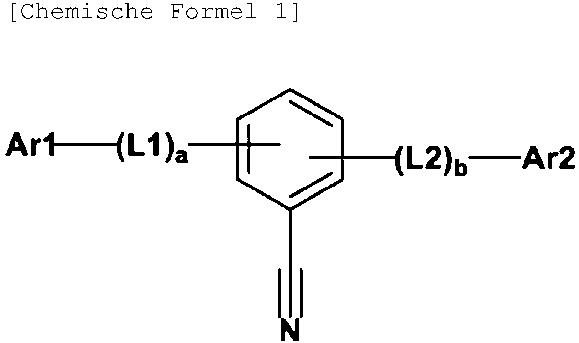 worin in der Chemischen Formel 1
L1 und L2 gleich oder voneinander verschieden sind und jeweils unabhängig eine Arylengruppe, die unsubstituiert ist oder mit einer Arylgruppe substituiert ist, sind,
Ar1 und Ar2 gleich oder voneinander verschieden sind und jeweils unabhängig durch die folgende Chemische Formel A dargestellt sind, 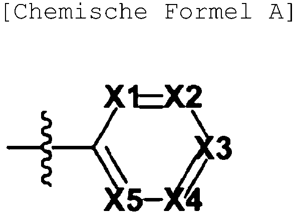
worin in der Chemischen Formel A
X1 N oder CR1 ist, X2 N oder CR2 ist, X3 N oder CR3 ist, X4 N oder CR4 ist und X5 N oder CR5 ist, wobei mindestens zwei von X1 bis X5 N sind,
R1 bis R5 gleich oder voneinander verschieden und jeweils unabhängig Wasserstoff; Deuterium; eine substituierte oder unsubstituierte Alkylgruppe; eine substituierte oder unsubstituierte Cycloalkylgruppe; eine substituierte oder unsubstituierte Arylgruppe; oder eine substituierte oder unsubstituierte heterocyclische Gruppe sind, oder benachbarte Gruppen miteinander verbunden sind, um einen substituierten oder unsubstituierten aromatischen Kohlenwasserstoffring zu bilden,
a und b jeweils eine ganze Zahl von 1 bis 3 sind,
wenn a 2 oder größer ist, die L1 gleich oder voneinander verschieden sind,
wenn b 2 oder größer ist, die L2 gleich oder voneinander verschieden sind, und 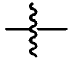 eine Position bezeichnet, die an die Chemische Formel 1 bindet.

2. Verbindung gemäß Anspruch 1, worin die Chemische Formel A durch irgendeine der folgenden Strukturformeln A-1 bis A-8 dargestellt ist: 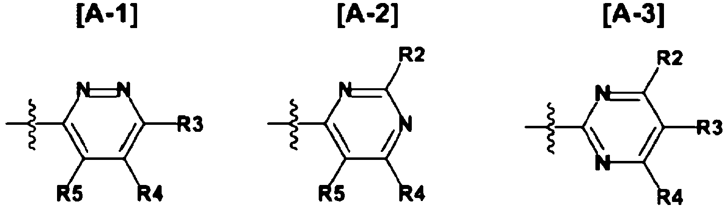 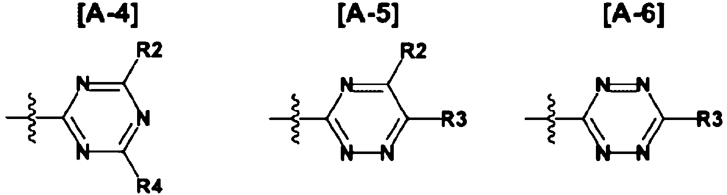 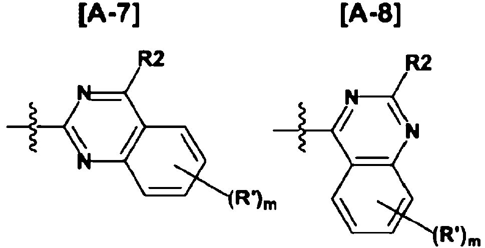
worin in den Strukturformeln A-1 bis A-8 die Definitionen von R2 bis R5 die gleichen sind wie die Definitionen in der Chemischen Formel A,
R' Wasserstoff; Deuterium; eine Cyanogruppe; eine substituierte oder unsubstituierte Alkylgruppe; eine substituierte oder unsubstituierte Cycloalkylgruppe; eine substituierte oder unsubstituierte Arylgruppe oder eine substituierte oder unsubstituierte heterocyclische Gruppe ist,
m eine ganze Zahl von 0 bis 4 ist, und wenn m 2 oder größer ist, zwei oder mehr R' gleich oder voneinander verschieden sind, und 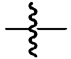 eine Position bezeichnet, die an die chemische Formel 1 bindet.

3. Verbindung gemäß Anspruch 1, worin die Chemische Formel 1 durch irgendeine der folgenden chemischen Formeln 1-1 bis 1-6 dargestellt ist: 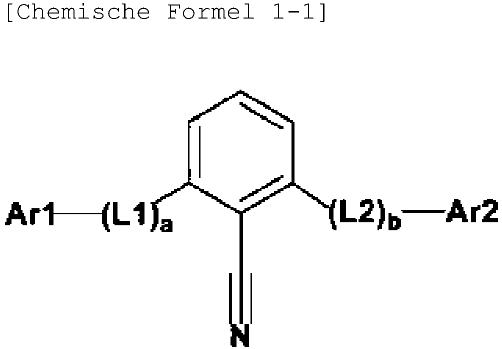 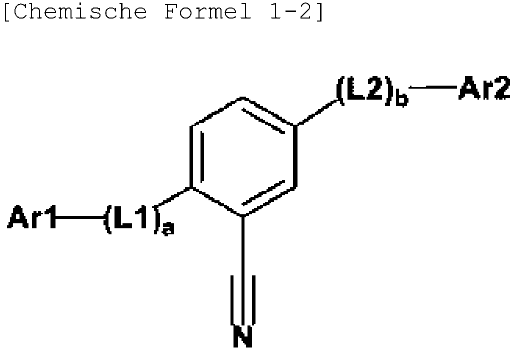 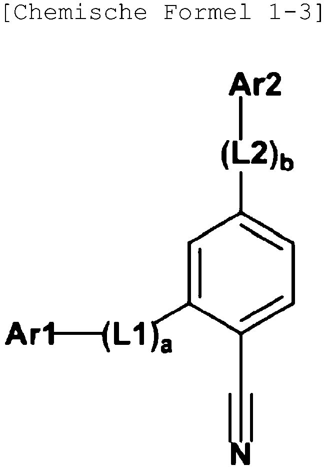 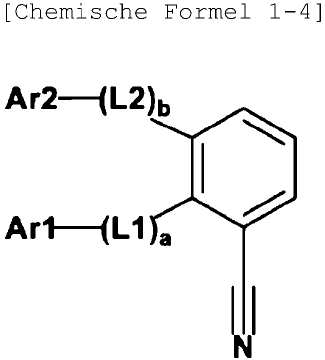 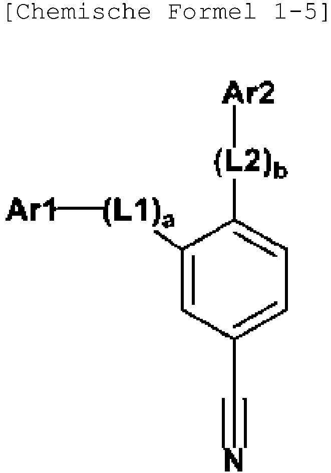 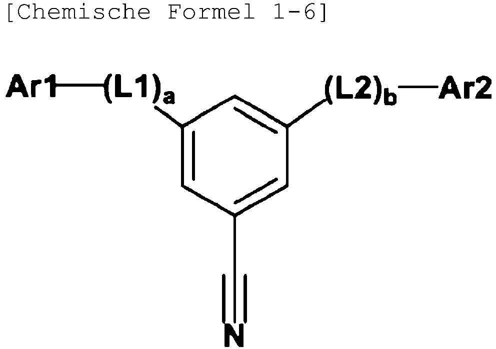 worin in den chemischen Formeln 1-1 bis 1-6 die Definitionen von L1, L2, Ar1, Ar2, a und b die gleichen sind, wie die Definitionen in der Chemischen Formel 1.

4. Verbindung gemäß Anspruch 1, worin die Chemische Formel 1 durch irgendeine der folgenden Chemischen Formeln 2-1 bis 2-6 dargestellt ist: 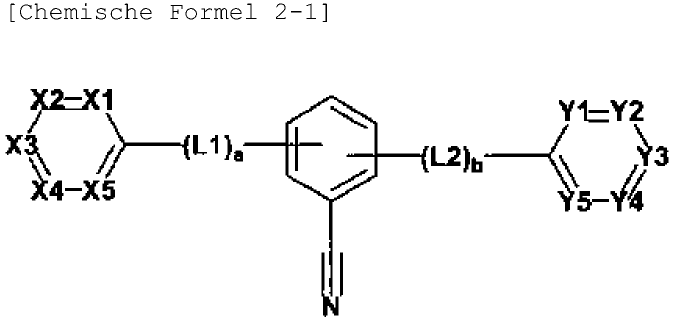 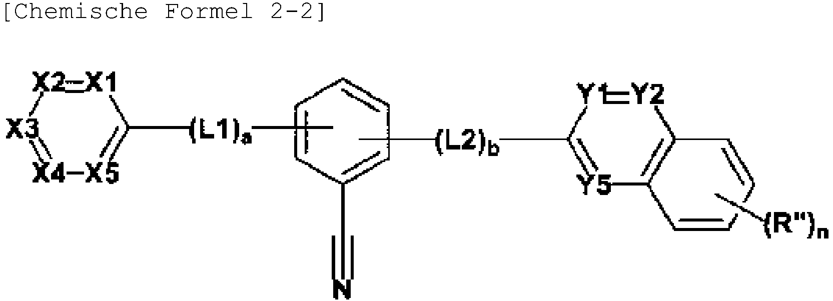 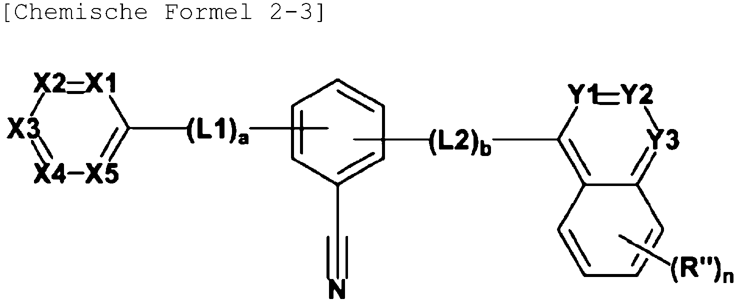 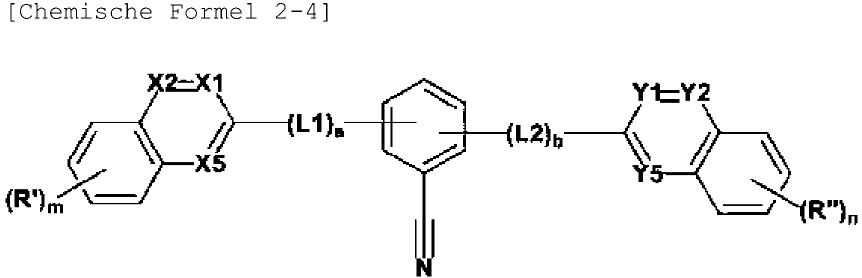 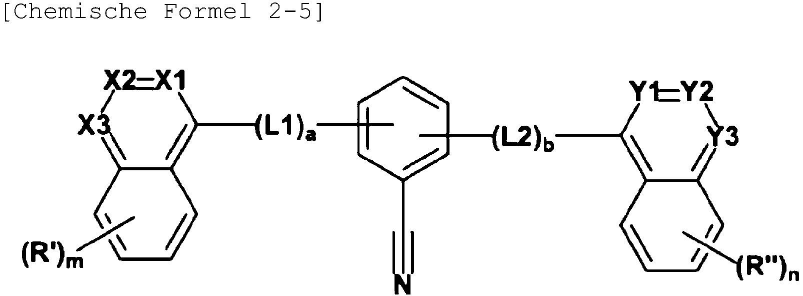 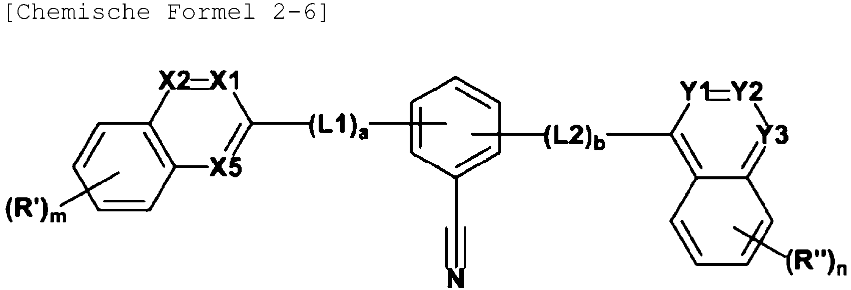
worin in den chemischen Formeln 2-1 bis 2-6 die Definitionen von X1 bis X5, L1, L2, a und b die gleichen sind wie die Definitionen in der Chemischen Formel 1,
Y1 N oder CR11 ist, Y2 N oder CR12 ist, Y3 N oder CR13 ist, Y4 N oder CR14 ist und Y5 N oder CR15 ist, worin zumindest zwei von Y1 bis Y5 N sind,
R11 bis R15 gleich oder voneinander verschieden sind und jeweils unabhängig Wasserstoff; Deuterium; eine substituierte oder unsubstituierte Alkylgruppe; eine substituierte oder unsubstituierte Cycloalkylgruppe; eine substituierte oder unsubstituierte Arylgruppe; oder eine substituierte oder unsubstituierte heterocyclische Gruppe sind,
R' und R" gleich oder voneinander verschieden sind und jeweils unabhängig Wasserstoff; Deuterium; eine substituierte oder unsubstituierte Alkylgruppe; eine substituierte oder unsubstituierte Cycloalkylgruppe; eine substituierte oder unsubstituierte Arylgruppe; oder eine substituierte oder unsubstituierte heterocyclische Gruppe sind, und
m und n jeweils eine ganze Zahl von 0 bis 4 sind, und wenn m und n jeweils 2 oder größer sind, zwei oder mehr R' gleich oder voneinander verschieden sind und zwei oder mehr R" gleich oder voneinander verschieden sind.

5. Verbindung gemäß Anspruch 1, worin die Chemische Formel 1 durch irgendeine der folgenden Verbindungen dargestellt ist:

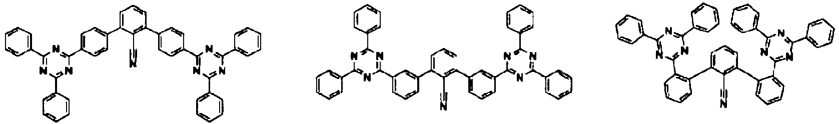

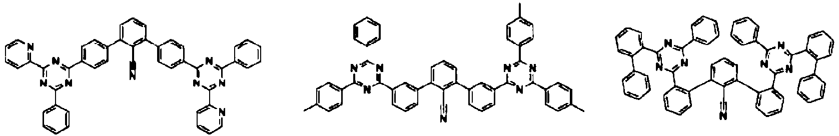

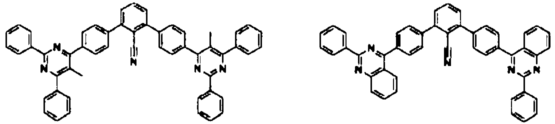

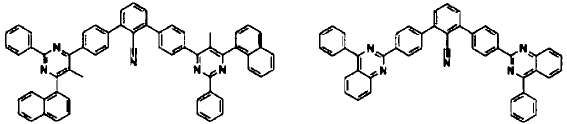

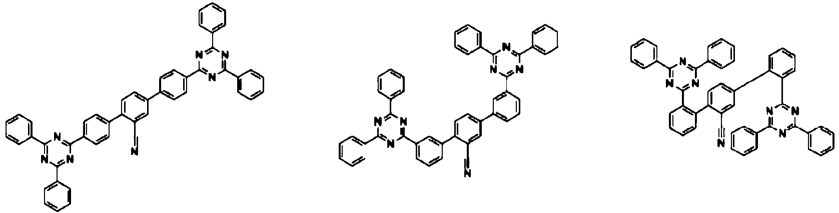

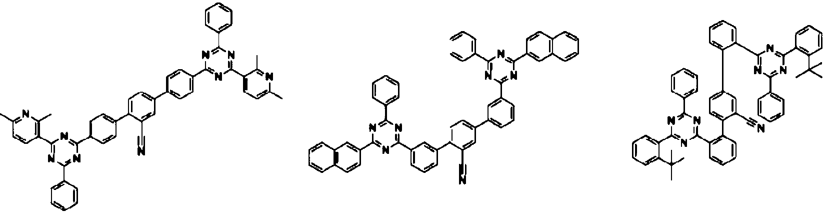

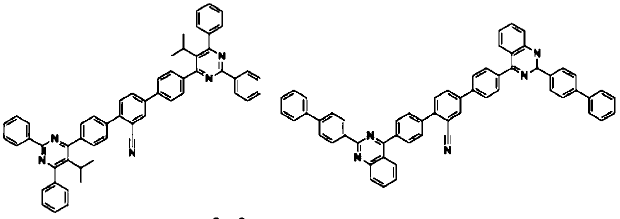

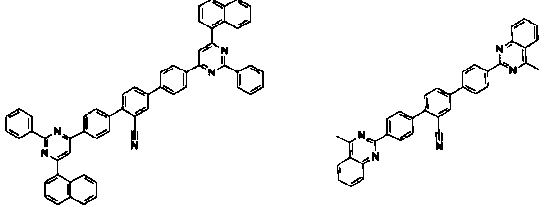

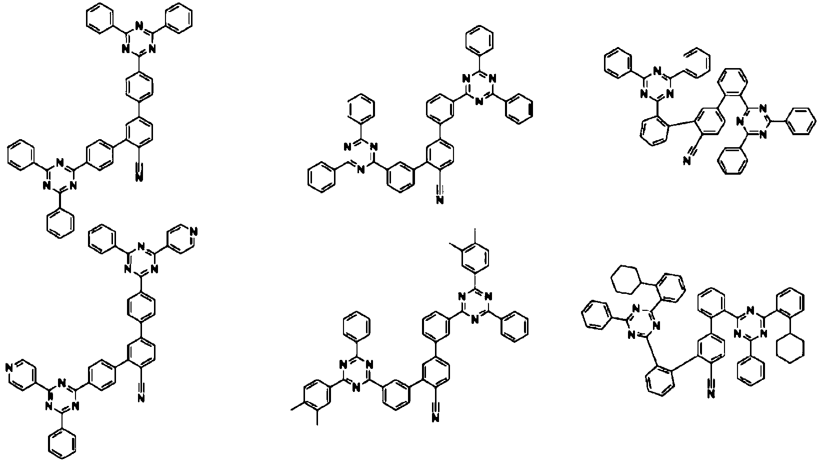

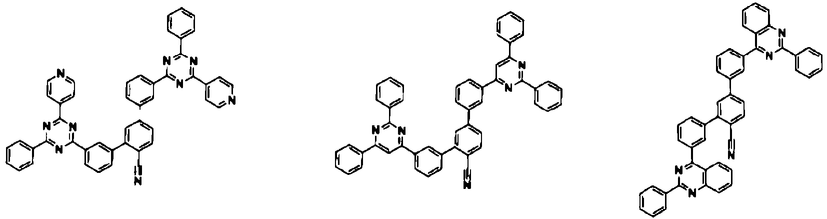

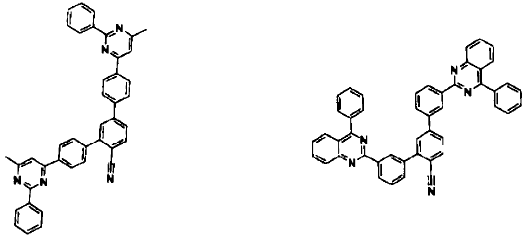

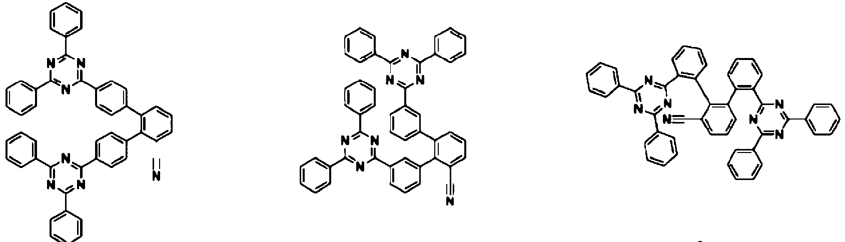

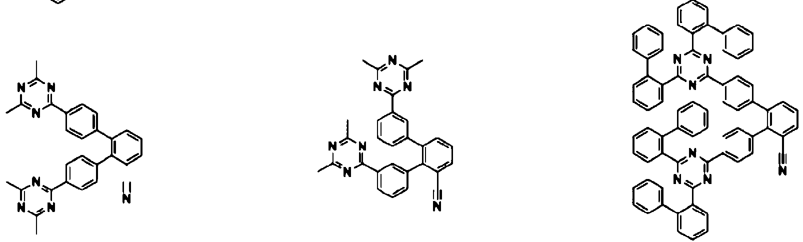

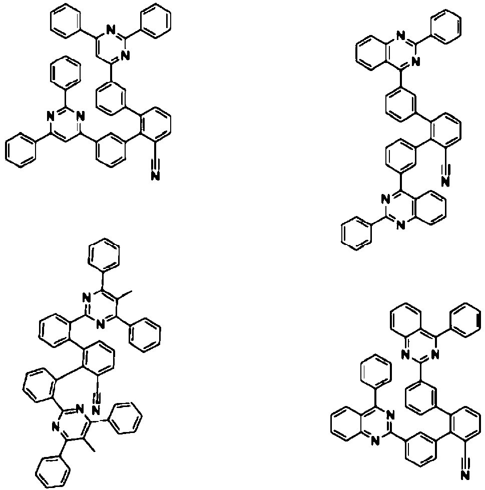

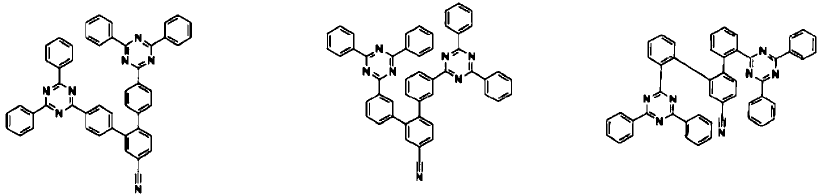

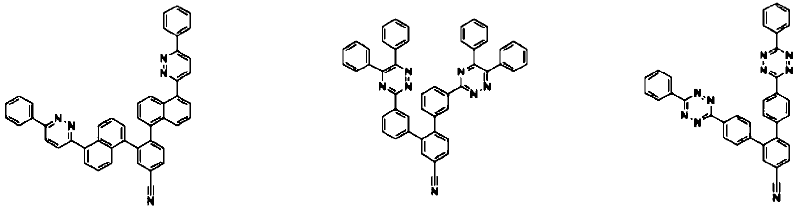

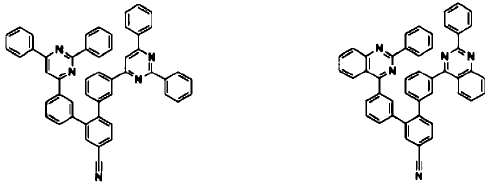

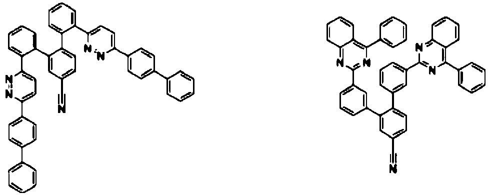

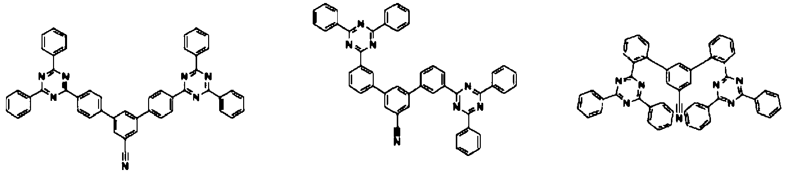

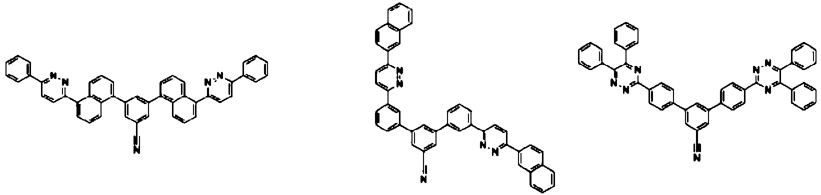

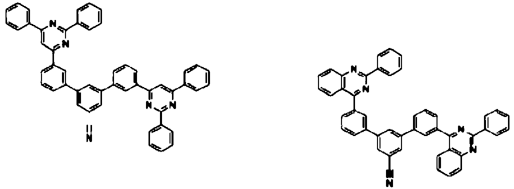

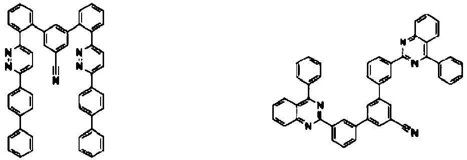

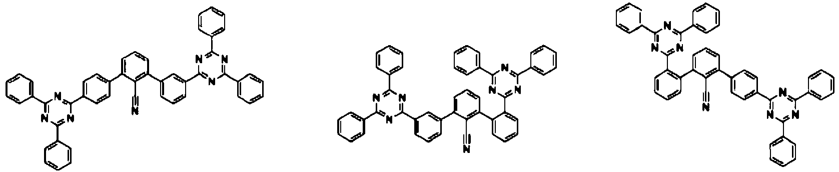

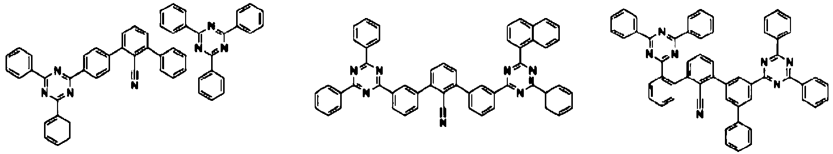

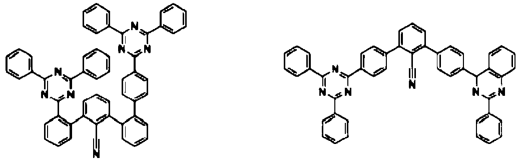

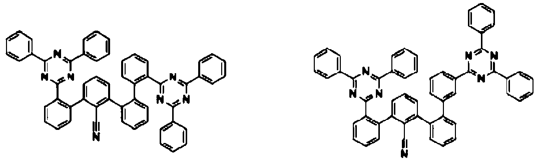

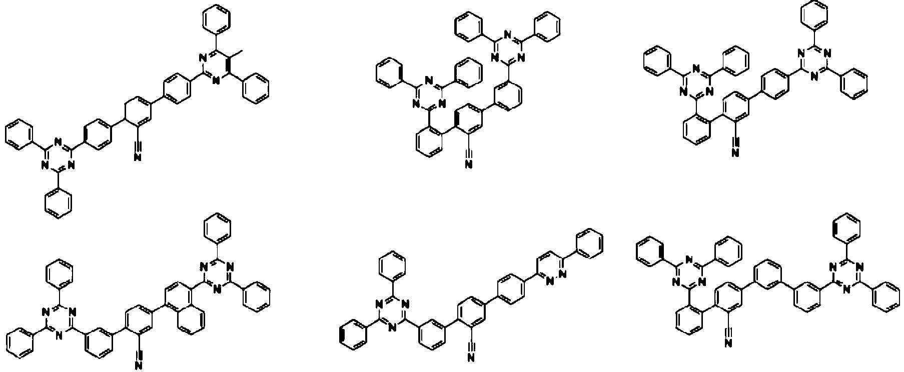

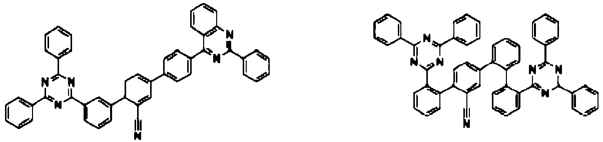

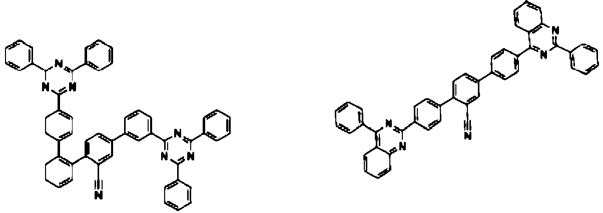

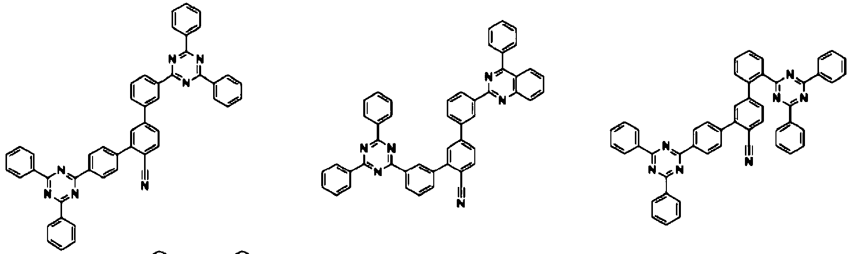

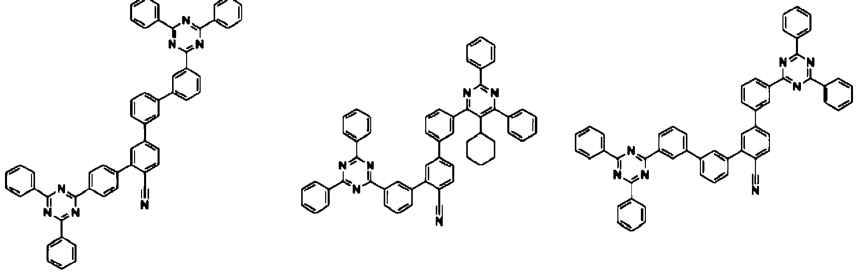

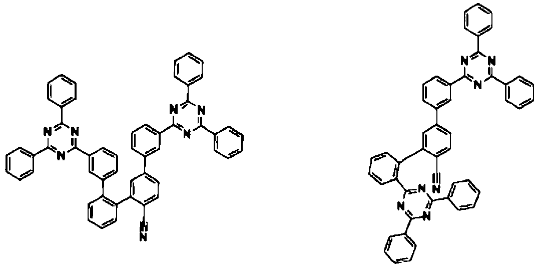

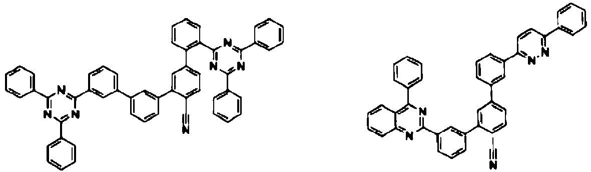

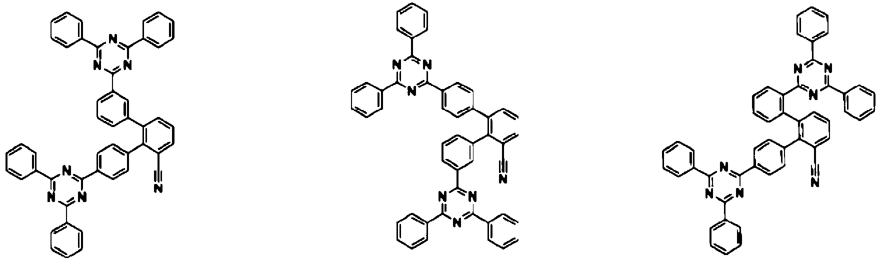

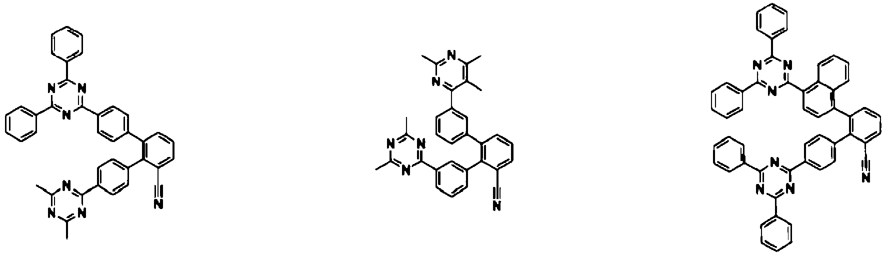

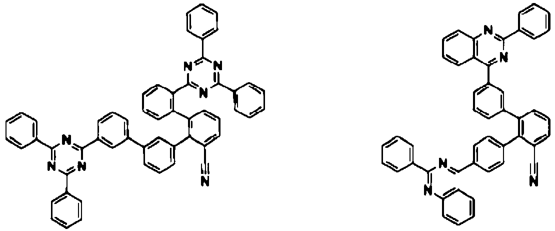

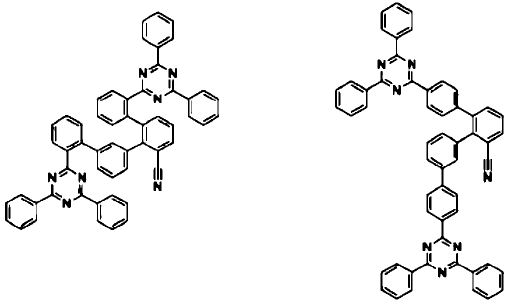

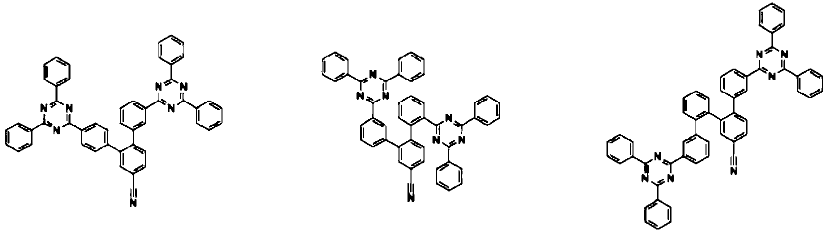

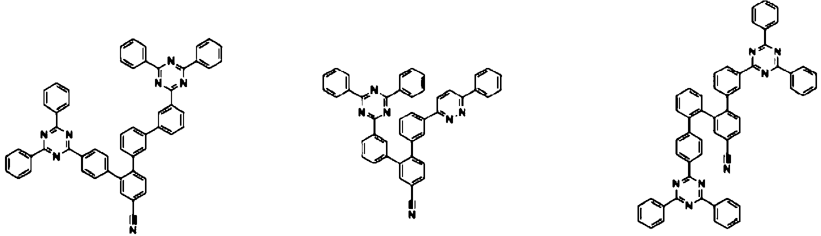

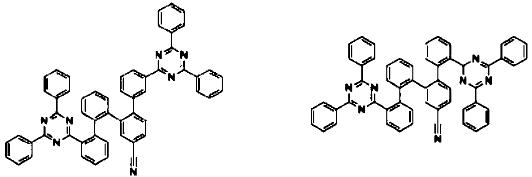

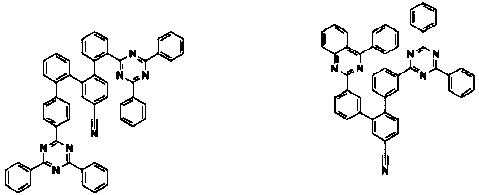

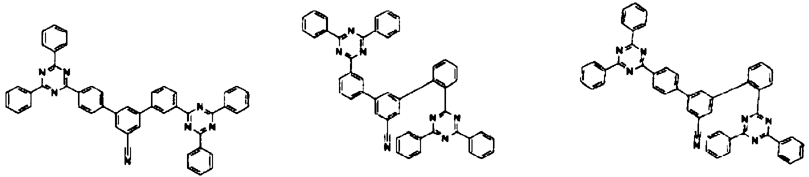

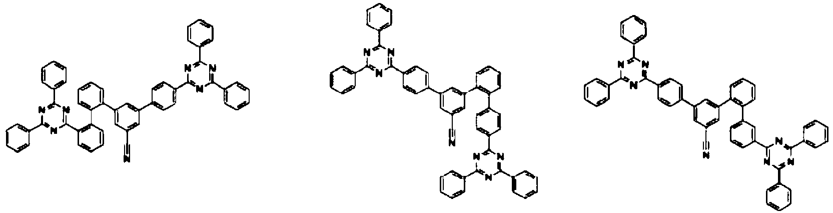

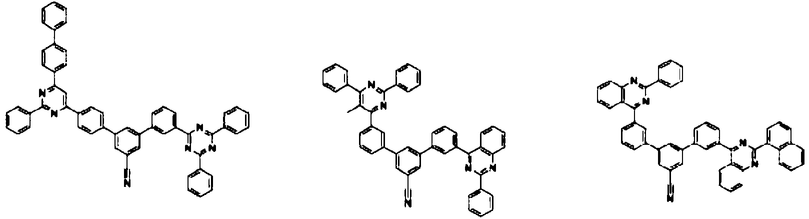

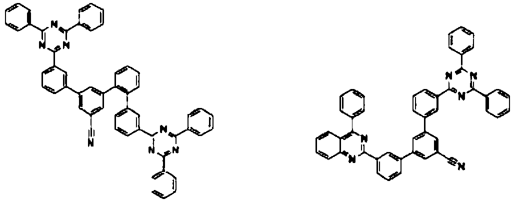

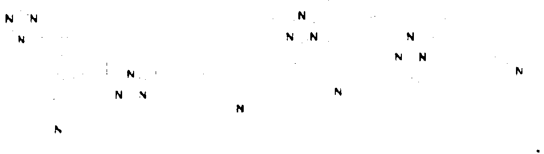

6. Organische lichtemittierende Vorrichtung, umfassend:
eine Anode;
eine Kathode; und
eine organische Materialschicht, die eine oder mehrere Schichten aufweist, vorgesehen zwischen der Anode und der Kathode,
worin eine oder mehr Schichten der organischen Materialschicht die Verbindung gemäß irgendeinem der Ansprüche 1 bis 5 umfasst.

7. Organische lichtemittierende Vorrichtung gemäß Anspruch 6, worin die organische Materialschicht eine Elektronentransportschicht, eine Elektroneninjektionsschicht oder eine Elektronentransport- und -injektionsschicht umfasst, und worin die Elektronentransportschicht, die Elektroneninjektionsschicht oder die Elektronentransport- und -injektionsschicht die Verbindung umfasst.

8. Organische lichtemittierende Vorrichtung gemäß Anspruch 7, worin die Elektronentransportschicht, die Elektroneninjektionsschicht oder die Elektronentransport- und -injektionsschicht ferner eine Dotiersubstanz vom n-Typ oder eine organische Metallverbindung umfasst.

9. Organische lichtemittierende Vorrichtung gemäß Anspruch 8, worin die Verbindung und die Dotiersubstanz vom n-Typ oder die organische Metallverbindung in einem Gewichtsverhältnis von 2:8 bis 8:2 umfasst sind.

10. Organische lichtemittierende Vorrichtung gemäß Anspruch 6, worin die organische Materialschicht eine Loch-Sperrschicht umfasst und die Loch-Sperrschicht die Verbindung umfasst.

11. Organische lichtemittierende Vorrichtung gemäß Anspruch 6, worin die organische Materialschicht ferner eine oder mehr Schichten von einer Lochinjektionsschicht, einer Lochtransportschicht, einer Elektronen-Sperrrschicht, einer Lochinjektions- und Transportschicht, einer lichtemittierenden Schicht, einer Elektronentransportschicht, einer Elektroneninjektionsschicht, einer Loch-Sperrschicht und einer Elektronentransport- und -injektionsschicht umfasst.

## Revendications

1. Composé représenté par la formule chimique 1 suivante : 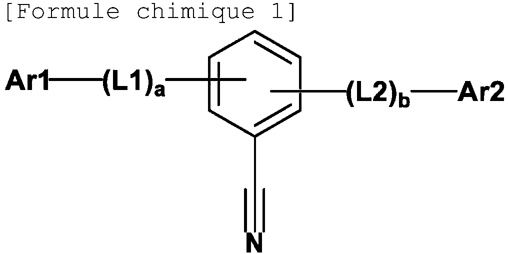 dans la formule chimique 1,
L1 et L2 sont identiques ou différents l'un de l'autre et représentent chacun indépendamment un groupe arylène non substitué ou substitué par un groupe aryle,
Ar1 et Ar2 sont identiques ou différents l'un de l'autre, et sont chacun indépendamment représentés par la formule chimique A suivante : 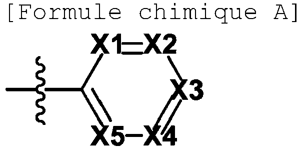
dans la formule chimique A,
X1 est N ou CR1, X2 est N ou CR2, X3 est N ou CR3, X4
est N ou CR4, et X5 est N ou CR5,
au moins deux des X1 à X5 sont N,
R1 à R5 sont identiques ou différents les uns des autres, et sont chacun indépendamment un atome d'hydrogène ; un atome de deutérium ; un groupe alkyle substitué ou non substitué ; un groupe cycloalkyle substitué ou non substitué ; un groupe aryle substitué ou non substitué ; ou un groupe hétérocyclique substitué ou non substitué, ou des groupes adjacents sont liés les uns aux autres pour former un cycle hydrocarboné aromatique substitué ou non substitué,
a et b sont chacun un nombre entier de 1 à 3, lorsque a est égal ou supérieur à 2, les L1 sont identiques ou différents les uns des autres, lorsque b est égal ou supérieur à 2, les L2 sont identiques ou différents les uns des autres, et 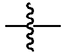 signifie une position liée à la formule chimique 1.

2. Le composé de la revendication 1, dans lequel la formule chimique A est représentée par l'une quelconque des formules structurales A-1 à A-8 suivantes : 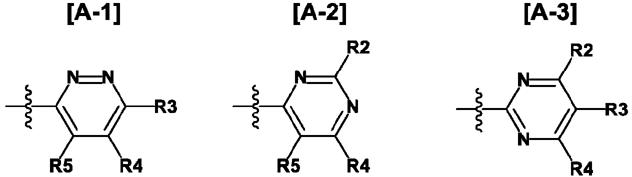 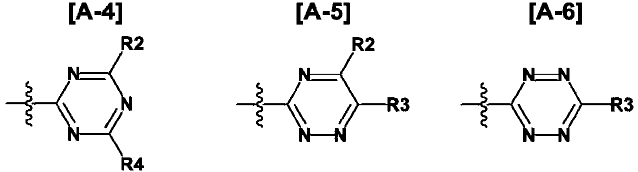 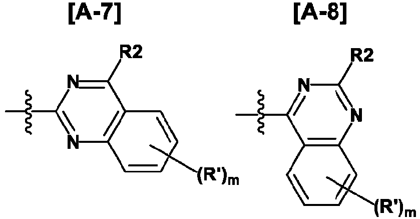
dans les formules structurales A-1 à A-8, les définitions de R2 à R5 sont les mêmes que les définitions dans la formule chimique A,
R' est un atome d'hydrogène ; un atome de deutérium ; un groupe cyano ; un groupe alkyle substitué ou non substitué ; un groupe cycloalkyle substitué ou non substitué ; un groupe aryle substitué ou non substitué ;
ou un groupe hétérocyclique substitué ou non substitué, m est un nombre entier compris entre 0 et 4, et lorsque m est égal ou supérieur à 2, deux ou plusieurs R' sont identiques ou différents les uns des autres, et 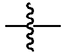 signifie une position liée à la formule chimique 1.

3. Le composé de la revendication 1, dans lequel la formule chimique 1 est représentée par l'une quelconque des formules chimiques 1-1 à 1-6 suivantes : 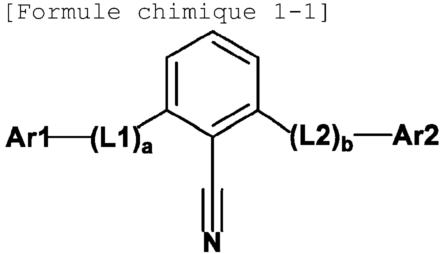 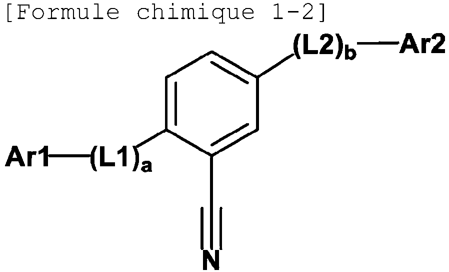 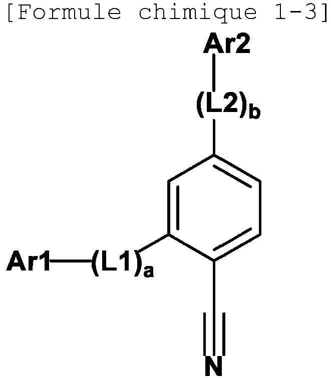 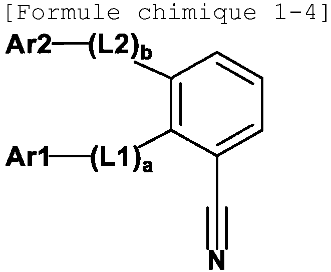 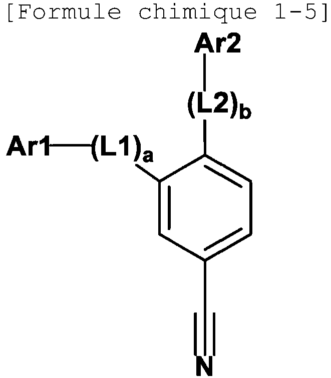 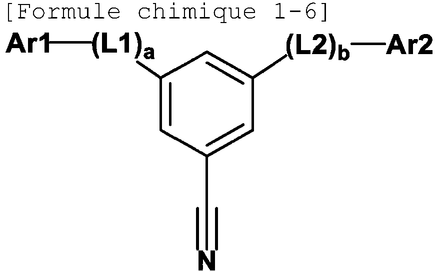 dans les formules chimiques 1-1 à 1-6, les définitions de L1, L2, Ar1, Ar2, a et b sont les mêmes que celles de la formule chimique 1.

4. Le composé de la revendication 1, dans lequel la formule chimique 1 est représentée par l'une quelconque des formules chimiques 2-1 à 2-6 suivantes : 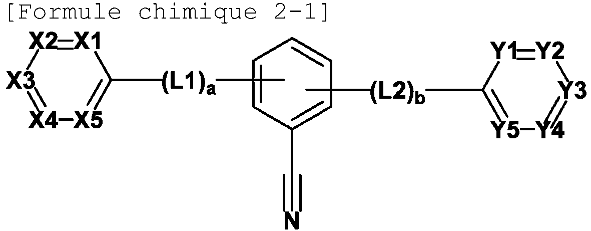 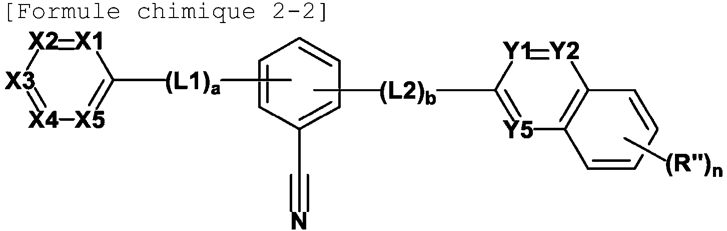 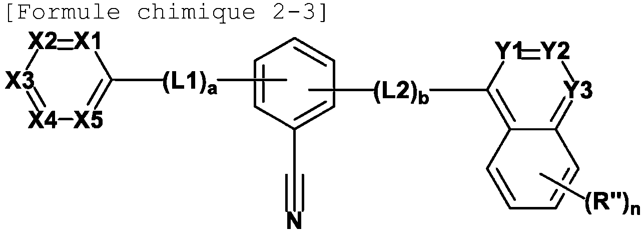 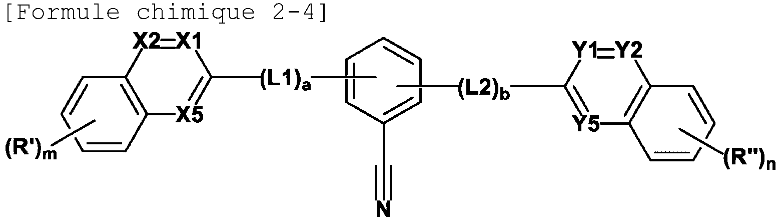 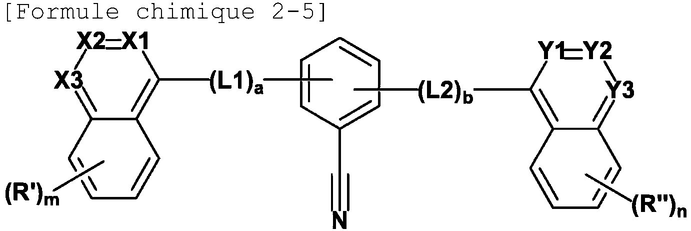 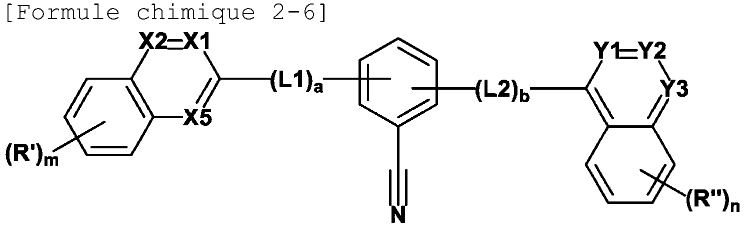
dans les formules chimiques 2-1 à 2-6, les définitions de X1 à X5, L1, L2, a et b sont les mêmes que celles de la formule chimique 1,
Y1 est N ou CR11, Y2 est N ou CR12, Y3 est N ou CR13, Y4 est N ou CR14, et Y5 est N ou CR15,
au moins deux des Y1 à Y5 sont N,
R11 à R15 sont identiques ou différents les uns des autres, et sont chacun indépendamment un atome d'hydrogène ; un atome de deutérium ; un groupe alkyle substitué ou non substitué ; un groupe cycloalkyle substitué ou non substitué ; un groupe aryle substitué ou non substitué ; ou un groupe hétérocyclique substitué ou non substitué,
R' et R'' sont identiques ou différents l'un de l'autre, et représentent chacun indépendamment un atome d'hydrogène, un atome de deutérium, un groupe alkyle substitué ou non substitué, un groupe cycloalkyle substitué ou non substitué, un groupe aryle substitué ou non substitué ; ou un groupe hétérocyclique substitué ou non substitué, et
m et n sont chacun un nombre entier de 0 à 4, et lorsque m et n sont chacun 2 ou plus, deux ou plusieurs R' sont identiques ou différents les uns des autres et deux ou plusieurs R'' sont identiques ou différents les uns des autres.

5. Le composé de la revendication 1, dans lequel la formule chimique 1 est représentée par l'un quelconque des composés suivants :

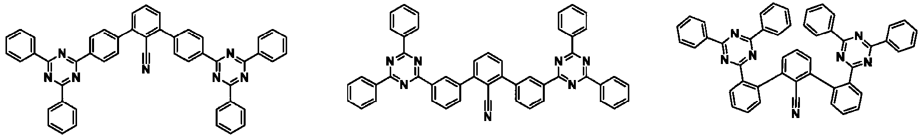

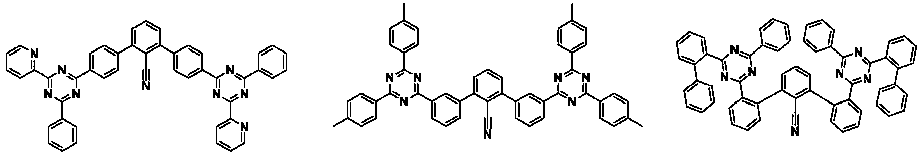

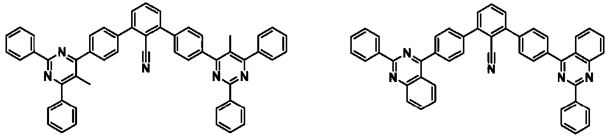

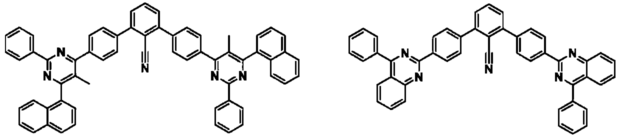

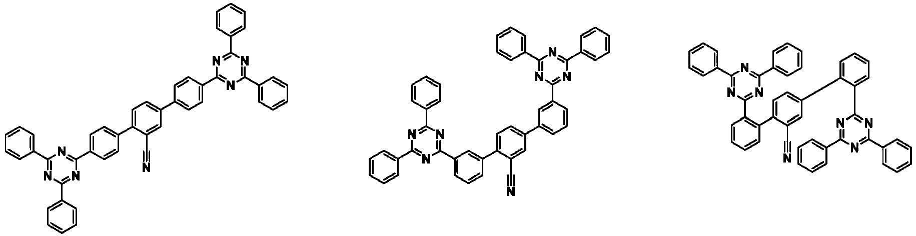

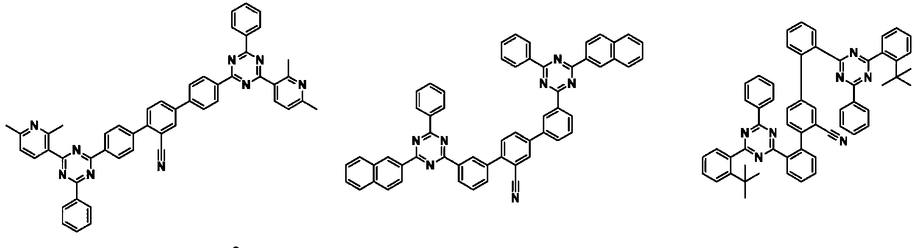

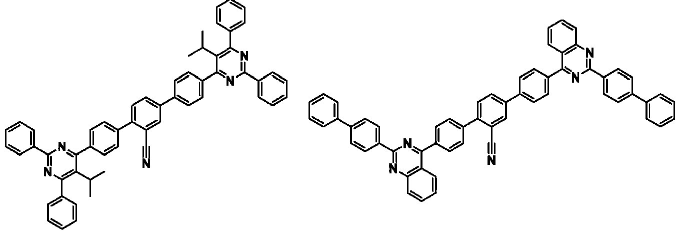

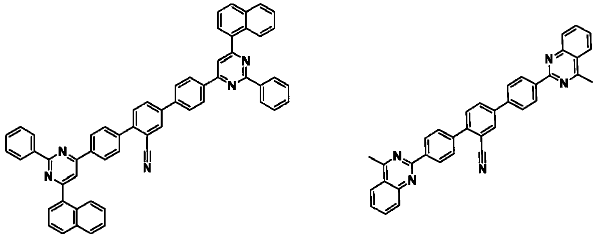

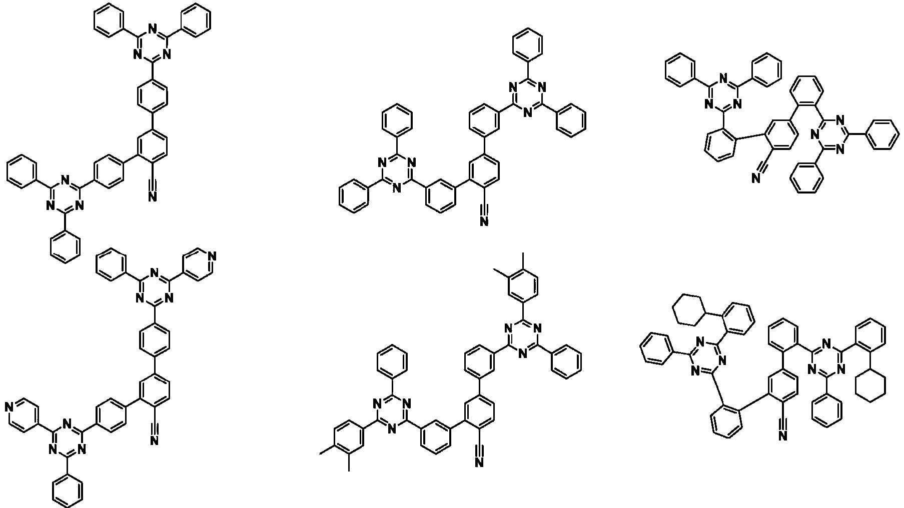

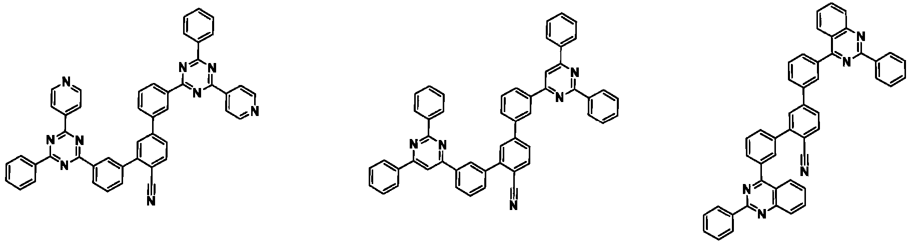

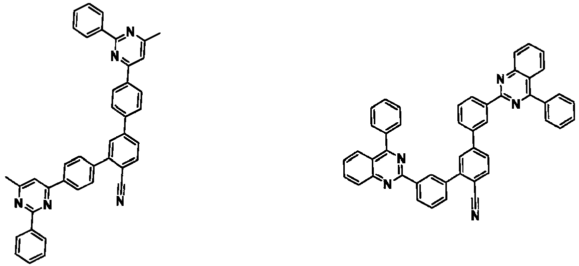

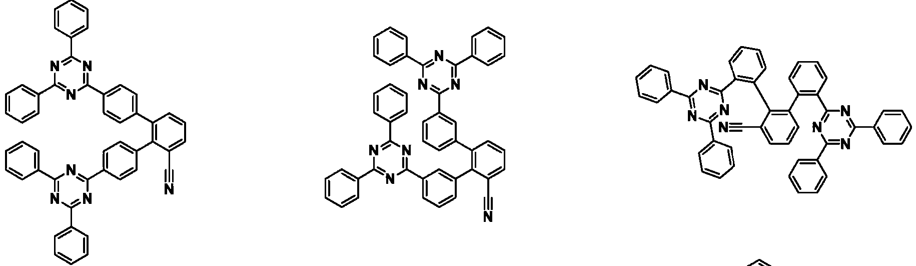

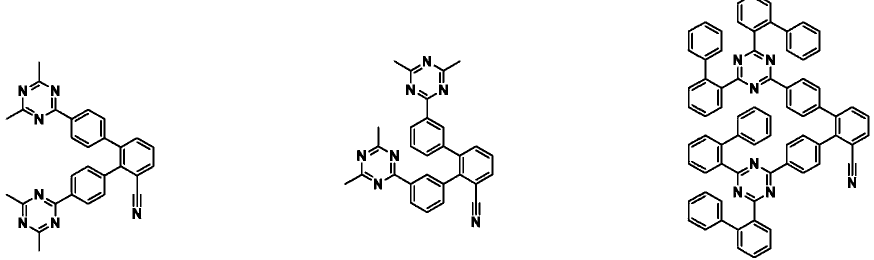

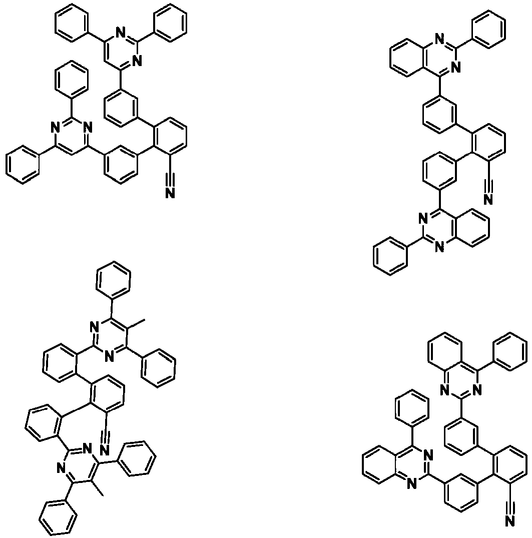

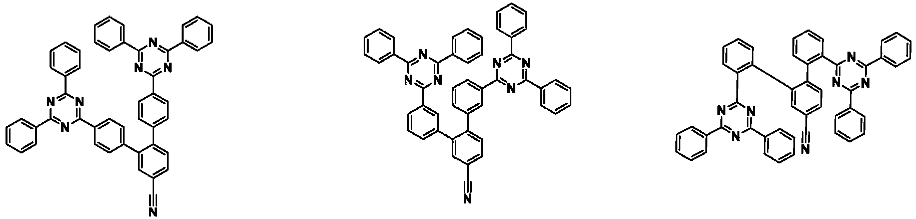

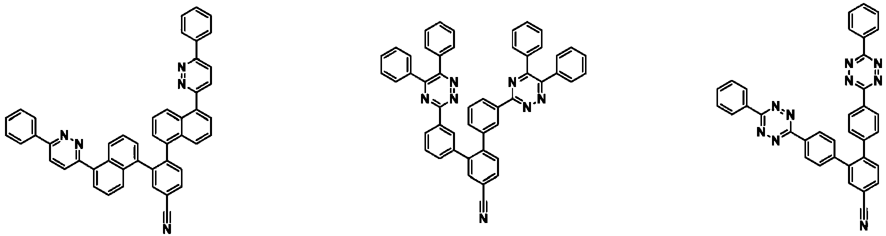

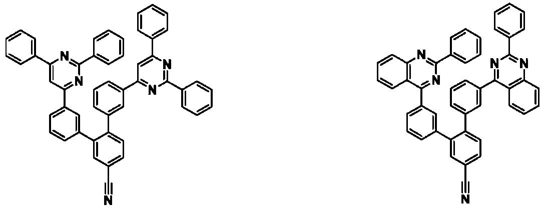

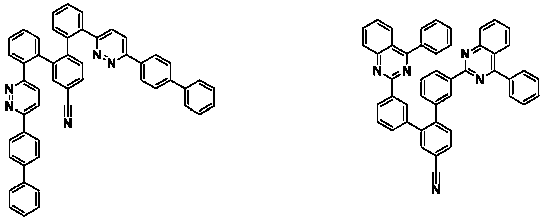

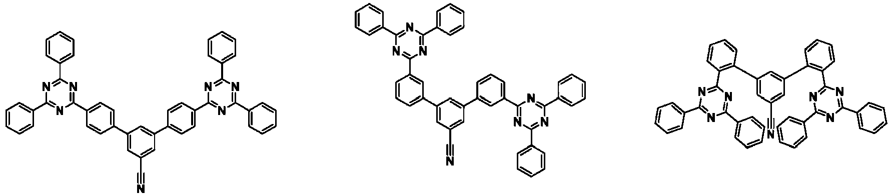

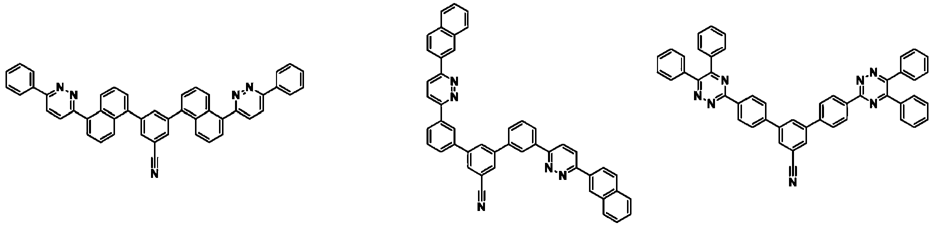

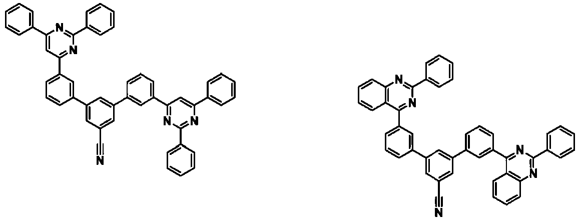

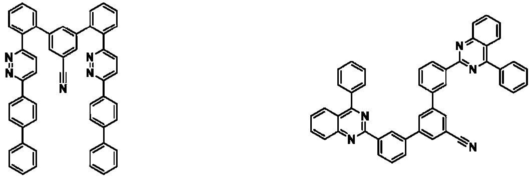

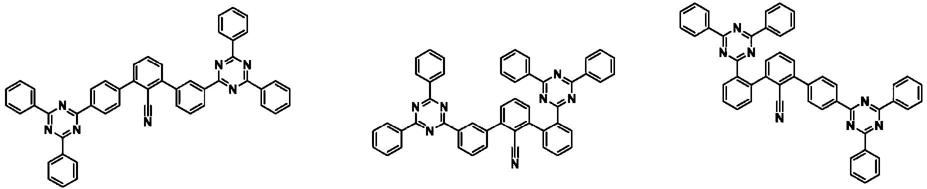

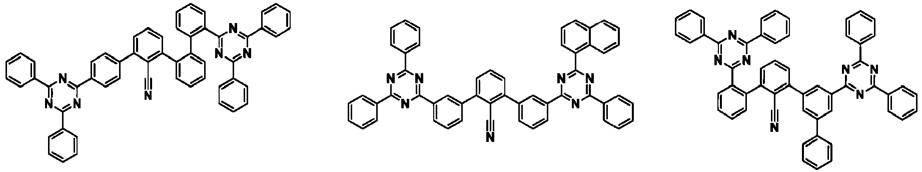

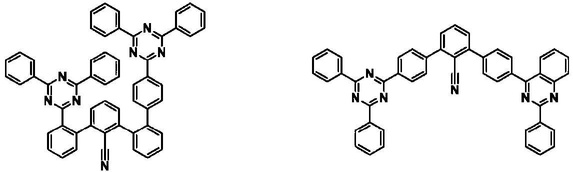

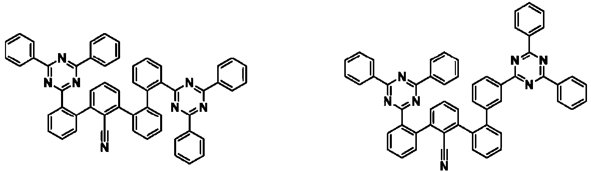

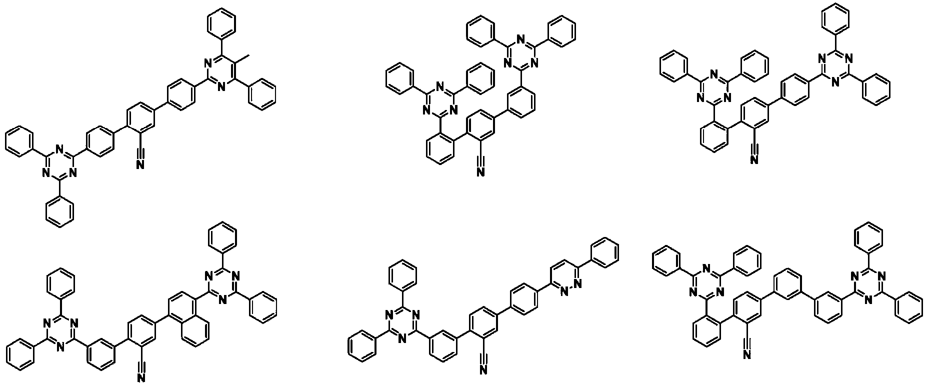

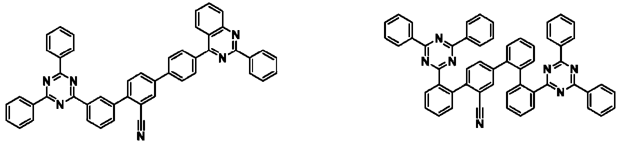

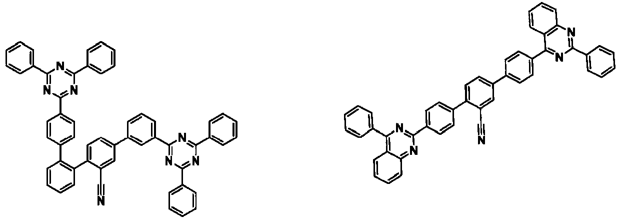

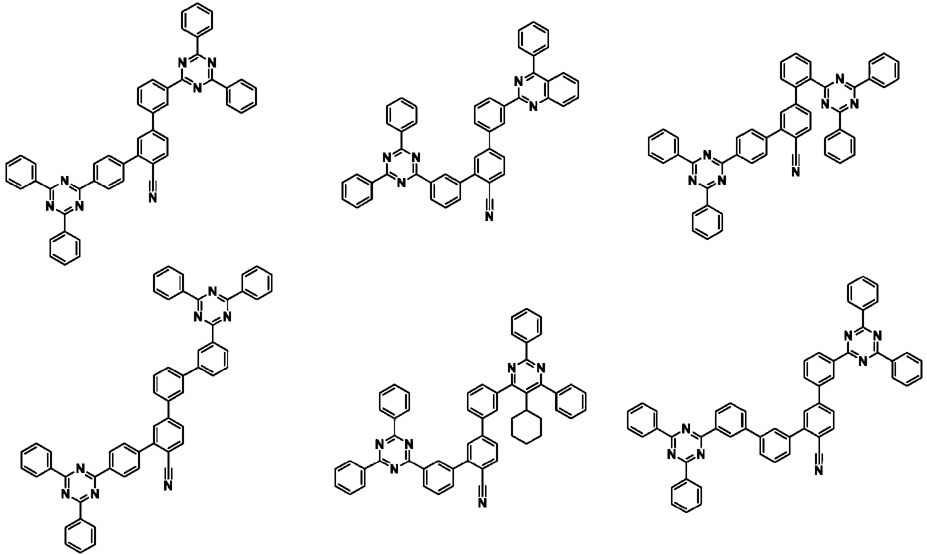

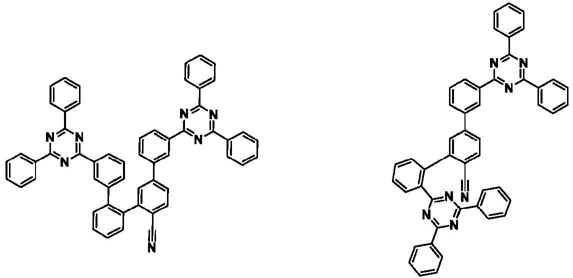

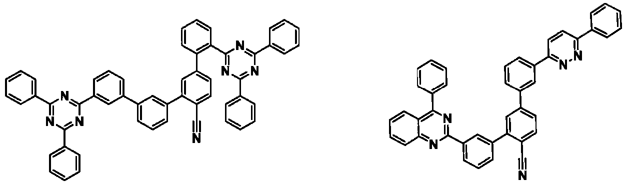

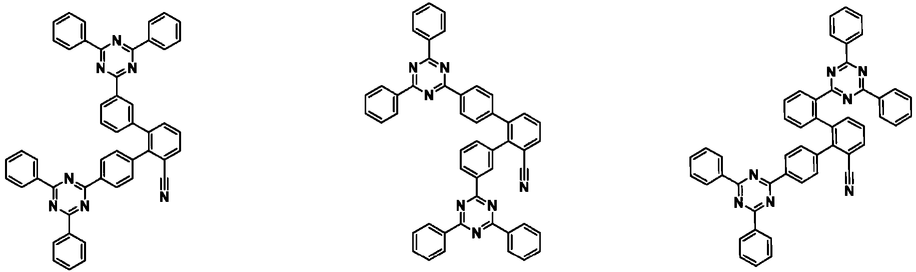

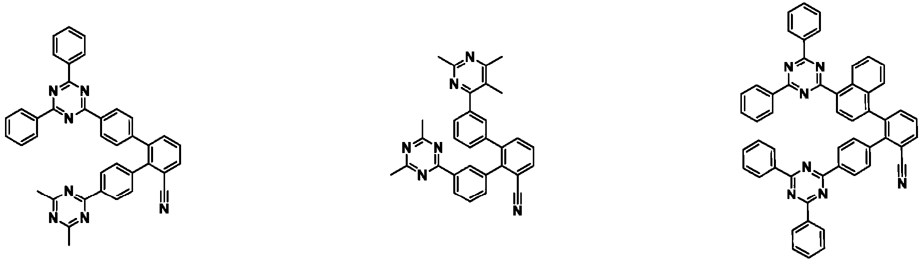

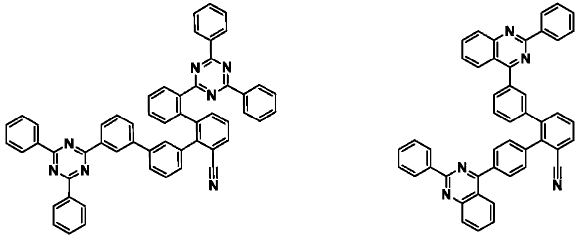

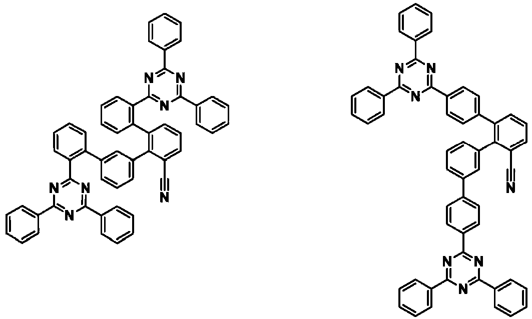

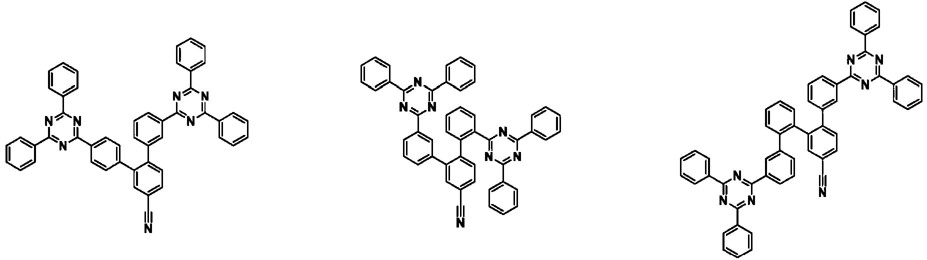

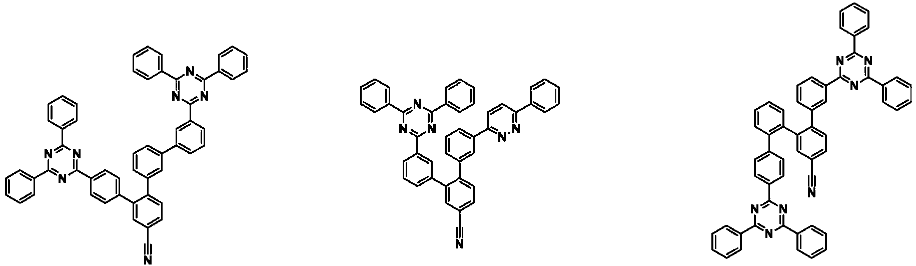

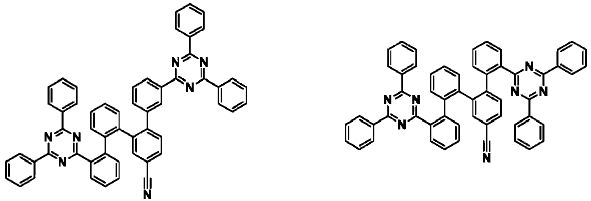

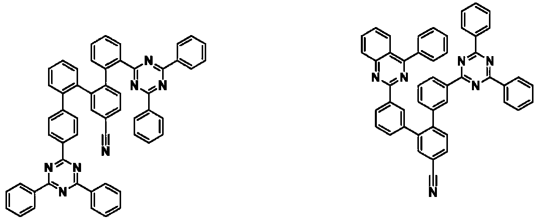

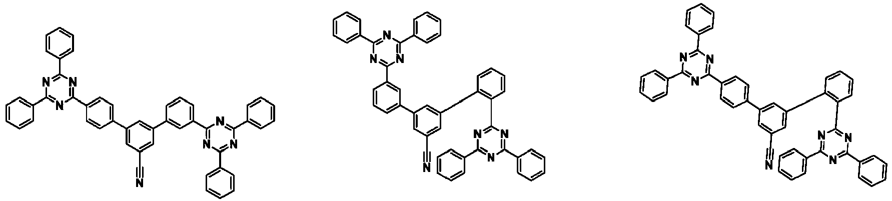

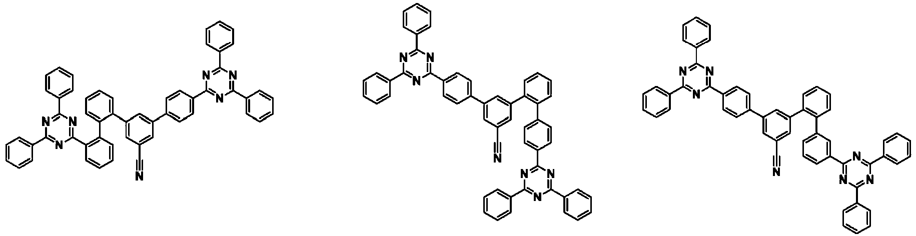

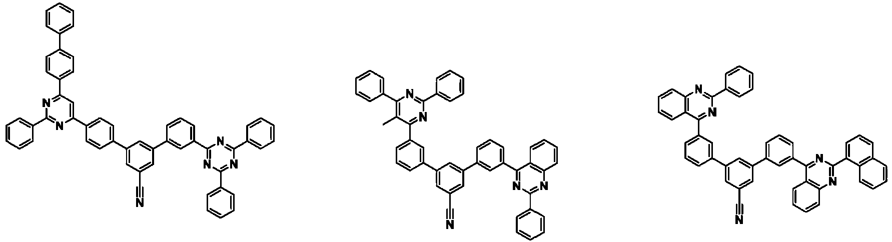

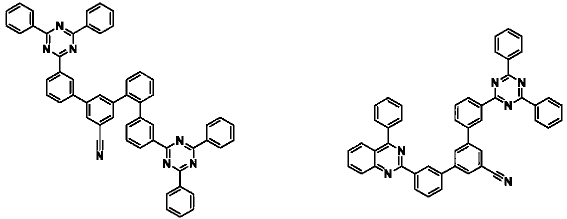

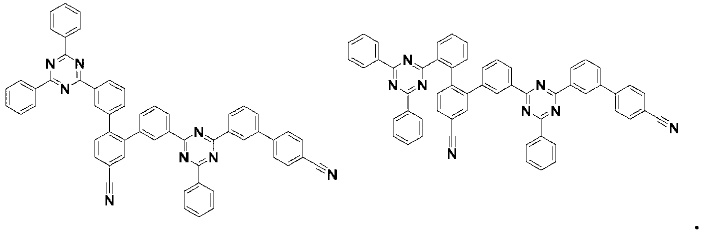

6. Dispositif électroluminescent organique comprenant :
une anode ;
une cathode ; et
une couche de matériau organique comportant une ou plusieurs couches disposées entre l'anode et la cathode, dans lequel une ou plusieurs couches de la couche de matériau organique comprennent le composé selon l'une quelconque des revendications 1 à 5.

7. Le dispositif électroluminescent organique selon la revendication 6, dans lequel la couche de matériau organique comprend une couche de transport d'électrons, une couche d'injection d'électrons ou une couche de transport et d'injection d'électrons, et la couche de transport d'électrons, la couche d'injection d'électrons ou la couche de transport et d'injection d'électrons comprend le composé.

8. Le dispositif électroluminescent organique selon la revendication 7, dans lequel la couche de transport d'électrons, la couche d'injection d'électrons ou la couche de transport et d'injection d'électrons comprend en outre un dopant de type n ou un composé métallique organique.

9. Le dispositif électroluminescent organique selon la revendication 8, dans lequel le composé et le dopant de type n ou le composé métallique organique sont compris dans un rapport pondéral de 2:8 à 8:2.

10. Le dispositif électroluminescent organique selon la revendication 6, dans lequel la couche de matériau organique comprend une couche de blocage des trous, et la couche de blocage des trous comprend le composé.

11. Le dispositif électroluminescent organique selon la revendication 6, dans lequel la couche de matériau organique comprend en outre une ou plusieurs couches parmi une couche d'injection de trous, une couche de transport de trous, une couche de blocage d'électrons, une couche d'injection et de transport de trous, une couche électroluminescente, une couche de transport d'électrons, une couche d'injection d'électrons, une couche de blocage de trous et une couche de transport et d'injection d'électrons.
